# EUROPEAN PATENT SPECIFICATION

(11) **EP 3 044 313 B1**
(45) Date of publication and mention of the grant of the patent: **06.11.2019**
(21) Application number: 14784386.6
(22) Date of filing: 11.09.2014
(51) Int. Cl.: C12N 9/54, C11D 3/386

(54) **COMPOSITIONS AND METHODS COMPRISING LG12-CLADE PROTEASE VARIANTS**
ZUSAMMENSETZUNGEN UND VERFAHREN MIT PROTEASE VARIANTEN DER LG12-KLADE
COMPOSITIONS ET MÉTHODES COMPRENANT DES VARIANTS DE PROTÉASES DU CLADE LG12

(30) Priority: 12.09.2013 US 201361877087 P
(43) Date of publication of application: 20.07.2016
(60) Divisional application: 19199383.1
(73) Proprietor: Danisco US Inc., Palo Alto, California 94304 (US)
(72) Inventor: BABE, Lilia M., Palo Alto, California 94304 (US); ESTELL, David A., Palo Alto, California 94304 (US); GOEDEGEBUUR, Frits, Palo Alto, California 94304 (US); BOTT, Richard R., Palo Alto, California 94304 (US); KOLKMAN, Marc, Palo Alto, California 94304 (US); MULDER, Harm, Palo Alto, California 94304 (US); SCHMIDT, Brian F., Palo Alto, California 94304 (US); AUGUSTYN, Katherine, Palo Alto, California 94304 (US); ATWAL, Munroop, Palo Alto, California 94304 (US); MARQUEZ, David, Palo Alto, California 94304 (US)
(74) Representative: Mewburn Ellis LLP
(86) International application number: PCT/US2014/055223
(87) International publication number: WO 2015/038792

(56) References cited:
- EP-A1- 1 321 513
- WO-A1-94/06915
- US-A- 5 955 340
- DATABASE Geneseq [Online] 27 January 2005 (2005-01-27), "Novel Bacillus KS alkaline protease protein sequence SeqID2.", XP002734384, retrieved from EBI accession no. GSP:ADU05756 Database accession no. ADU05756 -& JP 2004 313043 A (KAO CORP) 11 November 2004 (2004-11-11)
- DATABASE Geneseq [Online] 26 August 2004 (2004-08-26), "Bacillus sp. KSM-LD1 alkaline protease mature protein.", XP002734385, retrieved from EBI accession no. GSP:ADP70361 Database accession no. ADP70361 -& JP 2004 154003 A (KAO CORP) 3 June 2004 (2004-06-03)
- YASUSHI TAKIMURA ET AL: "Alkaliphilic Bacillus sp. strain KSM-LD1 contains a record number of subtilisin-like serine proteases genes", APPLIED MICROBIOLOGY AND BIOTECHNOLOGY, SPRINGER, BERLIN, DE, vol. 76, no. 2, 15 June 2007 (2007-06-15), pages 395-405, XP019538683, ISSN: 1432-0614, DOI: 10.1007/S00253-007-1022-9
- HAN-SEUNG JOO ET AL: "Purification and Characterization of a Novel Alkaline Protease from Bacillus horikoshii", JOURNAL OF MICROBIOLOGY AND BIOTECHNOLOGY, vol. 22, no. 1, 28 January 2012 (2012-01-28), pages 58-68, XP55161644, ISSN: 1017-7825, DOI: 10.4014/jmb.1109.09006

## Description

### BACKGROUND OF THE INVENTION

Serine proteases are a class of enzymes that cleave peptide bonds in proteins. The serine proteases contain a nucleophilic serine residue at the active site. Subtilisins are a large family of serine proteases, and although they have long been known in the art of industrial enzymes, there remains a need for engineered proteases that are suitable for particular conditions and uses.

### SUMMARY OF THE INVENTION

The present disclosure provides, *inter alia*, LG12-clade protease enzymes, including variant LG12-clade protease enzymes, nucleic acids encoding the same, and compositions and methods related to the production and use thereof.

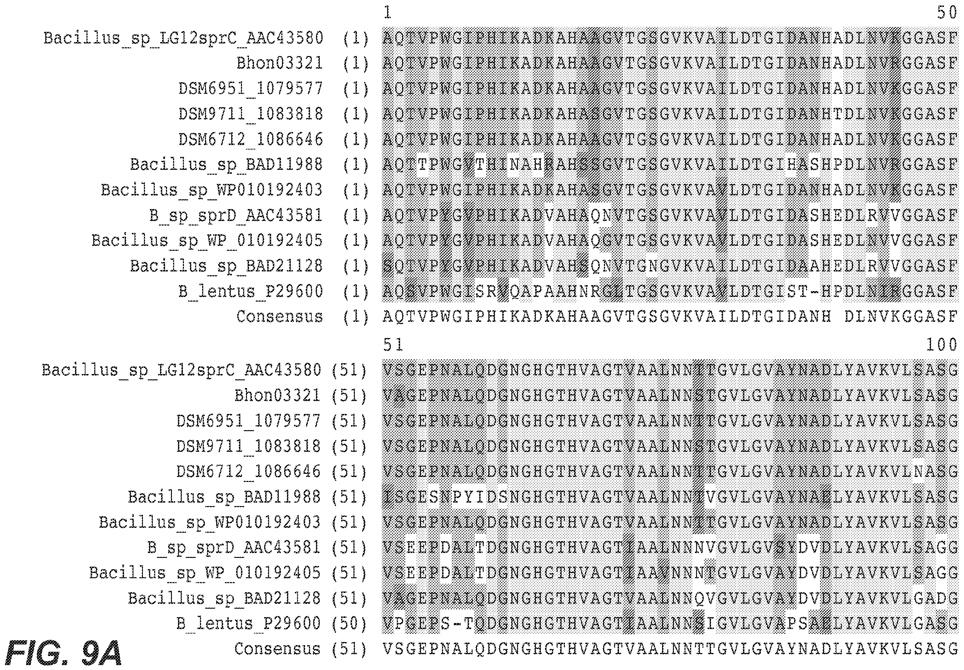

The invention provides an LG12-clade variant subtilisin enzyme or an active fragment thereof comprising an amino acid modification to a parent LG12-clade subtilisin enzyme, wherein the modification is at productive position 166, wherein said variant has at least 75% identity to the amino acid sequence of a parent LG12-clade subtilisin enzyme selected from Bacillus sp. LG12sprC substilin set forth in SEQ ID NO:3; Bacillus sp. M3-13 subtilisin enzyme E(1) set forth in SEQ ID NO:7; Bacillus horikoshii strain DSM 8719, Bhon03321 set forth in SEQ ID NO:10; Bacillus horikoshii strain DSM_9711 set forth in SEQ ID NO:13; Bacillus horikoshii strain DSM_9712 set forth in SEQ ID NO:16; Bacillus horikoshii strain DSM_6951 set forth in SEQ ID NO:19; B_sp_sprD_AAC43581 as shown in Fig. 9A-C; Bacillus_sp_WP_010192405 as shown in Fig. 9A-C; Bacillus_sp_BAD11988 as shown in Fig. 9A-C; and/or Bacillus sp. KSM-LD1, SB (BAD21128.1) set forth in SEQ ID NO:20, wherein the modification is selected from the group consisting of residues 166 C, F, I, L, A, D, E, H, M, N, Q, S, T and Y, wherein the amino acid positions of the LG12-clade subtilisin variant are numbered by correspondence with the amino acid sequence of *Bacillus sp.* LG12sprC subtilisin set forth in SEQ ID NO:3.

The invention further provides a composition comprising at least one subtilisin enzyme as set out in the claims, optionally wherein said composition is selected from a granular, powder, solid, bar, liquid, tablet, gel, unit dose or paste composition. In some aspects the composition is a cleaning composition or a detergent composition, optionally wherein said detergent composition is selected from the group consisting of a laundry detergent, a fabric softening detergent, a dishwashing detergent, and a hard-surface cleaning detergent.

The invention further provides a method of cleaning, comprising contacting a surface or an item with a cleaning composition comprising at least one subtilisin enzyme or cleaning composition as set forth in the claims, and optionally rinsing said surface or item after contacting said surface or item, respectively, with said cleaning composition.

The invention also provides a textile processing, animal feed, leather processing, feather processing, grain processing, cellulosic ethanol processing, lens cleaning, tissue debridement, or tissue cell culture additive composition comprising the subtilisin enzyme set forth in the claims.

Further aspects and embodiments are set forth in the accompanying claims and are described in detail below.

### BRIEF DESCRIPTION OF THE DRAWINGS

FIG. 1 provides a plasmid map of pHYT-LG12 for the expression of LG12 sprC wildtype and variant proteases
FIG. 2 provides the sequence of the precursor to LG12 sprC protein as encoded in plasmid pHYT-LG12.
FIG. 3 provides provides a plasmid map of pHYT-Bhon03321 for the expression of Bhon03321.
FIG. 4 provides the cleaning performance of LG12 homologs in OMO HDL detergent.
FIG.5 provides the cleaning performance of LG12 homologs in OMO HDD detergent.
FIG. 6 provides the cleaning performance of LG12 homologs in GSM-B pH 9 detergent.
FIG. 7 provides the cleaning performance of LG12 homologs in GSM-B pH 10.5 detergent.
FIG. 8 provides a phylogenetic tree for numerous subtilisins with greater than 60% sequence identity to LG12 sprC, including the LG12-clade of homologs to sprC shown within brackets.
FIGS. 9A-9B provide an alignment of the various LG12-clade proteases: Bac sp LG12SprC, DSM_9711, DSM_9712, DSM_6951, Bhon03321, Bac sp BAD11988, Bac sp WP_010192403, Bac sp LG12SprD, Bac sp WP_010192405, Bac sp BAD21128, in addition to the subtilisin *B lentus* P29600.
FIGS. 10A-10B provide the structure-based alignment of useful detergent proteases with LG12 sprC protease and other known subtilisins of close sequence identity to LG12 sprC. Regions underlined point to motif sequences shared by members of LG12-clade.
FIG. 11 provides a region of the homology model of the structure of LG12 sprC protease based on the structure of subtilisin from *B. licheniformis.* In this figure, the side chains of Tyr 86, Asn 87, Thr 22 and His 17 are shown as stick figures.
FIG. 12 provides a region of the homology model of LG12 sprC protease with the substrate binding cleft and active site triad at the base of image. In this figure, the side chains of Thr103 and Leu 104 are shown as stick figures.

### DESCRIPTION OF THE INVENTION

The present invention provides novel serine protease enzymes, particularly enzymes and variants of the LG12-clade serine proteases and especially enzymes useful for detergent compositions as set out in the claims. In some embodiments, the present invention provides serine protease enzyme variants having one or more modifications, such as a substitution, as compared to a parent serine protease enzyme. In some embodiments, the present invention provides novel serine protease enzymes of the LG12-clade serine proteases. In each of the above, the serine protease enzymes of the present invention can be useful in several industries, including soil removal, grain ethanol production, animal feed, food processing and digestive aids, leather processing, and personal care. This can be achieved by making improvements to the enzyme by improving cleaning performance, stability of the enzyme in the presence of chemical agents, and/or thermostability of the enzyme that improve effectiveness of the enzyme. The present invention provides variant LG12 sprC serine protease enzymes, that are particularly well suited to and useful in a variety of cleaning applications. The invention includes compositions comprising at least one of the novel and/or variant serine protease enzymes (e.g., variant LG12 sprC) set forth in the claims. Some such compositions comprise detergent compositions. The invention provides various species, including *Bacillus species* variant serine protease enzymes and compositions comprising one or more such variant LG12-clade serine proteases. The serine protease enzyme variants of the present invention can be combined with other enzymes useful in detergent compositions. The invention also provides methods of using a novel and/or variant serine protease enzyme of the present invention.

The invention includes enzyme variants of serine protease enzymes having one or more modifications from a parent serine protease enzyme. The enzyme variants can be useful in a detergent composition by having a minimum performing index for wash performance, stability of the enzyme in detergent compositions and thermostability of the enzyme, while having at least one of these characteristics improved from a parent serine protease enzyme. These variants are set out in the claims.

Additionally, the invention provides modifications, such as a substitution, at one or more amino acid positions in a serine protease enzyme which can be useful in a detergent composition where favorable modifications result in a minimum performing index for wash performance, stability of the enzyme in detergent compositions and thermostability of the enzyme, while having at least one of these characteristics improved from a parent serine protease enzyme. These modifications are considered suitable modifications of the invention. These amino acid positions can be considered useful positions for combinatorial modifications to a parent serine protease enzyme. Serine protease enzyme amino acid positions found to be useful positions can be further characterized by having multiple modifications that are suitable for use in a detergent composition. For each position, greater numbers of possible suitable modifications denotes a higher productivity of a particular position. Useful modifications are set out in the claims.

In addition, the present invention provides compositions comprising a novel and/or variant serine protease enzyme of the present invention. In some embodiments, the present invention provides cleaning compositions comprising at least one of these novel and/or variant serine protease enzymes.

### DEFINITIONS

Unless otherwise indicated, the practice of the present invention involves conventional techniques commonly used in molecular biology, protein engineering, microbiology, and recombinant DNA, which are within the skill of the art. Such techniques are known to those of skill in the art and are described in numerous texts and reference works well known to those of skill in the art.

Unless defined otherwise herein, all technical and scientific terms used herein have the same meaning as commonly understood by one of ordinary skill in the art to which this invention pertains. Many technical dictionaries are known to those of skill in the art. Although any methods and materials similar or equivalent to those described herein find use in the practice of the present invention, some suitable methods and materials are described herein. Accordingly, the terms defined immediately below are more fully described by reference to the Specification as a whole. Also, as used herein, the singular "a", "an" and "the" includes the plural reference unless the context clearly indicates otherwise. Numeric ranges are inclusive of the numbers defining the range. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

The practice of the present invention employs, unless otherwise indicated, conventional techniques of protein purification, molecular biology, microbiology, recombinant DNA techniques and protein sequencing, all of which are within the skill of those in the art.

Furthermore, the headings provided herein are not limitations of the various aspects or embodiments of the invention which can be had by reference to the specification as a whole. Accordingly, the terms defined immediately below are more fully defined by reference to the specification as a whole. Nonetheless, in order to facilitate understanding of the invention, a number of terms are defined below.

It is intended that every maximum numerical limitation given throughout this specification include every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

As used herein, the terms "protease" and "proteinase" refer to an enzyme protein that has the ability to break down other proteins. A protease has the ability to conduct "proteolysis," which begins protein catabolism by hydrolysis of peptide bonds that link amino acids together in a peptide or polypeptide chain forming the protein. This activity of a protease as a protein-digesting enzyme is referred to as "proteolytic activity." Many well known procedures exist for measuring proteolytic activity (*See e.g.,* Kalisz, "Microbial Proteinases," In: Fiechter (ed.), Advances in Biochemical Engineering/Biotechnology, (1988)). For example, proteolytic activity may be ascertained by comparative assays which analyze the respective protease's ability to hydrolyze a commercial substrate. Exemplary substrates useful in the analysis of protease or proteolytic activity, include, but are not limited to, di-methyl casein (Sigma C-9801), bovine collagen (Sigma C-9879), bovine elastin (Sigma E-1625), and bovine keratin (ICN Biomedical 902111). Colorimetric assays utilizing these substrates are well known in the art (*See e.g.,* WO 99/34011 and U.S. Pat. No. 6,376,450, both of which are incorporated herein by reference). The pNA assay (*See e.g.,* Del Mar et al., Anal. Biochem. 99:316-320 [1979]) also finds use in determining the active enzyme concentration for fractions collected during gradient elution. This assay measures the rate at which p-nitroaniline is released as the enzyme hydrolyzes a soluble synthetic peptide substrate, such as succinyl-alanine-alanine-proline-phenylalanine-p-nitroanilide (suc-AAPF-pNA), and cleavage occurs between the C-terminal amino acid (phenylalanine) and the p-NA, causing the production of yellow color from the hydrolysis reaction, which is measured at 410 nm on a spectrophotometer and is proportional to the active enzyme concentration. Measurement of the color change allows calculation of the rate of the reaction. In addition, absorbance measurements at 280 nanometers (nm) can be used to determine the total protein concentration. The active enzyme/total protein ratio gives the enzyme purity when a reference standard is used.

As used herein, the term "variant polypeptide" refers to a polypeptide comprising an amino acid sequence that differs in at least one amino acid residue from the amino acid sequence of a parent or reference polypeptide (including but not limited to wild-type polypeptides).

As used herein, "the genus *Bacillus"* includes all species within the genus *"Bacillus,"* as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. pumilus, B. gibsonii, B. pseudofirmus, B. lehensis, B. megaterium, B. coagulans, B. circulans, B. lautus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus stearothermophilus."* The production of resistant endospores in the presence of oxygen is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

The terms "polynucleotide" and "nucleic acid," which are used interchangeably herein, refer to a polymer of any length of nucleotide monomers covalently bonded in a chain. DNA (deoxyribonucleic acid), a polynucleotide comprising deoxyribonucleotides, and RNA (ribonucleic acid), a polymer of ribonucleotides, are examples of polynucleotides or nucleic acids having distinct biological function. Polynucleotides or nucleic acids include, but are not limited to, a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases. The following are non-limiting examples of polynucleotides: genes, gene fragments, chromosomal fragments, expressed sequence tag(s) (EST(s)), exons, introns, messenger RNA (mRNA), transfer RNA (tRNA), ribosomal RNA (rRNA), ribozymes, complementary DNA (cDNA), recombinant polynucleotides, branched polynucleotides, plasmids, vectors, isolated DNA of any sequence, isolated RNA of any sequence, nucleic acid probes, and primers. In some embodiments, polynucleotides comprise modified nucleotides, such as methylated nucleotides and nucleotide analogs, uracyl, other sugars and linking groups such as fluororibose and thioate, and nucleotide branches. In a particular embodiment, a sequence of nucleotides is interrupted by non-nucleotide components.

As used herein, the term "mutation" refers to changes made in a starting amino acid or nucleic acid sequence. It is intended that the term encompass substitutions, insertions and deletions.

As used herein, the term "vector" refers to a nucleic acid construct or polynucleotide construct used to introduce or transfer nucleic acid(s) or polynucleotide(s) into a target cell or tissue. A vector is typically used to introduce foreign DNA into another cell or tissue. A vector generally comprises a DNA sequence that is a transgene and a larger polynucleotide sequence that serves as the "backbone" of the vector. The vector typically serves to transfers genetic information, such as the inserted transgene, to a target cell or tissue so as to isolate, multiply, or express the insert in the target cell or tissue. Vectors include plasmids, cloning vectors, bacteriophages, viruses (*e.g.,* viral vector), cosmids, expression vectors, shuttle vectors, cassettes, and the like. A vector typically includes an origin of replication, a multicloning site, and a selectable marker. The process of inserting a vector into a target cell is typically referred to as transfection. The transfection of a cell with a viral vector is typically referred to as transduction. The present invention includes, in some embodiments, a vector that comprises a DNA sequence encoding a variant protease (*e.g.,* precursor or mature variant protease) that is operably linked to a suitable prosequence (*e.g.,* secretory, signal peptide sequence, etc.) capable of effecting the expression of the DNA sequence in a suitable host.

As used herein, the term "expression cassette," "expression plasmid" or "expression vector" refers to a nucleic acid construct or vector generated recombinantly or synthetically for the expression of a nucleic acid of interest (*e.g.,* a foreign nucleic acid or transgene) in a target cell. The nucleic acid of interest typically expresses a protein of interest. An expression vector or expression cassette typically comprises a promoter nucleotide sequence that drives or promotes expression of the foreign nucleic acid. The expression vector or cassette also typically includes any other specified nucleic acid elements that permit transcription of a particular nucleic acid in a target cell. A recombinant expression cassette can be incorporated into a plasmid, chromosome, mitochondrial DNA, plastid DNA, virus, or nucleic acid fragment. Some expression vectors have the ability to incorporate and express heterologous DNA fragments in a host cell. Many prokaryotic and eukaryotic expression vectors are commercially available. Selection of appropriate expression vectors is within the knowledge of those of skill in the art. Selection of appropriate expression vectors for expression of a protein from a nucleic acid sequence incorporated into the expression vector is within the knowledge of those of skill in the art.

A DNA construct is an artificially constructed segment of nucleic acid that may be introduced into a target cell or tissue. A DNA construct typically comprises a DNA insert comprising a nucleotide sequence encoding a protein of interest that has been subcloned into a vector. The vector may contain bacterial resistance genes for growth in bacteria and a promoter for expression of the protein of interest in an organism. The DNA may be generated *in vitro* by PCR or any other suitable technique(s) known to those in the art. In some embodiments, the DNA construct comprises a nucleic acid sequence of interest. In some embodiments, the sequence is operably linked to additional elements such as control elements (*e.g.,* promoters, etc.). The DNA construct may further comprise a selectable marker and may further comprise an incoming sequence flanked by homology boxes. The construct may comprise other non-homologous sequences, added to the ends (*e.g.,* stuffer sequences or flanks). In some embodiments, the ends of the sequence are closed such that the DNA construct forms a closed circle. The nucleic acid sequence of interest, which is incorporated into the DNA construct, using techniques well known in the art, may be a wild-type, mutant, or modified nucleic acid. In some embodiments, the DNA construct comprises one or more nucleic acid sequences homologous to the host cell chromosome. In other embodiments, the DNA construct comprises one or more non-homologous nucleotide sequences. Once the DNA construct is assembled *in vitro,* it may be used, for example, to: 1) insert heterologous sequences into a desired target sequence of a host cell; and/or 2) mutagenize a region of the host cell chromosome (*i.e.,* replace an endogenous sequence with a heterologous sequence); 3) delete target genes; and/or 4) introduce a replicating plasmid into the host. "DNA construct" is used interchangeably herein with "expression cassette."

As used herein, a "plasmid" refers to an extrachromosomal DNA molecule which is capable of replicating independently from the chromosomal DNA. A plasmid is double stranded (ds) and may be circular and is typically used as a cloning vector.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, electroporation, conjugation, and transduction (*See e.g.,* Ferrari et al., "Genetics," in Hardwood et al. (eds.), Bacillus, Plenum Publishing Corp., pp. 57-72 [1989]).

Transformation refers to the genetic alteration of a cell which results from the uptake, genomic incorporation, and expression of genetic material (*e.g.,* DNA).

As used herein, a nucleic acid is "operably linked" with another nucleic acid sequence when it is placed into a functional relationship with another nucleic acid sequence. For example, a promoter or enhancer is operably linked to a nucleotide coding sequence if the promoter affects the transcription of the coding sequence. A ribosome binding site may be operably linked to a coding sequence if it is positioned so as to facilitate translation of the coding sequence. Typically, "operably linked" DNA sequences are contiguous. However, enhancers do not have to be contiguous. Linking is accomplished by ligation at convenient restriction sites. If such sites do not exist, synthetic oligonucleotide adaptors or linkers may be used in accordance with conventional practice.

As used herein the term "gene" refers to a polynucleotide (*e.g.,* a DNA segment), that encodes a polypeptide and includes regions preceding and following the coding regions as well as intervening sequences (introns) between individual coding segments (exons).

As used herein, "recombinant" when used with reference to a cell typically indicates that the cell has been modified by the introduction of a heterologous nucleic acid sequence or that the cell is derived from a cell so modified. For example, a recombinant cell may comprise a gene not found in identical form within the native (non-recombinant) form of the cell, or a recombinant cell may comprise a native gene (found in the native form of the cell) but which has been modified and re-introduced into the cell. A recombinant cell may comprise a nucleic acid endogenous to the cell that has been modified without removing the nucleic acid from the cell; such modifications include those obtained by gene replacement, site-specific mutation, and related techniques known to those of ordinary skill in the art. Recombinant DNA technology includes techniques for the production of recombinant DNA *in vitro* and transfer of the recombinant DNA into cells where it may be expressed or propagated, thereby producing a recombinant polypeptide. "Recombination," "recombining," and "recombined" of polynucleotides or nucleic acids refer generally to the assembly or combining of two or more nucleic acid or polynucleotide strands or fragments to generate a new polynucleotide or nucleic acid. The recombinant polynucleotide or nucleic acid is sometimes referred to as a chimera. A nucleic acid or polypeptide is "recombinant" when it is artificial or engineered, or derived from an artificial or engineered protein or nucleic acid.

As used herein, the term nucleic acid or gene "amplification" refers to a process by which specific DNA sequences are disproportionately replicated such that the amplified nucleic acid or gene becomes present in a higher copy number than was initially present in the genome. In some embodiments, selection of cells by growth in the presence of a drug (*e.g.,* an inhibitor of an inhibitable enzyme) results in the amplification of either the endogenous gene encoding the gene product required for growth in the presence of the drug or by amplification of exogenous (*i.e.,* input) sequences encoding this nucleic acid or gene product or both.

"Amplification" is a special case of nucleic acid replication involving template specificity. It is to be contrasted with non-specific template replication (*i.e.,* replication that is template-dependent but not dependent on a specific template). Template specificity is here distinguished from fidelity of replication (*i.e.,* synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

As used herein, the term "primer" refers to an oligonucleotide (a polymer of nucleotide residues), whether occurring naturally as in a purified restriction digest or produced synthetically, which is capable of acting as a point of initiation of synthesis when placed under conditions in which synthesis of a primer extension product which is complementary to a nucleic acid strand is induced (*i.e.,* in the presence of nucleotides and an inducing agent such as DNA polymerase and at a suitable temperature and pH). A primer is preferably single stranded for maximum efficiency in amplification, but may alternatively be double stranded. If double stranded, the primer is first treated to separate its strands before being used to prepare extension products. In some embodiments, the primer is an oligodeoxyribonucleotide. The primer must be sufficiently long to prime the synthesis of extension products in the presence of the inducing agent. The exact length of a primer depends on a variety of factors, including temperature, source of primer, and the use of the method.

As used herein, the term "probe" refers to an oligonucleotide, whether occurring naturally as in a purified restriction digest or produced synthetically, recombinantly or by PCR amplification, which is typically capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that it is detectable in any detection system, including, but not limited to enzyme (*e.g.,* ELISA, as well as enzyme-based histochemical assays), fluorescent, radioactive, and luminescent systems. It is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A nucleotide "segment" is a region of a nucleic acid within the target nucleic acid sequence.

As used herein, the term "polymerase chain reaction" (PCR) refers to the methods of U.S. Patent Nos. 4,683,195 4,683,202, and 4,965,188, which include methods for increasing the concentration of a segment of a target sequence in a mixture of genomic DNA without cloning or purification. This process for amplifying the target sequence is well known in the art.

As used herein, the term "amplification reagents" refers to those reagents (*e.g.,* deoxyribonucleotide triphosphates, buffer, etc.) needed for amplification except for primers, nucleic acid template, and the amplification enzyme. Typically, amplification reagents along with other reaction components are placed and contained in a reaction vessel (test tube, microwell, etc.).

As used herein, the term "restriction endonuclease" or "restriction enzyme" refers to an enzyme (*e.g.,* bacterial enzyme) that is capable of cutting double-stranded or single-stranded DNA at or near a specific sequence of nucleotides known as a restriction site. The nucleotide sequence comprising the restriction site is recognized and cleaved by a given restriction endonuclease or restriction enzyme and is frequently the site for insertion of DNA fragments. A restriction site can be engineered into an expression vector or DNA construct.

"Homologous recombination" refers to the exchange of DNA fragments between two DNA molecules or paired chromosomes at the site of identical or nearly identical nucleotide sequences. In some embodiments, chromosomal integration is homologous recombination.

A nucleic acid or polynucleotide is said to "encode" a polypeptide if, in its native state or when manipulated by methods known to those of skill in the art, it can be transcribed and/or translated to produce the polypeptide or a fragment thereof. The anti-sense strand of such a nucleic acid is also said to encode the sequence.

As is known in the art, a DNA sequence can be transcribed by an RNA polymerase to produce an RNA sequence, but an RNA sequence can be reverse transcribed by reverse transcriptase to produce a DNA sequence.

"Host strain" or "host cell" refers to a suitable host for an expression vector comprising a DNA sequence of interest. The DNA sequence of interest may express a protein of interest in the host strain or host cell.

A "protein" or "polypeptide" comprises a polymeric sequence of amino acid residues. The terms "protein" and "polypeptide" are used interchangeably herein. The single and 3-letter code for amino acids as defined in conformity with the IUPAC-IUB Joint Commission on Biochemical Nomenclature (JCBN) is used through out this disclosure. The single letter X refers to any of the twenty amino acids. It is also understood that a polypeptide may be coded for by more than one nucleotide sequence due to the degeneracy of the genetic code. Mutations are named by the one letter code for the parent amino acid, followed by a three or two digit position number and then the one letter code for the variant amino acid. For example, mutating glycine (G) at position 87 to serine (S) is represented as "G087S" or "G87S". Multiple mutations are indicated by inserting a "-" between the mutations. Mutations at positions 87 and 90 are represented as either "G087S-A090Y" or "G87S-A90Y" or "G87S + A90Y" or "G087S + A090Y". For deletions, the one letter code "Z" is used. For an insertion relative to the parent sequence, the one letter code "Z" is on the left side of the position number. For a deletion, the one letter code "Z" is on the right side of the position number. For insertions, the position number is the position number before the inserted amino acid(s), plus 0.01 for each amino acid. For example, an insertion of three amino acids alanine (A), serine (S) and tyrosine (Y) between position 87 and 88 is shown as "Z087.01A-Z087.02S-Z087.03Y." Thus, combining all the mutations above plus a deletion at position 100 is: "G087S- Z087.01A-Z087.02S-Z087.03Y-A090Y-A100Z." When describing modifications, a position followed by amino acids listed in parentheses indicates a list of substitutions at that position by any of the listed amino acids. For example, 6(L,I) means position 6 can be substituted with a leucine or isoleucine.

A "prosequence" or "propetide sequence" refers to an amino acid sequence between the signal peptide sequence and mature protease sequence that is necessary for proper folding and the secretion of the protease. Cleavage of the prosequence or propeptide sequence results in a mature active protease. The prosequence may be endogenous or the sequence may occur is a highly related protein such as another subtilisin.

The term "signal sequence" or "signal peptide" refers to a sequence of amino acid residues that may participate in the secretion or direct transport of the mature or precursor form of a protein. The signal sequence is typically located N-terminal to the precursor or mature protein sequence. The signal sequence may be endogenous or exogenous. A signal sequence is normally absent from the mature protein. A signal sequence is typically cleaved from the protein by a signal peptidase after the protein is transported.

The term "hybrid signal sequence" refers to signal sequences in which part of sequence is obtained from the expression host fused to the signal sequence of the gene to be expressed. In some embodiments, synthetic sequences are utilized.

The term "mature" form of a protein, polypeptide, or peptide refers to the functional form of the protein, polypeptide, or peptide without the signal peptide sequence and propeptide sequence.

The term "precursor" form of a protein or peptide refers to a mature form of the protein having a prosequence operably linked to the amino or carbonyl terminus of the protein. The precursor may also have a "signal" sequence operably linked to the amino terminus of the prosequence. The precursor may also have additional polynucleotides that are involved in post-translational activity (*e.g.,* polynucleotides cleaved therefrom to leave the mature form of a protein or peptide).

The term "wild-type" in reference to an amino acid sequence or nucleic acid sequence indicates that the amino acid sequence or nucleic acid sequence is native or naturally occurring sequence. As used herein, the term "naturally-occurring" refers to anything (*e.g.,* proteins, amino acids, or nucleic acid sequences) that are found in nature (*i.e.,* have not been manipulated by means of recombinant methods).

As used herein, the term "non-naturally occurring" refers to anything that is not found in nature (*e.g.,* recombinant polynucleotides or polypeptides produced in the laboratory).

As used herein with regard to amino acid residue positions, "corresponding to" or "corresponds to" or "corresponds" refers to an amino acid residue at the enumerated position in a protein or peptide, or an amino acid residue that is analogous, homologous, or equivalent to an enumerated residue in a protein or peptide. As used herein, "corresponding region" generally refers to an analogous position in a related proteins or a reference protein.

The terms "derived from" and "obtained from" refer to not only a protein produced or producible by a strain of the organism in question, but also a protease encoded by a DNA sequence isolated from such strain and produced in a host organism containing such DNA sequence. Additionally, the term refers to a protein which is encoded by a DNA sequence of synthetic and/or cDNA origin and which has the identifying characteristics of the protease in question. To exemplify, "proteases derived from *Bacillus*" refers to those enzymes having proteolytic activity which are naturally produced by *Bacillus,* as well as to serine proteases like those produced by *Bacillus* sources but which through the use of genetic engineering techniques are produced by a *Bacillus* strain or non*-Bacillus* organisms transformed with a nucleic acid encoding the serine proteases.

The term "identical" in the context of two polypeptide sequences refers to the residues in the two sequences that are the same when aligned for maximum correspondence, as measured using one of the following sequence comparison or analysis algorithms.

As used herein, "homologous genes" refers to a pair of genes from different, but usually related species, which correspond to each other and which are identical or very similar to each other. The term encompasses genes that are separated by speciation (i.e., the development of new species) *(e.g.,* orthologous genes), as well as genes that have been separated by genetic duplication (*e.g.,* paralogous genes).

As used herein, "homology" refers to sequence similarity or identity, with identity being preferred. Homology may be determined using standard techniques known in the art (*See e.g*., Smith and Waterman, Adv. Appl. Math. 2:482 [1981]; Needleman and Wunsch, J. Mol. Biol. 48:443 [1970]; Pearson and Lipman, Proc. Natl. Acad. Sci. USA 85:2444 [1988]; software programs such as GAP, BESTFIT, FASTA, and TFASTA in the Wisconsin Genetics Software Package (Genetics Computer Group, Madison, WI); and Devereux et al., Nucl. Acid Res. 12:387-395 [1984]). One example of a useful algorithm is PILEUP. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle (*See,* Feng and Doolittle, J. Mol. Evol. 35:351-360 [1987]). The method is similar to that described by Higgins and Sharp (*See,* Higgins and Sharp, CABIOS 5:151-153 [1989]). Useful PILEUP parameters including a default gap weight of 3.00, a default gap length weight of 0.10, and weighted end gaps.

The percent sequence identity between a reference sequence and a test sequence of interest may be readily determined by one skilled in the art. The percent identity shared by polynucleotide or polypeptide sequences is determined by direct comparison of the sequence information between the molecules by aligning the sequences and determining the identity by methods known in the art. An example of an algorithm that is suitable for determining sequence similarity is the BLAST algorithm, (*See,* Altschul, et al., J. Mol. Biol., 215:403-410 [1990]). Software for performing BLAST analyses is publicly available through the National Center for Biotechnology Information. This algorithm involves first identifying high scoring sequence pairs (HSPs) by identifying short words of length W in the query sequence that either match or satisfy some positive-valued threshold score T when aligned with a word of the same length in a database sequence. These initial neighborhood word hits act as starting points to find longer HSPs containing them. The word hits are expanded in both directions along each of the two sequences being compared for as far as the cumulative alignment score can be increased. Extension of the word hits is stopped when: the cumulative alignment score falls off by the quantity X from a maximum achieved value; the cumulative score goes to zero or below; or the end of either sequence is reached. The BLAST algorithm parameters W, T, and X determine the sensitivity and speed of the alignment. The BLAST program uses as defaults a wordlength (W) of 11, the BLOSUM62 scoring matrix (*See,* Henikoff and Henikoff, Proc. Natl. Acad. Sci. USA 89:10915 [1992]) alignments (B) of 50, expectation (E) of 10, M'5, N'-4, and a comparison of both strands.

The BLAST algorithm then performs a statistical analysis of the similarity between two sequences (*See e.g.,* Karlin and Altschul, *supra*). One measure of similarity provided by the BLAST algorithm is the smallest sum probability (P(N)), which provides an indication of the probability by which a match between two nucleotide or amino acid sequences would occur by chance. For example, a nucleic acid is considered similar to a serine protease nucleic acid of this invention if the smallest sum probability in a comparison of the test nucleic acid to a serine protease nucleic acid is less than about 0.1, more preferably less than about 0.01, and most preferably less than about 0.001. Where the test nucleic acid encodes a serine protease polypeptide, it is considered similar to a specified serine protease nucleic acid if the comparison results in a smallest sum probability of less than about 0.5, and more preferably less than about 0.2.

Percent "identical" or "identity" in the context of two or more nucleic acid or polypeptide sequences refers to two or more sequences that are the same or have a specified percentage of nucleic acid residues or amino acid residues, respectively, that are the same, when compared and aligned for maximum similarity, as determined using a sequence comparison algorithm or by visual inspection. "Percent sequence identity" or "% identity" or "% sequence identity or "% amino acid sequence identity" of a subject amino acid sequence to a reference (*i.e.,* query) amino acid sequence means that the subject amino acid sequence is identical (*i.e.,* on an amino acid-by-amino acid basis) by a specified percentage to the query amino acid sequence over a comparison length when the sequences are optimally aligned. Thus, 80% amino acid sequence identity or 80% identity with respect to two amino acid sequences means that 80% of the amino acid residues in two optimally aligned amino acid sequences are identical.

"Percent sequence identity" or "% identity" or "% sequence identity or "% nucleotide sequence identity" of a subject nucleic acid sequence to a reference (*i.e.* query) nucleic acid sequence means that the subject nucleic acid sequence is identical (*i.e.,* on a nucleotide-by-nucleotide basis for a polynucleotide sequence) by a specified percentage to the query sequence over a comparison length when the sequences are optimally aligned. Thus, 80% nucleotide sequence identity or 80% identity with respect to two nucleic acid sequences means that 80% of the nucleotide residues in two optimally aligned nucleic acid sequences are identical.

"Percent sequence identity" or "% identity" or "% sequence identity or "% protein sequence identity" of a subject protein sequence to a reference (*i.e.* query) protein sequence means that the subject protein sequence is identical (*i.e.,* on a residue by residue basis for a polypeptide sequence) by a specified percentage to the query sequence over a comparison length when the sequences are optimally aligned. Thus, 80% protein sequence identity or 80% identity with respect to two polypeptide sequences means that 80% of the residues in two optimally aligned protein sequences are identical.

In some embodiments, the "percent sequence identity" or "% sequence identity" or "% identity" of a subject sequence to a query sequence can be calculated by optimally aligning the two sequences and comparing the two optimally aligned sequences over the comparison length. The number of positions in the optimal alignment at which identical residues occur in both sequences is determined, thereby providing the number of matched positions, and the number of matched positions is then divided by the total number of positions of the comparison length (which, unless otherwise specified, is the length of the query sequence). The resulting number is multiplied by 100 to yield the percent sequence identity of the subject sequence to the query sequence.

"Optimal alignment" or "optimally aligned" refers to the alignment of two (or more) sequences giving the highest percent identity score. For example, optimal alignment of two protein sequences can be achieved by manually aligning the sequences such that the maximum number of identical amino acid residues in each sequence are aligned together or by using software programs or procedures described herein or known in the art. Optimal alignment of two nucleic acid sequences can be achieved by manually aligning the sequences such that the maximum number of identical nucleotide residues in each sequence are aligned together or by using software programs or procedures described herein or known in the art.

In some embodiments, two polypeptide sequences are deemed "optimally aligned" when they are aligned using defined parameters, such as a defined amino acid substitution matrix, gap existence penalty (also termed gap open penalty), and gap extension penalty, so as to achieve the highest similarity score possible for that pair of sequences. The BLOSUM62 scoring matrix (*See,* Henikoff and Henikoff, *supra*) is often used as a default scoring substitution matrix in polypeptide sequence alignment algorithms (*e.g.,* BLASTP). The gap existence penalty is imposed for the introduction of a single amino acid gap in one of the aligned sequences, and the gap extension penalty is imposed for each residue position in the gap. Exemplary alignment parameters employed are: BLOSUM62 scoring matrix, gap existence penalty=11, and gap extension penalty=1. The alignment score is defined by the amino acid positions of each sequence at which the alignment begins and ends (e.g., the alignment window), and optionally by the insertion of a gap or multiple gaps into one or both sequences, so as to achieve the highest possible similarity score.

Optimal alignment between two or more sequences can be determined manually by visual inspection or by using a computer, such as, but not limited to for example, the BLASTP program for amino acid sequences and the BLASTN program for nucleic acid sequences (*See e.g.,* Altschul et al., Nucleic Acids Res. 25(17):3389-3402 (1997); *See also*, the National Center for Biotechnology Information (NCBI) website).

A polypeptide of interest may be said to be "substantially similar" to a reference polypeptide if the polypeptide of interest comprises an amino acid sequence having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 99.5% sequence identity to the amino acid sequence of the reference polypeptide. The percent identity between two such polypeptides can be determined manually by inspection of the two optimally aligned polypeptide sequences or by using software programs or algorithms (*e.g.,* BLAST, ALIGN, CLUSTAL) using standard parameters. One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative amino acid substitution or one or more conservative amino acid substitutions.

A nucleic acid of interest may be said to be "substantially similar" to a reference nucleic acid if the nucleic acid of interest comprises a nucleotide sequence having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 99.5% sequence identity to the nucleotide sequence of the reference nucleic acid. The percent identity between two such nucleic acids can be determined manually by inspection of the two optimally aligned nucleic acid sequences or by using software programs or algorithms (*e.g.,* BLAST, ALIGN, CLUSTAL) using standard parameters. One indication that two nucleic acid sequences are substantially identical is that the two nucleic acid molecules hybridize to each other under stringent conditions (*e.g.,* within a range of medium to high stringency).

A nucleic acid or polynucleotide is "isolated" when it is partially or completely separated from other components, including but not limited to for example, other proteins, nucleic acids, cells, etc. Similarly, a polypeptide, protein or peptide is "isolated" when it is partially or completely separated from other components, including but not limited to for example, other proteins, nucleic acids, cells, etc. On a molar basis, an isolated species is more abundant than are other species in a composition. For example, an isolated species may comprise at least about 50%, about 70%, about 80%, about 85%, about 90%, about 91%, about 92%, about 93%, about 94%, about 95%, about 96%, about 97%, about 98%, about 99%, or about 100% (on a molar basis) of all macromolecular species present. Preferably, the species of interest is purified to essential homogeneity (*i.e.,* contaminant species cannot be detected in the composition by conventional detection methods). Purity and homogeneity can be determined using a number of techniques well known in the art, such as agarose or polyacrylamide gel electrophoresis of a protein or nucleic acid sample, followed by visualization upon staining. If desired, a high-resolution technique, such as high performance liquid chromatography (HPLC) or a similar means can be utilized for purification of the material.

The term "purified" as applied to nucleic acids or polypeptides generally denotes a nucleic acid or polypeptide that is essentially free from other components as determined by analytical techniques well known in the art (*e.g.,* a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (*e.g.,* percent by weight on a molar basis). In a related sense, the invention provides methods of enriching compositions for one or more molecules of the invention, such as one or more polypeptides or polynucleotides of the invention. A composition is enriched for a molecule when there is a substantial increase in the concentration of the molecule after application of a purification or enrichment technique. A substantially pure polypeptide or polynucleotide of the invention (*e.g.,* substantially pure variant protease or polynucleotide encoding a variant protease of the invention, respectively) will typically comprise at least about 55%, about 60%, about 70%, about 80%, about 85%, about 90%, about 91%, about 92%, about 93%, about 94%, about 95%, about 96%, about 97%, about 98, about 99%, about 99.5% or more by weight (on a molar basis) of all macromolecular species in a particular composition.

In a related sense, the invention provides methods of enriching compositions for one or more molecules of the invention, such as one or more polypeptides of the invention (*e.g.,* one or more variant proteases of the invention) or one or more nucleic acids of the invention (*e.g.,* one or more nucleic acids encoding one or more variant proteases of the invention). A composition is enriched for a molecule when there is a substantial increase in the concentration of the molecule after application of a purification or enrichment technique. A substantially pure polypeptide or polynucleotide will typically comprise at least about 55%, about 60%, about 70%, about 80%, about 85%, about 90%, about 91%, about 92%, about 93%, about 94%, about 95%, about 96%, about 97%, about 98, about 99%, about 99.5% or more by weight (on a molar basis) of all macromolecular species in a particular composition.

As used herein, the term "combinatorial mutagenesis" or "combinatorial" refers to methods in which libraries of nucleic acid variants of a reference nucleic acid sequence are generated. In these libraries, the variants contain one or several mutations chosen from a predefined set of mutations. The methods also provide means to introduce random mutations which were not members of the predefined set of mutations. Some such methods include those set forth in U.S. Patent No. 6,582,914. Some such combinatorial mutagenesis methods include and/or encompass methods embodied in commercially available kits (*e.g*., QUIKCHANGE® Multi Site-Directed Mutagenesis Kit (Stratagene), PCR fusion/extension PCR).

As used herein, "having improved properties" used in connection with a variant protease refers to a variant protease with improved or enhanced wash or cleaning performance, and/or improved or enhanced stability optionally with retained wash or cleaning performance, relative to the corresponding reference protease (*e.g.,* wild-type or naturally-occurring protease). The improved properties of a variant protease may comprise improved wash or cleaning performance and/or improved stability. In some embodiments, the invention provides variant proteases of the invention that exhibit one of more of the following properties: improved hand wash performance, improved hand or manual dishwashing performance, improved automatic dishwashing performance, improved laundry performance, and/or improved stability relative to a reference protease (*e.g.,* wild-type protease, such as a wild-type LG12).

As used herein, the term "functional assay" refers to an assay that provides an indication of a protein's activity. In some embodiments, the term refers to assay systems in which a protein is analyzed for its ability to function in its usual capacity. For example, in the case of enzymes, a functional assay involves determining the effectiveness of the enzyme in catalyzing a reaction.

As used herein, the term "target property" refers to the property of the starting gene that is to be altered. It is not intended that the present invention be limited to any particular target property. However, in some embodiments, the target property is the stability of a gene product (*e.g.,* resistance to denaturation, proteolysis or other degradative factors), while in other embodiments, the level of production in a production host is altered.

The term "property" or grammatical equivalents thereof in the context of a nucleic acid, as used herein, refer to any characteristic or attribute of a nucleic acid that can be selected or detected. These properties include, but are not limited to, a property affecting binding to a polypeptide, a property conferred on a cell comprising a particular nucleic acid, a property affecting gene transcription (e.g., promoter strength, promoter recognition, promoter regulation, enhancer function), a property affecting RNA processing (*e.g.,* RNA splicing, RNA stability, RNA conformation, and post-transcriptional modification), a property affecting translation (*e.g.,* level, regulation, binding of mRNA to ribosomal proteins, post-translational modification). For example, a binding site for a transcription factor, polymerase, regulatory factor, etc., of a nucleic acid may be altered to produce desired characteristics or to identify undesirable characteristics.

The term "property" or grammatical equivalents thereof in the context of a polypeptide (including proteins), as used herein, refer to any characteristic or attribute of a polypeptide that can be selected or detected. These properties include, but are not limited to oxidative stability, substrate specificity, catalytic activity, enzymatic activity, thermal stability, alkaline stability, pH activity profile, resistance to proteolytic degradation, K_{M}, k_{cat}, k_{cat}/k_{M} ratio, protein folding, inducing an immune response, ability to bind to a ligand, ability to bind to a receptor, ability to be secreted, ability to be displayed on the surface of a cell, ability to oligomerize, ability to signal, ability to stimulate cell proliferation, ability to inhibit cell proliferation, ability to induce apoptosis, ability to be modified by phosphorylation or glycosylation, and/or ability to treat disease, etc.

As used herein, the term "screening" has its usual meaning in the art. In one exemplary screening process, a mutant nucleic acid or variant polypeptide encoded therefrom is provided and a property of the mutant nucleic acid or variant polypeptide, respectively, is assessed or determined. The determined property of the mutant nucleic acid or variant polypeptide may be compared to a property of the corresponding precursor (parent) nucleic acid or to the property of the corresponding parent polypeptide, respectively.

It will be apparent to the skilled artisan that the screening procedure for obtaining a nucleic acid or protein with an altered property depends upon the property of the starting material the modification of which the generation of the mutant nucleic acid is intended to facilitate. The skilled artisan will therefore appreciate that the invention is not limited to any specific property to be screened for and that the following description of properties lists illustrative examples only. Methods for screening for any particular property are generally described in the art. For example, one can measure binding, pH, specificity, etc., before and after mutation, wherein a change indicates an alteration. Preferably, the screens are performed in a high-throughput manner, including multiple samples being screened simultaneously, including, but not limited to assays utilizing chips, phage display, and multiple substrates and/or indicators.

As used herein, in some embodiments, a screening process encompasses one or more selection steps in which variants of interest are enriched from a population of variants. Examples of these embodiments include the selection of variants that confer a growth advantage to the host organism, as well as phage display or any other method of display, where variants can be captured from a population of variants based on their binding or catalytic properties. In some embodiments, a library of variants is exposed to stress (*e.g.,* heat, denaturation, etc.) and subsequently variants that are still intact are identified in a screen or enriched by selection. It is intended that the term encompass any suitable means for selection. Indeed, it is not intended that the present invention be limited to any particular method of screening.

The terms "modified nucleic acid sequence" and "modified gene" are used interchangeably herein to refer to a nucleic acid sequence that includes a deletion, insertion or interruption of naturally occurring (*i.e.,* wild-type) nucleic acid sequence. In some embodiments, the expression product of the modified nucleic acid sequence is a truncated protein (*e.g.,* if the modification is a deletion or interruption of the sequence). In some embodiments, the truncated protein retains biological activity. In alternative embodiments, the expression product of the modified nucleic acid sequence is an elongated protein (*e.g.,* modifications comprising an insertion into the nucleic acid sequence). In some embodiments, a nucleotide insertion in the nucleic acid sequence leads to a truncated protein (*e.g.,* when the insertion results in the formation of a stop codon). Thus, an insertion may result in either a truncated protein or an elongated protein as an expression product.

A "mutant" nucleic acid sequence typically refers to a nucleic acid sequence that has an alteration in at least one codon occurring in a host cell's wild-type sequence such that the expression product of the mutant nucleic acid sequence is a protein with an altered amino acid sequence relative to the wild-type protein. The expression product may have an altered functional capacity (*e.g.,* enhanced enzymatic activity).

As used herein, the phrase "alteration in substrate specificity" refers to changes in the substrate specificity of an enzyme. In some embodiments, a change in substrate specificity is defined as a change in k_{cat} and/or Kₘ for a particular substrate, resulting from mutations of the enzyme or alteration of reaction conditions. The substrate specificity of an enzyme is determined by comparing the catalytic efficiencies it exhibits with different substrates. These determinations find particular use in assessing the efficiency of mutant enzymes, as it is generally desired to produce variant enzymes that exhibit greater ratios of k_{cat}/Kₘ for substrates of interest. However, it is not intended that the present invention be limited to any particular substrate composition or substrate specificity.

As used herein, "surface property" is used in reference to electrostatic charge, as well as properties such as the hydrophobicity and hydrophilicity exhibited by the surface of a protein.

As used herein, the term "net charge" is defined as the sum of all charges present in a molecule. "Net charge changes" are made to a parent protein molecule to provide a variant that has a net charge that differs from that of the parent molecule (*i.e.,* the variant has a net charge that is not the same as that of the parent molecule). For example, substitution of a neutral amino acid with a negatively charged amino acid or a positively charged amino acid with a neutral amino acid results in net charge of -1 with respect to the parent molecule. Substitution of a positively charged amino acid with a negatively charged amino acid results in a net charge of -2 with respect to the parent. Substitution of a neutral amino acid with a positively charged amino acid or a negatively charged amino acid with a neutral amino acid results in net charge of +1 with respect to the parent. Substitution of a negatively charged amino acid with a positively charged amino acid results in a net charge of +2 with respect to the parent. The net charge of a parent protein can also be altered by deletion and/or insertion of charged amino acids

The terms "thermally stable" and "thermostable" and "thermostability" refer to proteases that retain a specified amount of enzymatic activity after exposure to identified temperatures over a given period of time under conditions prevailing during the proteolytic, hydrolyzing, cleaning or other process of the invention, while being exposed to altered temperatures. "Altered temperatures" encompass increased or decreased temperatures. In some embodiments, the proteases retain at least about 50%, about 60%, about 70%, about 75%, about 80%, about 85%, about 90%, about 92%, about 95%, about 96%, about 97%, about 98%, or about 99% proteolytic activity after exposure to altered temperatures over a given time period, for example, at least about 60 minutes, about 120 minutes, about 180 minutes, about 240 minutes, about 300 minutes, etc.

The term "enhanced stability" in the context of an oxidation, chelator, thermal and/or pH stable protease refers to a higher retained proteolytic activity over time as compared to other proteases (*e.g.,* LG12 proteases) and/or wild-type enzymes.

The term "diminished stability" in the context of an oxidation, chelator, thermal and/or pH stable protease refers to a lower retained proteolytic activity over time as compared to other proteases (*e.g.,* LG12 proteases) and/or wild-type enzymes.

The term "cleaning activity" refers to a cleaning performance achieved by a variant protease or reference protease under conditions prevailing during the proteolytic, hydrolyzing, cleaning, soil/stain removal, or other process of the invention. In some embodiments, cleaning performance of a variant protease or reference protease may be determined by using various assays for cleaning one or more various enzyme sensitive stains on an item or surface (*e.g.,* a stain resulting from food, grass, blood, ink, milk, oil, and/or egg protein). Cleaning performance of a variant or reference protease can be determined by subjecting the stain on the item or surface to standard wash condition(s) and assessing the degree to which the stain is removed by using various chromatographic, spectrophotometric, or other quantitative methodologies. Exemplary cleaning assays and methods are known in the art and include, but are not limited to those described in WO 99/34011 and U.S. Pat. 6,605,458, as well as those cleaning assays and methods included in the Examples provided below.

The term "cleaning effective amount" of a variant protease or reference protease refers to the amount of protease that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (*e.g.,* granular, tablet, bar) composition is required, etc. The term "soil removal index" (SRI) of a variant protease or reference protease refers to the degree of soil removal achieved by a certain quantity of enzyme or enzymatic activity.

The term "cleaning adjunct material" refers to any liquid, solid, or gaseous material included in cleaning composition other than a variant protease of the invention. In some embodiments, the cleaning compositions of the present invention include one of more cleaning adjunct materials. Each cleaning adjunct material is typically selected depending on the particular type and form of cleaning composition (*e.g.,* liquid, granule, powder, bar, paste, spray, tablet, gel, foam, or other composition). Preferably, each cleaning adjunct material is compatible with the protease enzyme used in the composition.

The term "enhanced performance" in the context of cleaning activity refers to an increased or greater cleaning activity by an enzyme on certain enzyme sensitive stains such as egg, milk, grass, ink, oil, and/or blood, as determined by usual evaluation after a standard wash cycle and/or multiple wash cycles.

The term "diminished performance" in the context of cleaning activity refers to a decreased or lesser cleaning activity by an enzyme on certain enzyme sensitive stains such as egg, milk, grass or blood, as determined by usual evaluation after a standard wash cycle.

Cleaning performance can be determined by comparing the variant proteases of the present invention with reference proteases in various cleaning assays concerning enzyme sensitive stains such as grass, blood, ink, oil, and/or milk as determined by usual spectrophotometric or analytical methodologies after standard wash cycle conditions.

As used herein, the term "consumer product" means fabric and home care product. As used herein, the term "fabric and home care product" or "fabric and household care product" includes products generally intended to be used or consumed in the form in which they are sold and that are for treating fabrics, hard surfaces and any other surfaces, and cleaning systems all for the care and cleaning of inanimate surfaces, as well as fabric conditioner products and other products designed specifically for the care and maintenance of fabrics, and air care products, including: air care including air fresheners and scent delivery systems, car care, pet care, livestock care, personal care, jewelry care, dishwashing, fabric conditioning (including softening and/or freshening), laundry detergency, laundry and rinse additive and/or care, pre-treatment cleaning compositions, hard surface cleaning and/or treatment including floor and toilet bowl cleaners, glass cleaners and/or treatments, tile cleaners and /or treatments, ceramic cleaners and/or treatments, and other cleaning for consumer or institutional use. In some embodiments, the fabric and home care products are suitable for use on wounds and/or skin. "Fabric and home care product" includes consumer and institutional products.

As used herein, the term "non-fabric and home care products" refers to compositions that are added to other compositions to produce an end product that may be a fabric and home care product.

As used herein, the term "institutional cleaning composition" refers to products suitable for use in institutions including but not limited to schools, hospitals, factories, stores, corporations, buildings, restaurants, office complexes and buildings, processing and/or manufacturing plants, veterinary hospitals, factory farms, factory ranches, etc.

As used herein, the term "cleaning and/or treatment composition" is a subset of fabric and home care products that includes, unless otherwise indicated, compositions suitable for cleaning and/or treating items. Such products include, but are not limited to, products for treating fabrics, hard surfaces and any other surfaces in the area of fabric and home care, including: air care including air fresheners and scent delivery systems, car care, dishwashing, fabric conditioning (including softening and/or freshening), laundry detergency, laundry and rinse additive and/or care, hard surface cleaning and/or treatment including floor and toilet bowl cleaners, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use: car or carpet shampoos, bathroom cleaners including toilet bowl cleaners; as well as cleaning auxiliaries such as bleach additives and "stain-stick" or pre-treat types, substrate-laden products such as dryer added sheets.

Indeed, as used herein, "cleaning composition" or "cleaning formulation" of the invention refers to any composition of the invention useful for removing or eliminating a compound (*e.g.,* undesired compound) from an object, item or surface to be cleaned, including, but not limited to for example, a fabric, fabric item, dishware item, tableware item, glassware item, contact lens, other solid substrate, hair (shampoo) (including human or animal hair), skin (soap or and cream), teeth (mouthwashes, toothpastes), surface of an item or object (*e.g.,* hard surfaces, such as the hard surface of a table, table top, wall, furniture item, floor, ceiling, non-dishware item, non-tableware item, etc.), filters, membranes (*e.g.,* filtration membranes, including but not limited to ultrafiltration membranes), etc. The term encompasses any material and/or added compound selected for the particular type of cleaning composition desired and the form of the product (*e.g.,* liquid, gel, granule, spray, or other composition), as long as the composition is compatible with the protease and other enzyme(s) used in the composition. The specific selection of cleaning composition materials are readily made by considering the surface, object, item, or fabric to be cleaned, and the desired form of the composition for the cleaning conditions during use.

Cleaning compositions and cleaning formulations include any composition that is suited for cleaning, bleaching, disinfecting, and/or sterilizing any object, item, and/or surface. Such compositions and formulations include, but are not limited to for example, liquid and/or solid compositions, including cleaning or detergent compositions (*e.g*., liquid, tablet, gel, bar, granule, and/or solid laundry cleaning or detergent compositions and fine fabric detergent compositions; hard surface cleaning compositions and formulations, such as for glass, wood, ceramic and metal counter tops and windows; carpet cleaners; oven cleaners; fabric fresheners; fabric softeners; and textile, laundry booster cleaning or detergent compositions, laundry additive cleaning compositions, and laundry pre-spotter cleaning compositions; dishwashing compositions, including hand or manual dishwash compositions (*e.g.,* "hand" or "manual" dishwashing detergents) and automatic dishwashing compositions (*e.g.,* "automatic dishwashing detergents").

Cleaning composition or cleaning formulations, as used herein, include, unless otherwise indicated, granular or powder-form all-purpose or heavy-duty washing agents, especially cleaning detergents; liquid, granular, gel, solid, tablet, or paste-form all-purpose washing agents, especially the so-called heavy-duty liquid (HDL) detergent or heavy-duty powder detergent (HDD) types; liquid fine-fabric detergents; hand or manual dishwashing agents, including those of the high-foaming type; hand or manual dishwashing, automatic dishwashing, or dishware or tableware washing agents, including the various tablet, powder, solid, granular, liquid, gel, and rinse-aid types for household and institutional use; liquid cleaning and disinfecting agents, including antibacterial hand-wash types, cleaning bars, mouthwashes, denture cleaners, car shampoos, carpet shampoos, bathroom cleaners; hair shampoos and/or hair-rinses for humans and other animals; shower gels and foam baths and metal cleaners; as well as cleaning auxiliaries, such as bleach additives and "stain-stick" or pre-treat types. In some embodiments, granular compositions are in "compact" form; in some embodiments, liquid compositions are in a "concentrated" form.

As used herein, "fabric cleaning compositions" include hand and machine laundry detergent compositions including laundry additive compositions and compositions suitable for use in the soaking and/or pretreatment of stained fabrics (*e.g.,* clothes, linens, and other textile materials).

As used herein, "non-fabric cleaning compositions" include non-textile (*i.e.,* non-fabric) surface cleaning compositions, including, but not limited to for example, hand or manual or automatic dishwashing detergent compositions, oral cleaning compositions, denture cleaning compositions, and personal cleansing compositions.

As used herein, the term "fabric and/or hard surface cleaning and/or treatment composition" is a subset of cleaning and treatment compositions that includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, car or carpet shampoos, bathroom cleaners including toilet bowl cleaners; fabric conditioning products including softening and/or freshening that may be in liquid, solid and/or dryer sheet form ; as well as cleaning auxiliaries such as bleach additives and "stain-stick" or pre-treat types, substrate-laden products such as dryer added sheets. All of such products which are applicable may be in standard, concentrated or even highly concentrated form even to the extent that such products may in certain aspect be non-aqueous.

As used herein, the term "detergent composition" or "detergent formulation" is used in reference to a composition intended for use in a wash medium for the cleaning of soiled or dirty objects, including particular fabric and/or non-fabric objects or items. Such compositions of the present invention are not limited to any particular detergent composition or formulation. Indeed, in some embodiments, the detergents of the invention comprise at least one variant protease of the invention and, in addition, one or more surfactants, transferase(s), hydrolytic enzymes, oxido reductases, builders (*e.g.,* a builder salt), bleaching agents, bleach activators, bluing agents, fluorescent dyes, caking inhibitors, masking agents, enzyme activators, antioxidants, and/or solubilizers. In some instances, a builder salt is a mixture of a silicate salt and a phosphate salt, preferably with more silicate (*e.g.,* sodium metasilicate) than phosphate (*e.g.,* sodium tripolyphosphate). Some compositions of the invention, such as, but not limited to, cleaning compositions or detergent compositions, do not contain any phosphate (*e.g.,* phosphate salt or phosphate builder).

As used herein, the term "bleaching" refers to the treatment of a material (*e.g.,* fabric, laundry, pulp, etc.) or surface for a sufficient length of time and/or under appropriate pH and/or temperature conditions to effect a brightening (*i.e.,* whitening) and/or cleaning of the material. Examples of chemicals suitable for bleaching include, but are not limited to, for example, ClO₂, H₂O₂, peracids, NO₂, etc.

As used herein, "wash performance" of a protease (*e.g.,* a variant protease of the invention) refers to the contribution of a variant protease to washing that provides additional cleaning performance to the detergent as compared to the detergent without the addition of the variant protease to the composition. Wash performance is compared under relevant washing conditions. In some test systems, other relevant factors, such as detergent composition, sud concentration, water hardness, washing mechanics, time, pH, and/or temperature, can be controlled in such a way that condition(s) typical for household application in a certain market segment (*e.g.,* hand or manual dishwashing, automatic dishwashing, dishware cleaning, tableware cleaning, fabric cleaning, etc.) are imitated.

The term "relevant washing conditions" is used herein to indicate the conditions, particularly washing temperature, time, washing mechanics, sud concentration, type of detergent and water hardness, actually used in households in a hand dishwashing, automatic dishwashing, or laundry detergent market segment.

The term "improved wash performance" is used to indicate that a better end result is obtained in stain removal under relevant washing conditions, or that less variant protease, on weight basis, is needed to obtain the same end result relative to the corresponding wild-type or starting parent protease.

As used herein, the term "disinfecting" refers to the removal of contaminants from the surfaces, as well as the inhibition or killing of microbes on the surfaces of items. It is not intended that the present invention be limited to any particular surface, item, or contaminant(s) or microbes to be removed.

The "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically about 17 to about 35% by weight of the total composition. In contrast, in compact compositions, the filler salt is present in amounts not exceeding about 15% of the total composition. In some embodiments, the filler salt is present in amounts that do not exceed about 10%, or more preferably, about 5%, by weight of the composition. In some embodiments, the inorganic filler salts are selected from the alkali and alkaline-earth-metal salts of sulfates and chlorides. In some embodiments, the filler salt is sodium sulfate.

The position of an amino acid residue in a given amino acid sequence is typically numbered herein using the numbering of the position of the corresponding amino acid residue of the LG12 sprC amino acid sequence shown in SEQ ID NO:3. The LG12 sprC amino acid sequence shown in SEQ ID NO:3, thus serves as a reference sequence. A given amino acid sequence, such as a variant protease amino acid sequence described herein, can be aligned with the LG12 sprC sequence (SEQ ID NO:3) using an alignment algorithm as described herein, and an amino acid residue in the given amino acid sequence that aligns (preferably optimally aligns) with an amino acid residue in the LG12 sprC sequence can be conveniently numbered by reference to the corresponding amino acid residue in the LG12 sprC sequence.

Generally, the nomenclature used herein and many of the laboratory procedures in cell culture, molecular genetics, molecular biology, nucleic acid chemistry, and protein chemistry described below are well known and commonly employed by those of ordinary skill in the art. Methods for production and manipulation of recombinant nucleic acid methods, nucleic acid synthesis, cell culture methods, and transgene incorporation (*e.g.,* transfection, electroporation) are known to those skilled in the art and are described in numerous standard texts. Oligonucleotide synthesis and purification steps are typically performed according to specifications. Techniques and procedures are generally performed according to conventional methods well known in the art and various general references that are provided throughout this document. Procedures therein are believed to be well known to those of ordinary skill in the art and are provided for the convenience of the reader.

### LG12-clade enzymes of the invention

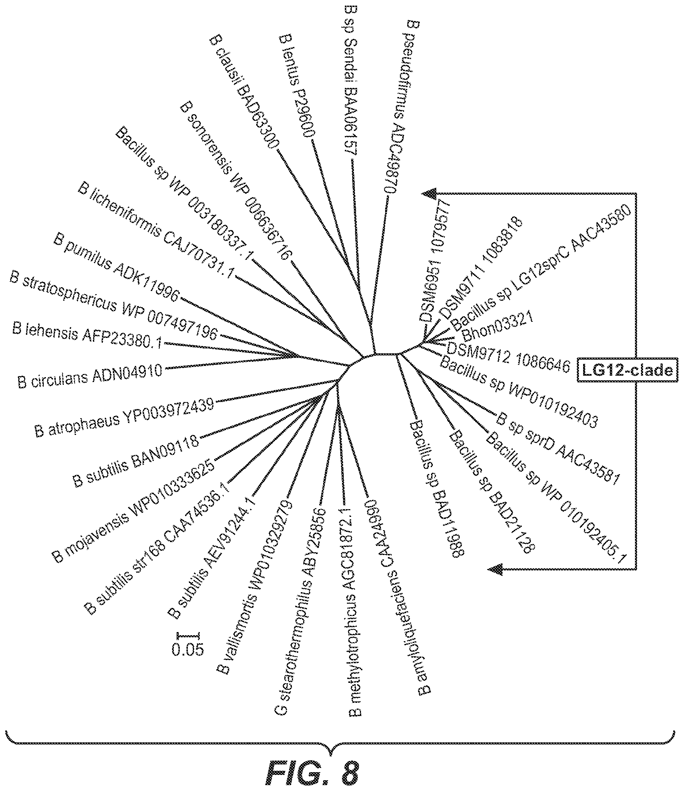

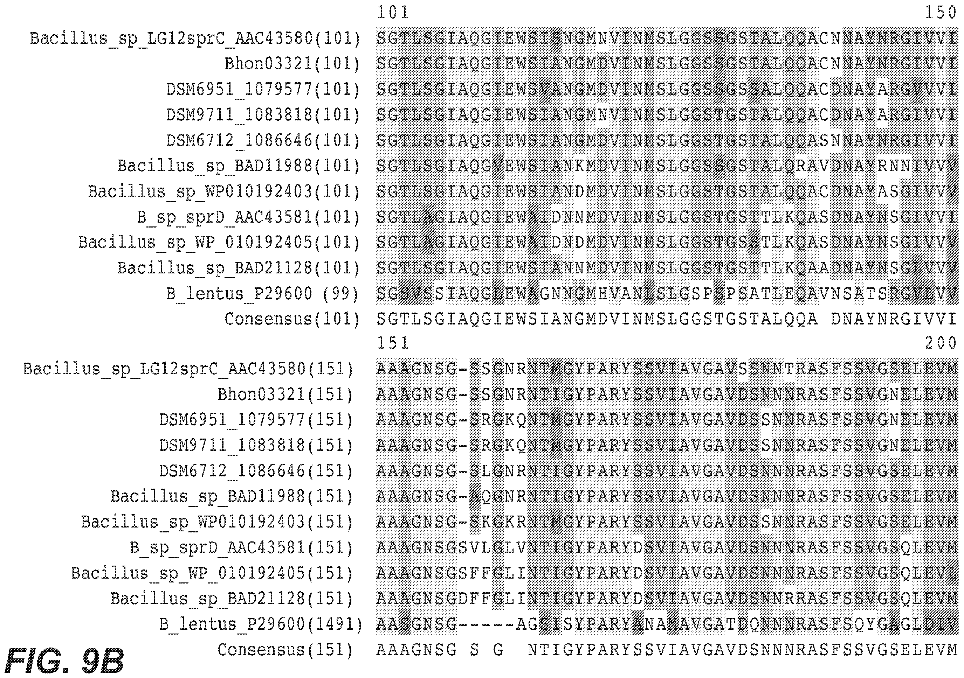

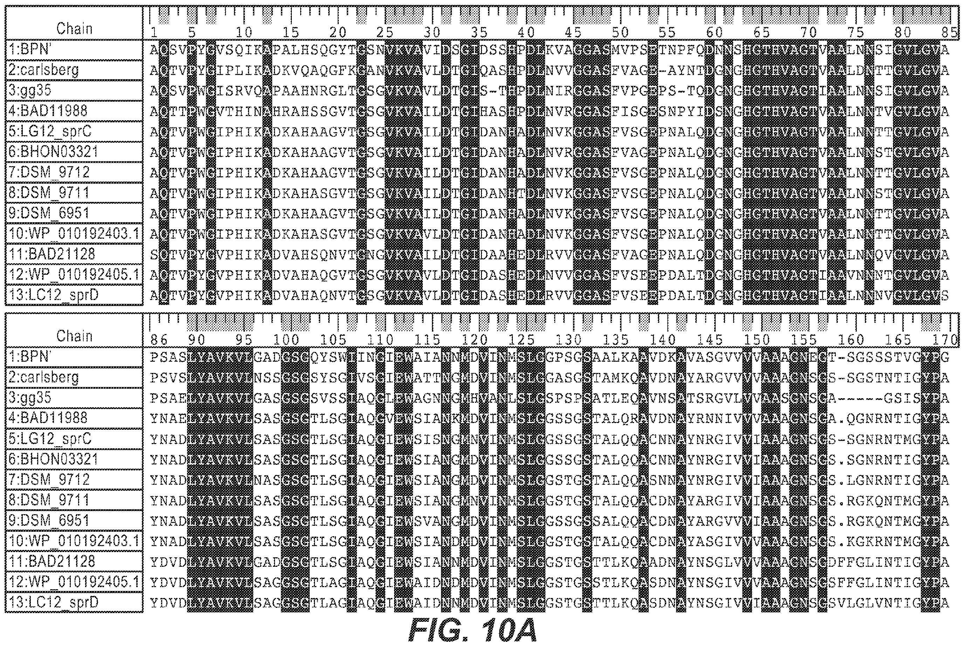

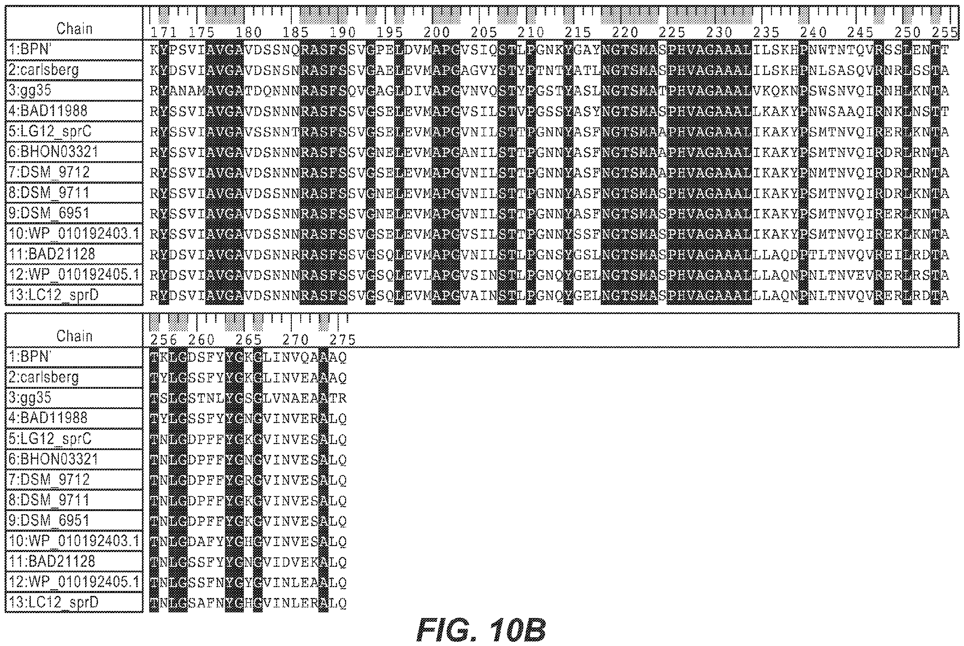

As used herein, a LG12-clade enzyme includes an enzyme, polypeptide, or protein, or an active fragment thereof, exhibiting a proteolytic activity. This includes members of the LG12-clade, as described in the Examples, alignments shown in FIG 9A, FIG 9B, FIG 10A, FIG10B and/or the phylogenetic tree of FIG 8. Members of the LG12-clade were identified by selecting subtilisin molecules with close homology to LG12 sprC, generating a structure-based alignment of these sequences to other subtilisins, and identifying regions of unique sequences conserved for all the LG12-clade enzymes. In some embodiments, the LG12 enzyme clade comprises subtilisin proteases where the parent, wildtype and/or variant sequence contains one or more of the following amino acid motifs: a catalytic triad comprising Ser221, His64, and Asp32; a Pro, Asp/Asn, Ala, Leu motif from positions 55-58; a Thr103-Leu104 motif; a Tyr86-Asn/Asp87 motif; an Ala16-His17 motif; a Val21-Thr22 motif, and/or a motif at the C-terminus, starting at position 267: Val,Ile,Asp/Asn,Val/Leu,Glu,X,Ala,Leu,Gln, where the X represents any amino acid, and where the residue numbers correspond to LG12 sprC (SEQ ID NO:3). Any of the wildtype LG12 enzyme clade sequences can be used as a parent sequence for generation of variant subtilisin enzymes of the present invention. In some embodiments, an LG12 enzyme clade subtilisin protease can have any one or more signature motif, as shown above. Members of the LG12-clade were confirmed to cluster to the same branching points in a phylogentic tree created using the Neighbor Joining method (Saitou, N. and Nei, M. (1987) MolBiol. Evol. 4:406-425). The sequence identity for the LG12-clade includes enzymes that are at least 75%, 80%, 85%, 90%, 91%, 92%, 93%, 94%, 95%, 96%, 97%, 98%, 99% or 100% identical to the LG12 sprC sequence (SEQ ID NO:3). Members of the LG12-clade include: LG12 sprC (SEQ ID NO:3, AAC43580.1), Bacillus sp. m3-13 subtilisin E(1) (SEQ ID NO:7, WP_010192403.1, formerly ZP_07707657.1), Bacillus sp. KSM-LD1, SB (BAD21128.1), Bacillus sp. m3-13 subtilisn E(2) (WP_10192405.1, formerly ZP_07707658.1), Bacillus horikoshii strain DSM 8719, Bhon03321 (SEQ ID NO:10), LG12sprD (AAC43581.1), Bacillus horikoshii strain DSM_9711 (SEQ ID NO:13), Bacillus horikoshii strain DSM_9712 (SEQ ID NO:16), Bacillus horikoshii strain DSM_6951 (SEQ ID NO:19), and Bac sp BAD21128 (SEQ ID NO:20).

The invention provides novel enzymes and variants of the LG12-clade as set out in the claims. The invention includes novel variant enzymes of the LG12-clade, wherein the variant has at least 75%, 80%, 85%, 90%, 91%, 92%, 93%, 94%, 95%, 96%, 97%, 98%, 99% or 100% identity to the LG12 sprC (SEQ ID NO:3, AAC43580.1) sequence of SEQ ID NO: 3. In some embodiments, the invention includes novel enzymes and/or variant enzymes of Bhon03321, wherein the variant has at least 75%, 80%, 85%, 90%, 91%, 92%, 93%, 94%, 95%, 96%, 97%, 98%, 99% or 100% identity to the the Bacillus sp. DSM 8719 subtilisin enzyme Bhon03321 sequence of SEQ ID NO: 10. In some embodiments, the invention includes novel enzymes and/or variant enzymes of Bacillus sp. m3-13 subtilisin E(1) (SEQ ID NO:7, WP_010192403.1, formerly ZP_07707657.1), wherein the variant has at least 75%, 80%, 85%, 90%, 91%, 92%, 93%, 94%, 95%, 96%, 97%, 98%, 99% or 100% identity to Bacillus sp. M3-13 sequence of SEQ ID NO: 7. In some embodiments, the invention includes novel enzymes and/or variant enzymes of Bac sp BAD21128, wherein the variant has at least 75%, 80%, 85%, 90%, 91%, 92%, 93%, 94%, 95%, 96%, 97%, 98%, 99% or 100% identity to Bacillus sp. KSM-LD1, SB (BAD21128.1). In some embodiments, the invention includes novel enzymes and/or variant enzymes of Bac sp WP_010192405.1, wherein the variant has at least 75%, 80%, 85%, 90%, 91%, 92%, 93%, 94%, 95%, 96%, 97%, 98%, 99% or 100% identity to Bacillus sp. m3-13 subtilisn E(2) (WP_10192405.1, formerly ZP_07707658.1). In some embodiments, the invention includes novel enzymes and/or variant enzymes of Bac sp LG12SprD AAC43581, wherein the variant has at least 75%, 80%, 85%, 90%, 91%, 92%, 93%, 94%, 95%, 96%, 97%, 98%, 99% or 100% identity to Bac sp LG12sprD (AAC43581.1). In some embodiments, the invention includes novel enzymes and/or variant enzymes of DSM_9711 (SEQ ID NO:13), wherein the variant has at least 75%, 80%, 85%, 90%, 91%, 92%, 93%, 94%, 95%, 96%, 97%, 98%, 99% or 100% identity to DSM_9711 (SEQ ID NO:13). In some embodiments, the invention includes novel enzymes and/or variant enzymes of DSM_9712 (SEQ ID NO:16), wherein the variant has at least 75%, 80%, 85%, 90%, 91%, 92%, 93%, 94%, 95%, 96%, 97%, 98%, 99% or 100% identity to DSM_9712 (SEQ ID NO:16). In some embodiments, the invention includes novel enzymes and/or variant enzymes of DSM_6951 (SEQ ID NO:19), wherein the variant has at least 75%, 80%, 85%, 90%, 91%, 92%, 93%, 94%, 95%, 96%, 97%, 98%, 99% or 100% identity to DSM_6951 (SEQ ID NO:19). In some embodiments, the invention includes novel enzymes and/or variant enzymes of Bac sp BAD21128 (SEQ ID NO:20), wherein the variant has at least 75%, 80%, 85%, 90%, 91%, 92%, 93%, 94%, 95%, 96%, 97%, 98%, 99% or 100% identity to Bac sp BAD21128 (SEQ ID NO:20).

### Productive Positions of LG12-clade enzymes

The invention provides amino acid positions in a LG12-clade enzyme, for example the LG12 sprC serine protease, which can be useful in a detergent composition where favorable modifications result in a minimum performing index for cleaning performance, stability of the enzyme in detergent compositions and thermostability of the enzyme, while having at least one of these characteristics improved from its parent LG12-clade enzyme, such as LG12 sprC of SEQ ID NO:3 in the above example. These modifications are considered suitable modifications of the invention.

The terms "thermal stability" and "thermostability" refer to LG12-clade serine proteases of the present invention that retain a specified amount of enzymatic activity after exposure to an identified temperature, often over a given period of time under conditions prevailing during the proteolytic, hydrolyzing, cleaning or other process disclosed herein, for example while exposed to altered temperatures. Altered temperatures include increased or decreased temperatures. In some embodiments, the LG12-clade protease retains at least about 50%, about 60%, about 70%, about 75%, about 80%, about 85%, about 90%, about 92%, about 95%, about 96%, about 97%, about 98%, or about 99% protease activity after exposure to altered temperatures over a given time period, for example, at least about 60 minutes, about 120 minutes, about 180 minutes, about 240 minutes, about 300 minutes, etc.

As used herein, improved properties of a LG12-clade protease enzyme includes a variant LG12-clade protease variant enzyme with improved or enhanced wash or cleaning performance, and/or improved or enhanced stability optionally with retained wash or cleaning performance, relative to the corresponding parent LG12-clade enzyme (*e.g.,* wild-type or naturally-occurring LG12-clade enzyme). The improved properties of a variant LG12-clade enzyme may comprise improved wash or cleaning performance and/or improved stability. In some embodiments, the invention provides variant LG12-clade enzymes of the invention that exhibit one of more of the following properties: improved hand wash performance, improved hand or manual dishwashing performance, improved automatic dishwashing performance, improved laundry performance, and/or improved stability relative to a reference parent LG12-clade enzyme (*e.g.,* wild-type LG12 sprC enzyme, such as a wild-type LG12 sprC protease having the mature sequence of SEQ ID NO:3).

Productive positions are described as those positions within a molecule that are most useful for making combinatorial variants exhibiting an improved characteristic, where the position itself allows for at least one combinable mutation. Combinable mutations can be described as those substitutions in a molecule that can be used to make combinatorial variants. Combinable mutations are ones that improve at least one desired property of the molecule, while not significantly decreasing either: expression, activity, or stability.

Combinable mutations are ones that improve at least one desired property of the molecule, while not significantly decreasing either: expression, activity, or stability. For example, Combinable mutations in LG12-clade proteases can be determined using performance index (PI) values resulting from the assays described in Example 1: N-suc-AAPF-pNA protease assay (activity), EMPA-116 assay (activity), PAS-38 microswatch assay (activity), detergent stability and thermostability assays, and protein determination (expression).

In addition to Combinable mutations, a second group of mutations for LG12-clade proteases is Activity Combinable mutations. Activity Combinable mutations are ones that improve at least one activity property of the molecule, with a performance index greater than or equal to 1.5, while not decreasing either expression or stability PI values below 0.5. These Activity Combinable mutations can be used to modify the molecule in order to achieve a desired property without significantly decreasing other known and desired properties of the molecule (e.g. expression or stability).

LG12-clade protease enzyme amino acid positions found to be useful positions can have different modifications that are suitable for use in a detergent composition. Modifications can include an insertion, deletion or substitution at the particular position. In one embodiment, a modification is a substitution. For each position, greater numbers of possible suitable modifications results in a higher productivity score for the position. For example, amino acid positions can have at least 50%, 30% or 15% of the modifications tested at a productive position as suitable modifications, wherein the modification meets at least one of the following suitability criteria:
a) a position wherein the minimum performance indices (PI) relative to the parent LG12-clade subtilisin enzyme for: 1) at least one cleaning assay selected from PAS-38 microswatch cleaning at pH9 or pH10; and EMPA-116 microswatch cleaning at pH6, pH8, or pH10; 2), activity on suc-AAPF-pNA; 3) protein expression; and 4) at least one stability assay selected from detergent stability and thermostability, are greater than or equal to 0.9, and in in addition have a PI for any one of these tests that is greater than or equal to 1.0;
b) a position wherein the minimum performance indices (PI) relative to the parent LG12-clade subtilisin enzyme for: 1) at least one cleaning assay selected from PAS-38 microswatch cleaning at pH9 or pH10; and EMPA-116 microswatch cleaning at pH6, pH8, or pH10; 2), activity on suc-AAPF-pNA; 3) protein expression; and 4) at least one stability assay selected from detergent stability and thermostability, are greater than or equal to 0.8, and in in addition have a PI for any one of these tests that is greater than or equal to 1.2; or
c) a position wherein the minimum performance indices (PI) relative to the parent LG12-clade subtilisin enzyme for: 1) at least one cleaning assay selected from PAS-38 microswatch cleaning at pH9 or pH10; and EMPA-116 microswatch cleaning at pH6, pH8, or pH10; 2), activity on suc-AAPF-pNA; 3) protein expression; and 4) at least one stability assay selected from detergent stability and thermostability, are greater than or equal to 0.5, and in in addition have a PI for any one of these tests that is greater than or equal to 1.5.

LG12-clade enzymes positions of the present invention comprise a modification at position 166, wherein the amino acid positions of the LG12 variant are numbered by correspondence with the amino acid sequence of LG12 sprC protease set forth in SEQ ID NO:3. The modification is selected from wherein the amino acid positions of the LG12 variant are numbered by correspondence with the amino acid sequence of LG12 sprC protease set forth in SEQ ID NO:3.

For each of the above sets of mutations, the first listed substitution represents the native amino acid for sprC (SEQ ID NO:3). LG12 variant are numbered by correspondence with the amino acid sequence of LG12 sprC protease set forth in SEQ ID NO:3.

In some embodiments of the invention, LG12-clade enzymes of the present invention have a beneficial mutation compared to the parent, wherein the benefit can be any one or more of the following: HDD PI value 1.01 or greater in: Kirkland Ultra HDD pH 10.6, or OMO Color HDD pH 10.6; HDL (pH 8) PI value 1.01 or greater in: OMO Klein & Krachtig HDL pH 8.2; HDL (pH 6) PI value 1.01 or greater in: Blue Moon pH6.5; Laundry stability PI value 1.01 or greater in detergent and buffers: OMO Klein & Krachtig HDL pH 8.2 at 51°C or LAS-EDTA buffer at 52°C; ADW PI value 1.01 or greater in detergents: GSM-B, GSM-B pH 9, GSM-C, Sun All in One, or Finish Quantum tablet; ADW stability PI value 1.01 or greater in detergent and buffers: GSM-B at 68.5°C, GSM-C at 62°C, Tris EDTA buffer at 51°C, Tris-calcium buffer at 72.5°C, as shown in the tables below. The criteria for each category include PI > 1.01 for the one or more benefits, and PI > 0.75 for expression.

In some embodiments of the invention, LG12-clade enzymes of the present invention having a beneficial mutation in laundry stability comprise substitutions from the group selected from: G166A, G166C, G166D, G166E, G166F, G166H, G166L, G166M, G166N, G166Q, G166S, G166T, G166Y,

In some embodiments of the invention, LG12-clade enzymes of the present invention having a beneficial mutation in laundry HDL pH6 performance comprise substitutions from the group selected from: G166D, G166E, G166H, G166N, G166Y,.

In some embodiments of the invention, LG12-clade enzymes of the present invention having a beneficial mutation in laundry HDL pH8 performance comprise substitutions from the group selected from:

In some embodiments of the invention, LG12-clade enzymes of the present invention having a beneficial mutation in laundry HDD performance comprise substitutions from the group selected from: embodiments of the invention, LG12-clade enzymes of the present invention having a beneficial mutation in laundry stability and laundry HDL pH6 performance comprise substitutions from the group selected from: G166D, G166E, G166H, G166N, G166Y,.

In some embodiments of the invention, LG12-clade enzymes of the present invention having a beneficial mutation in laundry stability and laundry HDL pH8 performance comprise substitutions from the group selected from: G166A, G166C, G166D, G166E, G166H, G166L, G166M, G166N, G166Q, G166S,.

In some embodiments of the invention, LG12-clade enzymes of the present invention having a beneficial mutation in laundry stability and laundry HDD performance comprise substitutions from the group selected from: G166A, G166C, G166D, G166E, G166F, G166H, G166M, G166N, G166Q, G166S, G166Y,.

In some embodiments of the invention, LG12-clade enzymes of the present invention having a beneficial mutation in laundry stability and laundry HDL pH8 and HDD comprise substitutions from the group selected from: G166A, G166C, G166D, G166E, G166H, G166M, G166N, G166Q, G166S,.

In some embodiments of the invention, LG12-clade enzymes of the present invention having a beneficial mutation in laundry stability and laundry HDD and laundry HDL pH6 and laundry HDL pH8 comprise substitutions from the group selected from: G166D, G166E, G166H, G166N,.

In some embodiments of the invention, LG12-clade enzymes of the present invention having a beneficial mutation in ADW stability comprise substitutions from the group selected from: G166A, G166D, G166E, G166F, G166H, G166I, G166L, G166M, G166N, G166Q, G166S, G166T, G166Y,.

In some embodiments of the invention, LG12-clade enzymes of the present invention having a beneficial mutation in ADW performance comprise substitutions from the group selected from: G166A, G166D, G166E, G166F, G166H, G166I, G166L, G166M, G166N, G166Q, G166S, G166T, G166Y,.

In some embodiments of the invention, LG12-clade enzymes of the present invention having a beneficial mutation in ADW stability and ADW performance comprise substitutions from the group selected from: G166A, G166D, G166E, G166F, G166H, G166I, G166L, G166M, G166N, G166Q, G166S, G166T, G166Y,.

### Polypeptides

Disclosed herein are novel polypeptides, which include isolated, recombinant, substantially pure, or non-naturally occurring variant and newly discovered LG12-clade enzyme polypeptides, including for example, variant LG12 sprC enzyme polypeptides, having enzymatic activity (*e.g.,* protease activity). In some embodiments, polypeptides are useful in cleaning applications and can be incorporated into cleaning compositions that are useful in methods of cleaning an item or a surface (*e.g.,* of surface of an item) in need of cleaning.

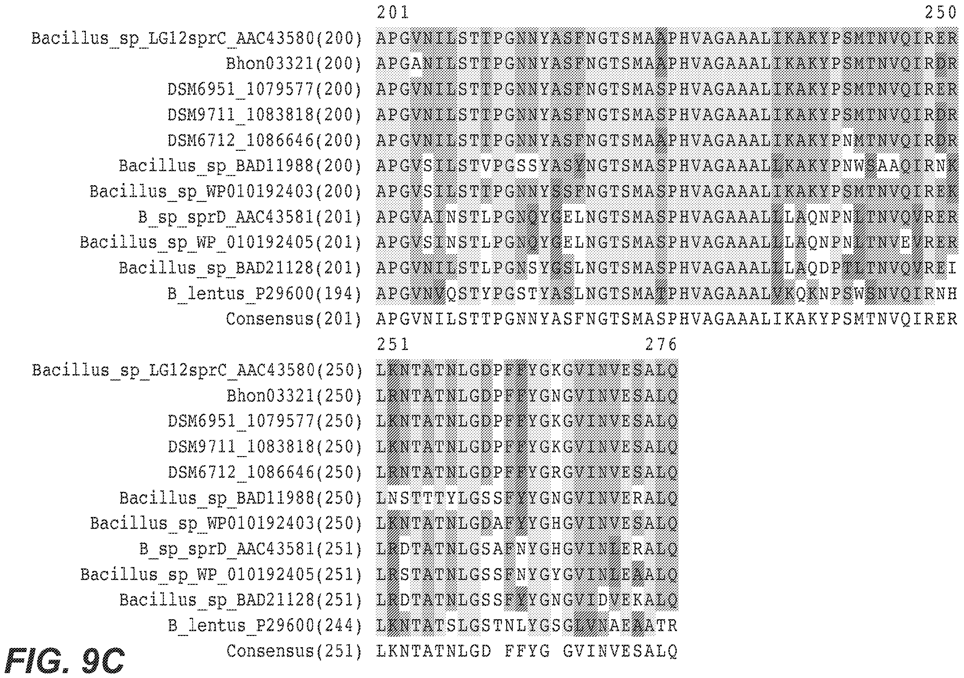

In some embodiments, the LG12-clade enzyme variant can be a variant of a parent LG12-clade enzyme from the Genus Bacillus. Various LG12-clade enzymes have been found in the genus Bacillus that have a high identity to each other and to the LG12 sprC mature enzyme from LG12 as shown in SEQ ID NO:3. See, for example, Tables 5 and 6, in Example 4 and Figure 8 in Example 11, Figure 9 in Example 12 and Figure 10 in Example 13.

In some embodiments, the LG12-clade enzyme variant can be a variant having 75, 80, 85, 90, 95, 96, 97, 98, 99 or 100% identity to any of the following: LG12 sprC (SEQ ID NO:3, AAC43580.1), Bacillus sp. m3-13 subtilisin E(1) (SEQ ID NO:7, WP_010192403.1, formerly ZP_07707657.1), Bacillus sp. KSM-LD1, SB (BAD21128.1), Bacillus sp. m3-13 subtilisn E(2) (WP_10192405.1, formerly ZP_07707658.1), Bacillus horikoshii strain DSM 8719, Bhon03321 (SEQ ID NO:10), LG12sprD (AAC43581.1), Bacillus horikoshii strain DSM_9711 (SEQ ID NO:13), Bacillus horikoshii strain DSM_9712 (SEQ ID NO:16), Bacillus horikoshii strain DSM_6951 (SEQ ID NO:19), and Bac sp BAD21128 (SEQ ID NO:20).

Described are compositions and methods relating to LG12-clade enzymes, for example, LG12 sprC cloned from LG12. The compositions and methods are based, in part, on the observation that cloned and expressed LG12 has proteolytic activity in the presence of a detergent composition. LG12 also demonstrates excellent stability in detergent compositions. These features of LG12 makes it well suited for use in a variety of cleaning applications, where the enzyme can hydrolyze proteins in the presence of surfactants and other components found in detergent compositions.

The present compositions and methods provide a variant LG12-clade polypeptide. The parent LG12-clade polypeptide was isolated from LG12. The mature LG12-clade polypeptide has the amino acid sequence of SEQ ID NO: 3.

A variant LG12 enzyme may have protease activity, which polypeptide comprises a polypeptide sequence having at least 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, at least about 99.5%, or 100% sequence identity to a parent LG12-clade enzyme as provided herein, for example, the LG12 sprC protease of SEQ ID NO:3, or to any of the following: LG12 sprC (SEQ ID NO:3, AAC43580.1), Bacillus sp. m3-13 subtilisin E(1) (SEQ ID NO:7,

WP_010192403.1, formerly ZP_07707657.1), Bacillus sp. KSM-LD1, SB (BAD21128.1), Bacillus sp. m3-13 subtilisn E(2) (WP_10192405.1, formerly ZP_07707658.1), Bacillus horikoshii strain DSM 8719, Bhon03321 (SEQ ID NO:10), LG12sprD (AAC43581.1), Bacillus horikoshii strain DSM_9711 (SEQ ID NO:13), Bacillus horikoshii strain DSM_9712 (SEQ ID NO:16), Bacillus horikoshii strain DSM_6951 (SEQ ID NO:19), and Bac sp BAD21128 (SEQ ID NO:20).

In some embodiments, the variant polypeptide is a variant having a specified degree of amino acid sequence homology to the exemplified LG12-clade polypeptide, *e*.*g*., at least 75%, at least 80%, at least 85%, at least 90%, at least 91%, at least 92%, at least 93%, at least 94%, at least 95%, at least 96%, at least 97%, at least 98%, or even at least 99% sequence homology to the amino acid sequence of SEQ ID NO: 3 or 4. Homology can be determined by amino acid sequence alignment, *e.g*., using a program such as BLAST, ALIGN, or CLUSTAL, as described herein.

Also provided is an isolated, recombinant, substantially pure, or non-naturally occurring sequence which encodes a variant LG12-clade enzyme having protease activity, said variant LG12-clade enzyme (*e.g.,* variant LG12) comprising an amino acid sequence which differs from the amino acid sequence of SEQ ID NO: 3, 7, 10, 13, 16, 19, or 20 by no more than 50, no more than 40, no more than 30, no more than 35, no more than 25, no more than 20, no more than 19, no more than 18, no more than 17, no more than 16, no more than 15, no more than 14, no more than 13, no more than 12, no more than 11, no more than 10, no more than 9, no more than 8, no more than 7, no more than 6, no more than 5, no more than 4, no more than 3, no more than 2, or no more than 1 amino acid residue(s), wherein amino acid positions of the variant LG12 are numbered according to the numbering of corresponding amino acid positions in the amino acid sequence of LG12 sprC shown in SEQ ID NO:3 as determined by alignment of the variant LG12-clade enzyme amino acid sequence with the LG12 sprC amino acid sequence.

As noted above, the variant LG12-clade enzyme polypeptides of the invention have enzymatic activities (*e.g.,* proteolytic activities) and thus are useful in cleaning applications, including but not limited to, methods for cleaning dishware items, tableware items, fabrics, and items having hard surfaces (*e.g.,* the hard surface of a table, table top, wall, furniture item, floor, ceiling, etc.). Exemplary cleaning compositions comprising one or more variant LG12-clade enzyme polypeptides of the invention are described *infra.* The enzymatic activity (*e.g.,* LG12-clade enzyme activity) of a variant LG12-clade enzyme polypeptide of the invention can be determined readily using procedures well known to those of ordinary skill in the art. The Examples presented *infra* describe methods for evaluating the enzymatic activity, cleaning performance, detergent stability and/or thermostability. The performance of variant LG12-clade enzymes of the invention in removing stains (*e.g.,* a lipid stain), cleaning hard surfaces, or cleaning laundry, dishware or tableware item(s) can be readily determined using procedures well known in the art and/or by using procedures set forth in the Examples.

A polypeptide of the invention can be subject to various changes, such as one or more amino acid insertions, deletions, and/or substitutions, either conservative or non-conservative, including where such changes do not substantially alter the enzymatic activity of the polypeptide. Similarly, a nucleic acid of the invention can also be subject to various changes, such as one or more substitutions of one or more nucleic acids in one or more codons such that a particular codon encodes the same or a different amino acid, resulting in either a silent variation (*e.g.,* mutation in a nucleotide sequence results in a silent mutation in the amino acid sequence, for example when the encoded amino acid is not altered by the nucleic acid mutation) or non-silent variation, one or more deletions of one or more nucleic acids (or codons) in the sequence, one or more additions or insertions of one or more nucleic acids (or codons) in the sequence, and/or cleavage of or one or more truncations of one or more nucleic acids (or codons) in the sequence. Many such changes in the nucleic acid sequence may not substantially alter the enzymatic activity of the resulting encoded variant LG12-clade enzyme compared to the variant LG12-clade enzyme encoded by the original nucleic acid sequence. A nucleic acid of the invention can also be modified to include one or more codons that provide for optimum expression in an expression system (*e.g.,* bacterial expression system), while, if desired, said one or more codons still encode the same amino acid(s).

In some embodiments, the present invention provides a genus of polypeptides comprising variant LG12-clade enzyme polypeptides having the desired enzymatic activity (*e.g.,* LG12-clade enzyme activity or cleaning performance activity) which comprise sequences having the amino acid substitutions described herein and also which comprise one or more additional amino acid substitutions, such as conservative and non-conservative substitutions, wherein the polypeptide exhibits, maintains, or approximately maintains the desired enzymatic activity (*e.g.,* LG12-clade enzyme activity or proteolytic activity, as reflected in the cleaning activity or performance of the variant LG12-clade enzyme). Amino acid substitutions in accordance with the invention may include, but are not limited to, one or more non-conservative substitutions and/or one or more conservative amino acid substitutions. A conservative amino acid residue substitution typically involves exchanging a member within one functional class of amino acid residues for a residue that belongs to the same functional class (identical amino acid residues are considered functionally homologous or conserved in calculating percent functional homology). A conservative amino acid substitution typically involves the substitution of an amino acid in an amino acid sequence with a functionally similar amino acid. For example, alanine, glycine, serine, and threonine are functionally similar and thus may serve as conservative amino acid substitutions for one another. Aspartic acid and glutamic acid may serve as conservative substitutions for one another. Asparagine and glutamine may serve as conservative substitutions for one another. Arginine, lysine, and histidine may serve as conservative substitutions for one another. Isoleucine, leucine, methionine, and valine may serve as conservative substitutions for one another. Phenylalanine, tyrosine, and tryptophan may serve as conservative substitutions for one another.

Other conservative amino acid substitution groups can be envisioned. For example, amino acids can be grouped by similar function or chemical structure or composition (*e.g.,* acidic, basic, aliphatic, aromatic, sulfur-containing). For instance, an aliphatic grouping may comprise: Glycine (G), Alanine (A), Valine (V), Leucine (L), Isoleucine (I). Other groups containing amino acids that are considered conservative substitutions for one another include: aromatic: Phenylalanine (F), Tyrosine (Y), Tryptophan (W); sulfur-containing: Methionine (M), Cysteine (C); Basic: Arginine (R), Lysine (K), Histidine (H); Acidic: Aspartic acid (D), Glutamic acid (E); non-polar uncharged residues, Cysteine (C), Methionine (M), and Proline (P); hydrophilic uncharged residues: Serine (S), Threonine (T), Asparagine (N), and Glutamine (Q). Additional groupings of amino acids are well-known to those of skill in the art and described in various standard textbooks. Listing of a polypeptide sequence herein, in conjunction with the above substitution groups, provides an express listing of all conservatively substituted polypeptide sequences.

More conservative substitutions exist within the amino acid residue classes described above, which also or alternatively can be suitable. Conservation groups for substitutions that are more conservative include: valine-leucine-isoleucine, phenylalanine-tyrosine, lysine-arginine, alanine-valine, and asparagine-glutamine.

Conservatively substituted variations of a polypeptide sequence of the invention (*e.g.,* variant proteases of the invention) include substitutions of a small percentage, sometimes less than 25%, 20%, 15%, 14%, 13%, 12%, 11%, 10%, 9%, 8%, 7%, or 6% of the amino acids of the polypeptide sequence, or less than 5%, 4%, 3%, 2%, or 1%, or less than 10, 9, 8, 7, 6, 5, 4, 3, 2, or 1 amino acid substitution of the amino acids of the polypeptide sequence, with a conservatively selected amino acid of the same conservative substitution group.

As described elsewhere herein in greater detail and in the Examples provided herein, polypeptides of the invention may have cleaning abilities that may be compared to known proteases, including known serine proteases.

In some embodiments, the protease variant comprises one or more mutations, and having a total net charge of -5, -4, -3, -2, -1, 0, 1, 2, 3, 4, or 5 relative to LG12 sprC (SEQ ID NO:3)

In some embodiments, the invention provides an isolated, recombinant, substantially pure, or non-naturally occurring variant protease (*e.g.,* variant LG12) having proteolytic activity, said variant protease comprising an amino acid sequence which differs from the amino acid sequence shown in SEQ ID NO: 3, 7, 10, 13, 16, 19, or 20 by no more than 50, no more than 45, no more than 40, no more than 35, no more than 30, no more than 25, no more than 20, no more than 19, no more than 18, no more than 17, no more than 16, no more than 15, no more than 14, no more than 13, no more than 12, no more than 11, no more than 10, no more than 9, no more than 6, no more than 5, no more than 4, no more than 3, no more than 2 amino acid residues, wherein amino acid positions are numbered according to the numbering of corresponding amino acid positions in the amino acid sequence of LG12 sprC shown in SEQ ID NO:3, as determined by alignment of the variant protease amino acid sequence with the LG12 sprC amino acid sequence.

### Nucleic Acids

Disclosed herein are non-naturally occurring, or recombinant nucleic acids (also referred to herein as "polynucleotides"), which encode polypeptides. Nucleic acids including all described below, are useful in recombinant production (*e.g.,* expression) of polypeptides of the invention, typically through expression of a plasmid expression vector comprising a sequence encoding the polypeptide of interest or fragment thereof. As discussed above, polypeptides include variant protease polypeptides, including variant LG12-clade polypeptides having enzymatic activity (*e.g.,* proteolytic activity) which are useful in cleaning applications and cleaning compositions for cleaning an item or a surface (*e.g.,* surface of an item) in need of cleaning.

Provided herein is an isolated, recombinant, substantially pure, or non-naturally occurring nucleic acid comprising a nucleotide sequence encoding any polypeptide (including any fusion protein, etc.) of the invention described above in the section entitled "Polypeptides of the Invention" and elsewhere herein. Also provided is an isolated, recombinant, substantially pure, or non-naturally-occurring nucleic acid comprising a nucleotide sequence encoding a combination of two or more of any polypeptides of the invention described above and elsewhere herein.

Disclosed herein is a polynucleotide encoding an isolated, recombinant, substantially pure, or non-naturally occurring variant LG12-clade enzyme having protease activity, which polypeptide comprises a polypeptide sequence having at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, at least about 99.5%, or 100% sequence identity to a parent LG12-clade enzyme as set out in the claims.

In some embodiments, the variant polypeptide is a variant having a specified degree of amino acid sequence homology to the exemplified LG12-clade polypeptide, *e.g*., at least 75%, at least 80%, at least 85%, at least 90%, at least 91%, at least 92%, at least 93%, at least 94%, at least 95%, at least 96%, at least 97%, at least 98%, or even at least 99% sequence homology to the amino acid sequence of SEQ ID NO: 3 or 4. Homology can be determined by amino acid sequence alignment, *e.g*., using a program such as BLAST, ALIGN, or CLUSTAL, as described herein.

Also provided is an isolated, recombinant, substantially pure, or non-naturally occurring nucleic acid comprising a polynucleotide sequence which encodes a variant protease having proteolytic activity, said variant protease (*e.g.,* variant LG12 sprC) comprising an amino acid sequence which differs from the amino acid sequence of SEQ ID NO: 3, 7, 10, 13, 16, 19, or 20 by no more than 50, no more than 40, no more than 30, no more than 35, no more than 25, no more than 20, no more than 19, no more than 18, no more than 17, no more than 16, no more than 15, no more than 14, no more than 13, no more than 12, no more than 11, no more than 10, no more than 9, no more than 8, no more than 7, no more than 6, no more than 5, no more than 4, no more than 3, no more than 2, or no more than 1 amino acid residue(s), wherein amino acid positions of the variant LG12-clade enzyme are numbered according to the numbering of corresponding amino acid positions in the amino acid sequence of LG12 sprC shown in SEQ ID NO:3 as determined by alignment of the variant protease amino acid sequence with the LG12 sprC amino acid sequence.

The present dislcosure provides nucleic acids encoding a LG12-clade variant of *Bacillus* LG12, wherein the LG12 variant is a mature form having proteolytic activity and comprises an amino acid sequence comprising a combination of amino acid substitutions as listed throughout the specification, wherein the amino acid positions of the LG12 variant are numbered by correspondence with the amino acid sequence of LG12 sprC set forth as SEQ ID NO:3.

Nucleic acids can be generated by using any suitable synthesis, manipulation, and/or isolation techniques, or combinations thereof. For example, a polynucleotide may be produced using standard nucleic acid synthesis techniques, such as solid-phase synthesis techniques that are well-known to those skilled in the art. In such techniques, fragments of up to 50 or more nucleotide bases are typically synthesized, then joined (*e.g.,* by enzymatic or chemical ligation methods, or polymerase mediated recombination methods) to form essentially any desired continuous nucleic acid sequence. The synthesis of the nucleic acids can be also facilitated (or alternatively accomplished) by any suitable method known in the art, including but not limited to chemical synthesis using the classical phosphoramidite method (*See e.g.,* Beaucage et al. Tetrahedron Letters 22:1859-69 [1981]); or the method described by Matthes *et al.* (*See,* Matthes et al., EMBO J. 3:801-805 [1984], as is typically practiced in automated synthetic methods. Nucleic acids also can be produced by using an automatic DNA synthesizer. Customized nucleic acids can be ordered from a variety of commercial sources (*e.g.,* The Midland Certified Reagent Company, the Great American Gene Company, Operon Technologies Inc., and DNA2.0). Other techniques for synthesizing nucleic acids and related principles are known in the art (*See e.g.,* Itakura et al., Ann. Rev. Biochem. 53:323 [1984]; and Itakura et al., Science 198:1056 [1984]).

As indicated above, recombinant DNA techniques useful in modification of nucleic acids are well known in the art. For example, techniques such as restriction endonuclease digestion, ligation, reverse transcription and cDNA production, and polymerase chain reaction (*e.g.,* PCR) are known and readily employed by those of skill in the art. Nucleotides may also be obtained by screening cDNA libraries (e.g., cDNA libraries generated using mutagenesis techniques commonly used in the art, including those described herein) using one or more oligonucleotide probes that can hybridize to or PCR-amplify polynucleotides which encode a variant protease polypeptide(s) of the invention. Procedures for screening and isolating cDNA clones and PCR amplification procedures are well known to those of skill in the art and described in standard references known to those skilled in the art. Some nucleic acids of the can be obtained by altering a naturally occurring polynucleotide backbone *(e.g.,* that encodes an enzyme or parent protease) by, for example, a known mutagenesis procedure *(e.g.,* site-directed mutagenesis, site saturation mutagenesis, and *in vitro* recombination).

### Methods for Making Modified Variant Proteases of the Invention

A variety of methods are known in the art that are suitable for generating modified polynucleotides of the invention that encode variant proteases of the invention, including, but not limited to, for example, site-saturation mutagenesis, scanning mutagenesis, insertional mutagenesis, deletion mutagenesis, random mutagenesis, site-directed mutagenesis, synthetic gene construction by oligonucleotide synthesis and ligation, and directed-evolution, as well as various other recombinatorial approaches. Methods for making modified polynucleotides and proteins (*e.g.,* variant proteases) include DNA shuffling methodologies, methods based on non-homologous recombination of genes, such as ITCHY (*See,* Ostermeier *et al.,* 7:2139-44 [1999]), SCRACHY (*See,* Lutz *et al.* 98:11248-53 [2001]), SHIPREC (*See,* Sieber *et al.,* 19:456-60 [2001]), and NRR (*See,* Bittker *et al.,* 20:1024-9 [2001]; Bittker *et al.,* 101:7011-6 [2004]), and methods that rely on the use of oligonucleotides to insert random and targeted mutations, deletions and/or insertions (*See,* Ness *et al.,* 20:1251-5 [2002]; Coco *et al.,* 20:1246-50 [2002]; Zha *et al.,* 4:34-9 [2003]; Glaser *et al.,* 149:3903-13 [1992]).

### Vectors, Cells, and Methods for Producing Variant Proteases of the Invention

Disclosed herein are isolated or recombinant vectors comprising at least one polynucleotide of the invention described herein (*e.g.,* a polynucleotide encoding a variant protease of the invention described herein), isolated or recombinant expression vectors or expression cassettes comprising at least one nucleic acid or polynucleotide of the invention, isolated, substantially pure, or recombinant DNA constructs comprising at least one nucleic acid or polynucleotide of the invention, isolated or recombinant cells comprising at least one polynucleotide of the invention, cell cultures comprising cells comprising at least one polynucleotide of the invention, cell cultures comprising at least one nucleic acid or polynucleotide of the invention, and compositions comprising one or more such vectors, nucleic acids, expression vectors, expression cassettes, DNA constructs, cells, cell cultures, or any combination or mixtures thereof.

In some embodiments, disclosed herein are recombinant cells comprising at least one vector (*e.g.,* expression vector or DNA construct) of the invention which comprises at least one nucleic acid or polynucleotide of the invention. Some such recombinant cells are transformed or transfected with such at least one vector. Such cells are typically referred to as host cells. Some such cells comprise bacterial cells, including, but are not limited to *Bacillus sp.* cells, such as *B. subtilis* cells. The disclosure also provides recombinant cells (*e.g*., recombinant host cells) comprising at least one variant protease of the invention.

Provided herein is a vector comprising a nucleic acid or polynucleotide of the invention. In some embodiments, the vector is an expression vector or expression cassette in which a polynucleotide sequence of the invention which encodes a variant protease of the invention is operably linked to one or additional nucleic acid segments required for efficient gene expression (*e.g.,* a promoter operably linked to the polynucleotide of the invention which encodes a variant protease of the invention). A vector may include a transcription terminator and/or a selection gene, such as an antibiotic resistance gene that enables continuous cultural maintenance of plasmid-infected host cells by growth in antimicrobial-containing media.

An expression vector may be derived from plasmid or viral DNA, or in alternative embodiments, contains elements of both. Exemplary vectors include, but are not limited to pXX, pC194, pJH101, pE194, pHP13 (*See,* Harwood and Cutting [eds.], Chapter 3, Molecular Biological Methods for Bacillus, John Wiley & Sons [1990]; suitable replicating plasmids for *B. subtilis* include those listed on p. 92; *See also,* Perego, Integrational Vectors for Genetic Manipulations in *Bacillus subtilis*, in Sonenshein et al., [eds.] Bacillus subtilis and Other Gram-Positive Bacteria: Biochemistry, Physiology and Molecular Genetics, American Society for Microbiology, Washington, D.C. [1993], pp. 615-624).

For expression and production of a protein of interest (*e.g.,* variant protease) in a cell, at least one expression vector comprising at least one copy of a polynucleotide encoding the modified protease, and preferably comprising multiple copies, is transformed into the cell under conditions suitable for expression of the protease. In some examples disclosed herein a polynucleotide sequence encoding the variant protease (as well as other sequences included in the vector) is integrated into the genome of the host cell, while in other examples, a plasmid vector comprising a polynucleotide sequence encoding the variant protease remains as autonomous extra-chromosomal element within the cell. Provided herein are both extrachromosomal nucleic acid elements as well as incoming nucleotide sequences that are integrated into the host cell genome. The vectors described herein are useful for production of the variant proteases of the invention. In some embodiments, a polynucleotide construct encoding the variant protease is present on an integrating vector that enables the integration and optionally the amplification of the polynucleotide encoding the variant protease into the bacterial chromosome. Examples of sites for integration are well known to those skilled in the art. In some embodiments, transcription of a polynucleotide encoding a variant protease of the invention is effectuated by a promoter that is the wild-type promoter for the selected precursor protease. In some other embodiments, the promoter is heterologous to the precursor protease, but is functional in the host cell. Specifically, examples of suitable promoters for use in bacterial host cells include, but are not limited to, for example, the AprE amyE,, amyL, pstS, sacB, pSPAC, , pVeg, pHpaII promoters, the promoter of the *B. stearothermophilus* maltogenic amylase gene, the *B. amyloliquefaciens* (BAN) amylase gene, the *B. subtilis* alkaline protease gene, the *B. clausii* alkaline protease gene the *B. pumilis* xylosidase gene, the *B. thuringiensis* cryIIIA, and the *B. licheniformis* alpha-amylase gene. Additional promoters include, but are not limited to the A4 promoter, as well as phage Lambda P_{R} or P_{L} promoters, and the *E*. *coli* lac, trp or tac promoters.

Variant proteases of the present invention can be produced in host cells of any suitable microorganism, including bacteria and fungi. For example, in some embodiments, the variant protease is produced in host cells of fungal and/or bacterial origin. In some embodiments, the host cells are *Bacillus sp., Streptomyces sp., Escherichia sp. or Aspergillus sp.* In some embodiments, the variant proteases are produced by *Bacillus sp.* host cells. Examples of *Bacillus* sp. host cells that find use in the production of the variant proteases of the invention include, but are not limited to *B. licheniformis, B. lentus, B. subtilis, B. amyloliquefaciens, B. lentus, B. brevis, B. stearothermophilus, B. alkalophilus, B. coagulans, B. circulans, B. pumilis, B. thuringiensis, B. clausii,* and *B. megaterium,* as well as other organisms within the genus *Bacillus.* In some embodiments, *B. subtilis* host cells are used for production of variant proteases. U.S. Patents 5,264,366 and 4,760,025 (RE 34,606) describe various *Bacillus* host strains that can be used for producing variant proteases of the invention, although other suitable strains can be used.

Several industrial bacterial strains that can be used to produce variant proteases of the invention include non-recombinant (*i.e.,* wild-type) *Bacillus sp.* strains, as well as variants of naturally-occurring strains and/or recombinant strains. In some embodiments, the host strain is a recombinant strain, wherein a polynucleotide encoding a polypeptide of interest has been introduced into the host. In some embodiments, the host strain is a *B. subtilis* host strain and particularly a recombinant *Bacillus subtilis* host strain. Numerous *B. subtilis* strains are known, including, but not limited to for example, 1A6 (ATCC 39085), 168 (1A01), SB19, W23, Ts85, B637, PB1753 through PB1758, PB3360, JH642, 1A243 (ATCC 39,087), ATCC 21332, ATCC 6051, MI113, DE100 (ATCC 39,094), GX4931, PBT 110, and PEP 211strain (*See e.g.,* Hoch et al., Genetics 73:215-228 [1973]; *See also*, U.S. Patent Nos. 4,450,235 and 4,302,544, and EP 0134048). The use of *B. subtilis* as an expression host cells is well known in the art (*See e.g.,* Palva et al., Gene 19:81-87 [1982]; Fahnestock and Fischer, J. Bacteriol., 165:796-804 [1986]; and Wang et al., Gene 69:39-47 [1988]).

In some embodiments, the *Bacillus* host cell is a *Bacillus* sp. that includes a mutation or deletion in at least one of the following genes, *degU*, *degS*, *degR* and *degQ.* Preferably the mutation is in a *degU* gene, and more preferably the mutation is *degU(Hy)32* (*See e.g*., Msadek et al., J. Bacteriol. 172:824-834 [1990]; and Olmos et al., Mol. Gen. Genet. 253:562-567 [1997]). One suitable host strain is a *Bacillus subtilis* carrying a *degU32(Hy)* mutation. In some embodiments, the *Bacillus* host comprises a mutation or deletion in *scoC4* (*See e.g.,* Caldwell et al., J. Bacteriol. 183:7329-7340 [2001]); *spoIIE* (*See e.g.,* Arigoni et al., Mol. Microbiol. 31:1407-1415 [1999]); and/or *oppA* or other genes of the *opp* operon *(See e.g.,* Perego et al., Mol. Microbiol. 5:173-185 [1991]). Indeed, it is contemplated that any mutation in the *opp* operon that causes the same phenotype as a mutation in the *oppA* gene will find use in some embodiments of the altered *Bacillus* strain of the invention. In some embodiments, these mutations occur alone, while in other embodiments, combinations of mutations are present. In some embodiments, an altered *Bacillus* host cell strain that can be used to produce a variant protease of the invention is a *Bacillus* host strain that already includes a mutation in one or more of the above-mentioned genes. In addition, *Bacillus* sp. host cells that comprise mutation(s) and/or deletions of endogenous protease genes find use. In some embodiments, the *Bacillus* host cell comprises a deletion of the *aprE* and the *nprE* genes. In other embodiments, the *Bacillus* sp. host cell comprises a deletion of 5 protease genes, while in other embodiments, the *Bacillus sp.* host cell comprises a deletion of 9 protease genes (*See e.g.,* U.S. Pat. Appln. Pub. No. 2005/0202535,).

Host cells are transformed with at least one nucleic acid encoding at least one variant protease of the invention using any suitable method known in the art. Whether the nucleic acid is incorporated into a vector or is used without the presence of plasmid DNA, it is typically introduced into a microorganism, in some embodiments, preferably an *E*. *coli* cell or a competent *Bacillus* cell. Methods for introducing a nucleic acid (*e.g.,* DNA) into *Bacillus* cells or *E. coli* cells utilizing plasmid DNA constructs or vectors and transforming such plasmid DNA constructs or vectors into such cells are well known. In some embodiments, the plasmids are subsequently isolated from *E. coli* cells and transformed into *Bacillus* cells. However, it is not essential to use intervening microorganisms such as *E. coli,* and in some embodiments, a DNA construct or vector is directly introduced into a *Bacillus* host.

Those of skill in the art are well aware of suitable methods for introducing nucleic acid or polynucleotide sequences of the invention into *Bacillus* cells (*See e.g.,* Ferrari et al., "Genetics," in Harwood et al. [eds.], Bacillus, Plenum Publishing Corp. [1989], pp. 57-72; Saunders et al., J. Bacteriol. 157:718-726 [1984]; Hoch et al., J. Bacteriol. 93:1925 -1937 [1967]; Mann et al., Current Microbiol. 13:131-135 [1986]; Holubova, Folia Microbiol. 30:97 [1985]; Chang et al., Mol. Gen. Genet. 168:11-115 [1979]; Vorobjeva et al., FEMS Microbiol. Lett. 7:261-263 [1980]; Smith et al., Appl. Env. Microbiol. 51:634 [1986]; Fisher et al., Arch. Microbiol. 139:213-217 [1981]; and McDonald, J. Gen. Microbiol. 130:203 [1984]). Indeed, such methods as transformation, including protoplast transformation and congression, transduction, and protoplast fusion are well known and suited for use in the present invention. Methods of transformation are used to introduce a DNA construct or vector comprising a nucleic acid encoding a variant protease of the present invention into a host cell. Methods known in the art to transform *Bacillus* cells include such methods as plasmid marker rescue transformation, which involves the uptake of a donor plasmid by competent cells carrying a partially homologous resident plasmid *(See,* Contente et al., Plasmid 2:555-571 [1979]; Haima et al., Mol. Gen. Genet. 223:185-191 [1990]; Weinrauch et al., J. Bacteriol. 154:1077-1087 [1983]; and Weinrauch et al., J. Bacteriol. 169:1205-1211 [1987]). In this method, the incoming donor plasmid recombines with the homologous region of the resident "helper" plasmid in a process that mimics chromosomal transformation.

In addition to commonly used methods, in some embodiments, host cells are directly transformed with a DNA construct or vector comprising a nucleic acid encoding a variant protease of the invention (*i.e.,* an intermediate cell is not used to amplify, or otherwise process, the DNA construct or vector prior to introduction into the host cell). Introduction of the DNA construct or vector into the host cell includes those physical and chemical methods known in the art to introduce a nucleic acid sequence (*e.g.,* DNA sequence) into a host cell without insertion into a plasmid or vector. Such methods include, but are not limited to calcium chloride precipitation, electroporation, naked DNA, liposomes and the like. In additional embodiments, DNA constructs or vector are co-transformed with a plasmid, without being inserted into the plasmid. In further embodiments, a selective marker is deleted from the altered *Bacillus* strain by methods known in the art (*See*, Stahl et al., J. Bacteriol. 158:411-418 [1984]; and Palmeros et al., Gene 247:255 -264 [2000]).

Transformed cells may be cultured in conventional nutrient media. The suitable specific culture conditions, such as temperature, pH and the like are known to those skilled in the art and are well described in the scientific literature. Also provided is a culture (*e.g.,* cell culture) comprising at least one variant protease or at least one nucleic acid of the invention. Also provided are compositions comprising at least one nucleic acid, vector, or DNA construct of the invention.

Host cells transformed with at least one polynucleotide sequence encoding at least one variant protease of the invention may be cultured in a suitable nutrient medium under conditions permitting the expression of the present protease, after which the resulting protease is recovered from the culture. The medium used to culture the cells comprises any conventional medium suitable for growing the host cells, such as minimal or complex media containing appropriate supplements. Suitable media are available from commercial suppliers or may be prepared according to published recipes (*See e.g.,* the catalogues of the American Type Culture Collection). The protease produced by the cells may be recovered from the culture medium by conventional procedures, including, but not limited to for example, separating the host cells from the medium by centrifugation or filtration, precipitating the proteinaceous components of the supernatant or filtrate by means of a salt (*e.g.,* ammonium sulfate), chromatographic purification (e.g., ion exchange, gel filtration, affinity, etc.). Any method suitable for recovering or purifying a variant protease finds use in the present invention.

A variant protease produced by a recombinant host cell may be secreted into the culture medium. A nucleic acid sequence that encodes a purification facilitating domain may be used to facilitate purification of soluble proteins. A vector or DNA construct comprising a polynucleotide sequence encoding a variant protease may further comprise a nucleic acid sequence encoding a purification facilitating domain to facilitate purification of the variant protease (*See e.g.,* Kroll et al., DNA Cell Biol. 12:441-53 [1993]). Such purification facilitating domains include, but are not limited to, for example, metal chelating peptides such as histidine-tryptophan modules that allow purification on immobilized metals (*See*, Porath, Protein Expr. Purif. 3:263-281 [1992]), protein A domains that allow purification on immobilized immunoglobulin, and the domain utilized in the FLAGS extension/affinity purification system (*e.g.,* protein A domains available from Immunex Corp., Seattle, WA). The inclusion of a cleavable linker sequence such as Factor XA or enterokinase (*e.g.,* sequences available from Invitrogen, San Diego, CA) between the purification domain and the heterologous protein also find use to facilitate purification.

Assays for detecting and measuring the enzymatic activity of an enzyme, such as a variant protease of the invention, are well known. Various assays for detecting and measuring activity of proteases (*e.g.,* variant proteases of the invention), are also known to those of ordinary skill in the art. In particular, assays are available for measuring protease activity that are based on the release of acid-soluble peptides from casein or hemoglobin, measured as absorbance at 280 nm or colorimetrically using the Folin method, well known to those skilled in the art. Other exemplary assays involve the solubilization of chromogenic substrates (*See e.g.,* Ward, "Proteinases," in Fogarty (ed.)., Microbial Enzymes and Biotechnology, Applied Science, London, [1983], pp. 251-317). Other exemplary assays include, but are not limited to succinyl-Ala-Ala-Pro-Phe-para nitroanilide assay (suc-AAPF-pNA) and the 2,4,6-trinitrobenzene sulfonate sodium salt assay (TNBS assay). Numerous additional references known to those in the art provide suitable methods (*See e.g.,* Wells et al., Nucleic Acids Res. 11:7911-7925 [1983]; Christianson et al., Anal. Biochem. 223:119 -129 [1994]; and Hsia et al., Anal Biochem. 242:221-227 [1999]).

A variety of methods can be used to determine the level of production of a mature protease (*e.g.,* mature variant proteases of the present invention) in a host cell. Such methods include, but are not limited to, for example, methods that utilize either polyclonal or monoclonal antibodies specific for the protease. Exemplary methods include, but are not limited to enzyme-linked immunosorbent assays (ELISA), radioimmunoassays (RIA), fluorescent immunoassays (FIA), and fluorescent activated cell sorting (FACS). These and other assays are well known in the art (*See e.g.,* Maddox et al., J. Exp. Med. 158:1211 [1983]).

Also disclosed herein are methods for making or producing a mature variant protease of the invention. A mature variant protease does not include a signal peptide or a propeptide sequence. Some methods comprise making or producing a variant protease of the invention in a recombinant bacterial host cell, such as for example, a *Bacillus sp.* cell (*e.g.,* a *B. subtilis* cell). Also provided is a method of producing a variant protease of the invention, the method comprising cultivating a recombinant host cell comprising a recombinant expression vector comprising a nucleic acid encoding a variant protease of the invention under conditions conducive to the production of the variant protease. Some such methods further comprise recovering the variant protease from the culture.

Also provided are methods of producing a variant protease of the invention, the methods comprising: (a) introducing a recombinant expression vector comprising a nucleic acid encoding a variant protease of the invention into a population of cells (*e.g.,* bacterial cells, such as *B. subtilis* cells); and (b) culturing the cells in a culture medium under conditions conducive to produce the variant protease encoded by the expression vector. Some such methods further comprise: (c) isolating the variant protease from the cells or from the culture medium.

### Cleaning Compositions

Unless otherwise noted, all component or composition levels provided herein are made in reference to the active level of that component or composition, and are exclusive of impurities, for example, residual solvents or by-products, which may be present in commercially available sources. Enzyme components weights are based on total active protein. All percentages and ratios are calculated by weight unless otherwise indicated. All percentages and ratios are calculated based on the total composition unless otherwise indicated. In the exemplified detergent compositions, the enzymes levels are expressed by pure enzyme by weight of the total composition and unless otherwise specified, the detergent ingredients are expressed by weight of the total compositions.

As indicated herein, in some embodiments, the cleaning compositions of the present invention further comprise adjunct materials including, but not limited to, surfactants, builders, bleaches, bleach activators, bleach catalysts, other enzymes, enzyme stabilizing systems, chelants, optical brighteners, soil release polymers, dye transfer agents, dispersants, suds suppressors, dyes, perfumes, colorants, filler salts, hydrotropes, photoactivators, fluorescers, fabric conditioners, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents (*See e.g.,* U.S. Pat. Nos. 6,610,642, 6,605,458, 5,705,464, 5,710,115, 5,698,504, 5,695,679, 5,686,014 and 5,646,101 ). Embodiments of specific cleaning composition materials are exemplified in detail below. In embodiments in which the cleaning adjunct materials are not compatible with the variant proteases of the present invention in the cleaning compositions, then suitable methods of keeping the cleaning adjunct materials and the protease(s) separated (*i.e.,* not in contact with each other) until combination of the two components is appropriate are used. Such separation methods include any suitable method known in the art (*e.g.,* gelcaps, encapsulation, tablets, physical separation, etc.).

The cleaning compositions of the present invention are advantageously employed for example, in laundry applications, hard surface cleaning, dishwashing applications, as well as cosmetic applications such as dentures, teeth, hair and skin. In addition, due to the unique advantages of increased effectiveness in lower temperature solutions, the enzymes of the present invention are ideally suited for laundry applications. Furthermore, the enzymes of the present invention find use in granular and liquid compositions.

The variant proteases of the present invention also find use in cleaning additive products. In some embodiments, low temperature solution cleaning applications find use. In some embodiments, the present invention provides cleaning additive products including at least one enzyme of the present invention is ideally suited for inclusion in a wash process when additional bleaching effectiveness is desired. Such instances include, but are not limited to low temperature solution cleaning applications. In some embodiments, the additive product is in its simplest form, one or more proteases. In some embodiments, the additive is packaged in dosage form for addition to a cleaning process. In some embodiments, the additive is packaged in dosage form for addition to a cleaning process where a source of peroxygen is employed and increased bleaching effectiveness is desired. Any suitable single dosage unit form finds use with the present invention, including but not limited to pills, tablets, gelcaps, or other single dosage units such as pre-measured powders or liquids. In some embodiments, filler(s) or carrier material(s) are included to increase the volume of such compositions. Suitable filler or carrier materials include, but are not limited to, various salts of sulfate, carbonate and silicate as well as talc, clay and the like. Suitable filler or carrier materials for liquid compositions include, but are not limited to water or low molecular weight primary and secondary alcohols including polyols and diols. Examples of such alcohols include, but are not limited to, methanol, ethanol, propanol and isopropanol. In some embodiments, the compositions contain from about 5% to about 90% of such materials. Acidic fillers find use to reduce pH. Alternatively, in some embodiments, the cleaning additive includes adjunct ingredients, as more fully described below.

The present cleaning compositions and cleaning additives require an effective amount of at least one of the protease variants provided herein, alone or in combination with other proteases and/or additional enzymes. The required level of enzyme is achieved by the addition of one or more protease variants of the present invention. Typically the present cleaning compositions comprise at least about 0.0001 weight percent, from about 0.0001 to about 10, from about 0.001 to about 1, or even from about 0.01 to about 0.1 weight percent of at least one of the variant proteases of the present invention.

The cleaning compositions herein are typically formulated such that, during use in aqueous cleaning operations, the wash water will have a pH of from about 5.0 to about 11.5 or even from about 7.5 to about 10.5. Liquid product formulations are typically formulated to have a neat pH from about 3.0 to about 9.0 or even from about 3 to about 5. Granular laundry products are typically formulated to have a pH from about 9 to about 11. Techniques for controlling pH at recommended usage levels include the use of buffers, alkalis, acids, etc., and are well known to those skilled in the art.

Suitable "low pH cleaning compositions" typically have a neat pH of from about 3 to about 5, and are typically free of surfactants that hydrolyze in such a pH environment. Such surfactants include sodium alkyl sulfate surfactants that comprise at least one ethylene oxide moiety or even from about 1 to about 16 moles of ethylene oxide. Such cleaning compositions typically comprise a sufficient amount of a pH modifier, such as sodium hydroxide, monoethanolamine or hydrochloric acid, to provide such cleaning composition with a neat pH of from about 3 to about 5. Such compositions typically comprise at least one acid stable enzyme. In some embodiments, the compositions are liquids, while in other embodiments, they are solids. The pH of such liquid compositions is typically measured as a neat pH. The pH of such solid compositions is measured as a 10% solids solution of said composition wherein the solvent is distilled water. In these embodiments, all pH measurements are taken at 20°C, unless otherwise indicated.

In some embodiments, when the variant protease(s) is/are employed in a granular composition or liquid, it is desirable for the variant protease to be in the form of an encapsulated particle to protect the variant protease from other components of the granular composition during storage. In addition, encapsulation is also a means of controlling the availability of the variant protease during the cleaning process. In some embodiments, encapsulation enhances the performance of the variant protease(s) and/or additional enzymes. In this regard, the variant proteases of the present invention are encapsulated with any suitable encapsulating material known in the art. In some embodiments, the encapsulating material typically encapsulates at least part of the catalyst for the variant protease(s) of the present invention. Typically, the encapsulating material is water-soluble and/or water-dispersible. In some embodiments, the encapsulating material has a glass transition temperature (Tg) of 0°C or higher. Glass transition temperature is described in more detail in WO 97/11151. The encapsulating material is typically selected from consisting of carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, polysaccharides, and combinations thereof. In some typical embodiments, the encapsulating material is a starch (*See e.g.,* EP 0 922 499; US 4,977,252; US 5,354,559, and US 5,935,826). In some embodiments, the encapsulating material is a microsphere made from plastic such as thermoplastics, acrylonitrile, methacrylonitrile, polyacrylonitrile, polymethacrylonitrile and mixtures thereof; commercially available microspheres that find use include, but are not limited to those supplied by EXPANCEL® (Stockviksverken, Sweden), and PM 6545, PM 6550, PM 7220, PM 7228, EXTENDOSPHERES®, LUXSIL®, Q-CEL®, and SPHERICEL® (PQ Corp., Valley Forge, PA).

As described herein, the variant proteases of the present invention find particular use in the cleaning industry, including, but not limited to laundry and dish detergents. These applications place enzymes under various environmental stresses. The variant proteases of the present invention provide advantages over many currently used enzymes, due to their stability under various conditions.

Indeed, there are a variety of wash conditions including varying detergent formulations, wash water volumes, wash water temperatures, and lengths of wash time, to which proteases involved in washing are exposed. In addition, detergent formulations used in different geographical areas have different concentrations of their relevant components present in the wash water. For example, European detergents typically have about 4500-5000 ppm of detergent components in the wash water, while Japanese detergents typically have approximately 667 ppm of detergent components in the wash water. In North America, particularly the United States, detergents typically have about 975 ppm of detergent components present in the wash water.

A low detergent concentration system includes detergents where less than about 800 ppm of the detergent components are present in the wash water. Japanese detergents are typically considered low detergent concentration system as they have approximately 667 ppm of detergent components present in the wash water.

A medium detergent concentration includes detergents where between about 800 ppm and about 2000ppm of the detergent components are present in the wash water. North American detergents are generally considered to be medium detergent concentration systems as they have approximately 975 ppm of detergent components present in the wash water. Brazil typically has approximately 1500 ppm of detergent components present in the wash water.

A high detergent concentration system includes detergents where greater than about 2000 ppm of the detergent components are present in the wash water. European detergents are generally considered to be high detergent concentration systems as they have approximately 4500-5000 ppm of detergent components in the wash water.

Latin American detergents are generally high suds phosphate builder detergents and the range of detergents used in Latin America can fall in both the medium and high detergent concentrations as they range from 1500 ppm to 6000 ppm of detergent components in the wash water. As mentioned above, Brazil typically has approximately 1500 ppm of detergent components present in the wash water. However, other high suds phosphate builder detergent geographies, not limited to other Latin American countries, may have high detergent concentration systems up to about 6000 ppm of detergent components present in the wash water.

In light of the foregoing, it is evident that concentrations of detergent compositions in typical wash solutions throughout the world varies from less than about 800 ppm of detergent composition ("low detergent concentration geographies"), for example about 667 ppm in Japan, to between about 800 ppm to about 2000 ppm ("medium detergent concentration geographies"), for example about 975 ppm in U.S. and about 1500 ppm in Brazil, to greater than about 2000 ppm ("high detergent concentration geographies"), for example about 4500 ppm to about 5000 ppm in Europe and about 6000 ppm in high suds phosphate builder geographies.

The concentrations of the typical wash solutions are determined empirically. For example, in the U.S., a typical washing machine holds a volume of about 64.4 L of wash solution. Accordingly, in order to obtain a concentration of about 975 ppm of detergent within the wash solution about 62.79 g of detergent composition must be added to the 64.4 L of wash solution. This amount is the typical amount measured into the wash water by the consumer using the measuring cup provided with the detergent.

As a further example, different geographies use different wash temperatures. The temperature of the wash water in Japan is typically less than that used in Europe. For example, the temperature of the wash water in North America and Japan is typically between about 10 and about 30°C (*e.g.,* about 20°C), whereas the temperature of wash water in Europe is typically between about 30 and about 60°C (*e.g.,* about 40°C). However, in the interest of saving energy, many consumers are switching to using cold water washing. In addition, in some further regions, cold water is typically used for laundry, as well as dish washing applications. In some embodiments, the "cold water washing" of the present invention utilizes "cold water detergent" suitable for washing at temperatures from about 10°C to about 40°C, or from about 20°C to about 30°C, or from about 15°C to about 25°C, as well as all other combinations within the range of about 15°C to about 35°C, and all ranges within 10°C to 40°C.

As a further example, different geographies typically have different water hardness. Water hardness is usually described in terms of the grains per gallon mixed Ca²⁺/Mg²⁺. Hardness is a measure of the amount of calcium (Ca²⁺) and magnesium (Mg²⁺) in the water. Most water in the United States is hard, but the degree of hardness varies. Moderately hard (60-120 ppm) to hard (121-181 ppm) water has 60 to 181 parts per million (parts per million converted to grains per U.S. gallon is ppm # divided by 17.1 equals grains per gallon) of hardness minerals.

| **Water** | **Grains per gallon** | **Parts per million** |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

European water hardness is typically greater than about 10.5 (for example about 10.5 to about 20.0) grains per gallon mixed Ca²⁺/Mg²⁺ (*e.g.,* about 15 grains per gallon mixed Ca²⁺/Mg²⁺). North American water hardness is typically greater than Japanese water hardness, but less than European water hardness. For example, North American water hardness can be between about 3 to about 10 grains, about 3 to about 8 grains or about 6 grains. Japanese water hardness is typically lower than North American water hardness, usually less than about 4, for example about 3 grains per gallon mixed Ca²⁺/Mg²⁺.

Accordingly, in some embodiments, the present invention provides variant proteases that show surprising wash performance in at least one set of wash conditions (*e.g.,* water temperature, water hardness, and/or detergent concentration). In some embodiments, the variant proteases of the present invention are comparable in wash performance to other LG12 proteases. In some embodiments of the present invention, the variant proteases provided herein exhibit enhanced oxidative stability, enhanced thermal stability, enhanced cleaning capabilities under various conditions, and/or enhanced chelator stability. In addition, the variant proteases of the present invention find use in cleaning compositions that do not include detergents, again either alone or in combination with builders and stabilizers.

In some embodiments of the present invention, the cleaning compositions comprise at least one variant protease of the present invention at a level from about 0.00001 % to about 10% by weight of the composition and the balance (*e.g.,* about 99.999% to about 90.0%) comprising cleaning adjunct materials by weight of composition. In some other embodiments of the present invention, the cleaning compositions of the present invention comprises at least one variant protease at a level of about 0.0001 % to about 10%, about 0.001% to about 5%, about 0.001% to about 2%, about 0.005% to about 0.5% by weight of the composition and the balance of the cleaning composition (*e.g.,* about 99.9999% to about 90.0%, about 99.999 % to about 98%, about 99.995% to about 99.5% by weight) comprising cleaning adjunct materials.

In some embodiments, the cleaning compositions of the present invention comprise one or more additional detergent enzymes, which provide cleaning performance and/or fabric care and/or dishwashing benefits. Examples of suitable enzymes include, but are not limited to, hemicellulases, cellulases, peroxidases, proteases, xylanases, lipases, phospholipases, esterases, cutinases, pectinases, pectate lyases, mannanases, keratinases, reductases, oxidases, phenoloxidases, lipoxygenases, ligninases, pullulanases, tannases, pentosanases, malanases, β-glucanases, arabinosidases, hyaluronidases, chondroitinases, laccases, and amylases, or any combinations or mixtures thereof. In some embodiments, a combination of enzymes is used (*i.e.,* a "cocktail") comprising conventional applicable enzymes like protease, lipase, cutinase and/or cellulase in conjunction with amylase is used.

In addition to the protease variants provided herein, any other suitable protease finds use in the compositions of the present invention. Suitable proteases include those of animal, vegetable or microbial origin. In some embodiments, microbial proteases are used. In some embodiments, chemically or genetically modified mutants are included. In some embodiments, the protease is a serine protease, preferably an alkaline microbial protease or a trypsin-like protease. Examples of alkaline proteases include subtilisins, especially those derived from *Bacillus* (*e.g.,* subtilisin, *lentus, amyloliquefaciens,* subtilisin Carlsberg, subtilisin 309, subtilisin 147 and subtilisin 168). Additional examples include those mutant proteases described in U.S. Pat. Nos. RE 34,606, 5,955,340, 5,700,676, 6,312,936, and 6,482,628. Additional protease examples include, but are not limited to trypsin (*e.g.,* of porcine or bovine origin), and the *Fusarium* protease described in WO 89/06270. In some embodiments, commercially available protease enzymes that find use in the present invention include, but are not limited to MAXATASE®, MAXACAL™, MAXAPEM™, OPTICLEAN®, OPTIMASE®, PROPERASE®, PURAFECT®, PURAFECT® OXP, PURAMAX™, EXCELLASE™, and PURAFAST™ (Genencor); ALCALASE®, SAVINASE®, PRIMASE®, DURAZYM™, POLARZYME®, OVOZYME®, KANNASE®, LIQUANASE®, NEUTRASE®, RELASE® and ESPERASE® (Novozymes); BLAP™ and BLAP™ variants (Henkel Kommanditgesellschaft auf Aktien, Duesseldorf, Germany), and KAP (B. alkalophilus subtilisin; Kao Corp., Tokyo, Japan). Various proteases are described in WO95/23221, WO 92/21760, U.S. Pat. Publ. No. 2008/0090747, and U.S. Pat. Nos. 5,801,039, 5,340,735, 5,500,364, 5,855,625, US RE 34,606, 5,955,340, 5,700,676, 6,312,936, and 6,482,628, and various other patents. In some further embodiments, serine proteases find use in the present invention, including but not limited to the neutral serine protease described in WO 07/044993.

In addition, any suitable lipase finds use in the present invention. Suitable lipases include, but are not limited to those of bacterial or fungal origin. Chemically or genetically modified mutants are encompassed by the present invention. Examples of useful lipases include *Humicola lanuginosa* lipase *(See e.g.,* EP 258 068, and EP 305 216), *Rhizomucor miehei* lipase (*See e.g.,* EP 238 023), *Candida* lipase, such as *C*. *antarctica* lipase (*e.g.,* the C. *antarctica* lipase A or B; *See e.g.,* EP 214 761), *Pseudomonas* lipases such as *P. alcaligenes* lipase and *P. pseudoalcaligenes* lipase *(See e.g.,* EP 218 272), *P. cepacia* lipase (*See e.g.,* EP 331 376), *P. stutzeri* lipase (*See e.g.,* GB 1,372,034), *P. fluorescens* lipase, *Bacillus* lipase (*e.g., B. subtilis* lipase [Dartois et al., Biochem. Biophys. Acta 1131:253-260 [1993]); *B. stearothermophilus* lipase [*See e.g.,* JP 64/744992]; and *B. pumilus* lipase [*See e.g.,* WO 91/16422]).

Furthermore, a number of cloned lipases find use in some embodiments of the present invention, including but not limited to *Penicillium camembertii* lipase (*See,* Yamaguchi et al., Gene 103:61-67 [1991]), *Geotricum candidum* lipase (*See,* Schimada et al., J. Biochem., 106:383-388 [1989]), and various *Rhizopus* lipases such as *R. delemar* lipase (*See,* Hass et al., Gene 109:117-113 [1991]), a *R. niveus* lipase (Kugimiya et al., Biosci. Biotech. Biochem. 56:716-719 [1992]) and *R. oryzae* lipase.

Other types of lipolytic enzymes such as cutinases also find use in some embodiments of the present invention, including but not limited to the cutinase derived from *Pseudomonas mendocina* (*See,* WO 88/09367), and the cutinase derived from *Fusarium solani pisi* (*See*, WO 90/09446).

Additional suitable lipases include commercially available lipases such as M1 LIPASE™, LUMA FAST™, and LIPOMAX™ (Genencor); LIPEX®, LIPOLASE® and LIPOLASE® ULTRA (Novozymes); and LIPASE P™ "Amano" (Amano Pharmaceutical Co. Ltd., Japan).

In some embodiments of the present invention, the cleaning compositions of the present invention further comprise lipases at a level from about 0.00001 % to about 10% of additional lipase by weight of the composition and the balance of cleaning adjunct materials by weight of composition. In some other embodiments of the present invention, the cleaning compositions of the present invention also comprise lipases at a level of about 0.0001 % to about 10%, about 0.001% to about 5%, about 0.001% to about 2%, about 0.005% to about 0.5% lipase by weight of the composition.

In some embodiments of the present invention, any suitable amylase finds use in the present invention. In some embodiments, any amylase (*e.g.,* alpha and/or beta) suitable for use in alkaline solutions also find use. Suitable amylases include, but are not limited to those of bacterial or fungal origin. Chemically or genetically modified mutants are included in some embodiments. Amylases that find use in the present invention, include, but are not limited to α-amylases obtained from *B. licheniformis* (*See e.g.,* GB 1,296,839). Commercially available amylases that find use in the present invention include, but are not limited to DURAMYL®, TERMAMYL®, FUNGAMYL®, STAINZYME®, STAINZYME PLUS®, STAINZYME ULTRA®, and BAN™ (Novozymes), as well as POWERASE™, RAPIDASE® and MAXAMYL® P (Genencor).

In some embodiments of the present invention, the cleaning compositions of the present invention further comprise amylases at a level from about 0.00001 % to about 10% of additional amylase by weight of the composition and the balance of cleaning adjunct materials by weight of composition. In some other embodiments of the present invention, the cleaning compositions of the present invention also comprise amylases at a level of about 0.0001 % to about 10%, about 0.001% to about 5%, about 0.001% to about 2%, about 0.005% to about 0.5% amylase by weight of the composition.

In some further embodiments, any suitable cellulase finds used in the cleaning compositions of the present invention. Suitable cellulases include, but are not limited to those of bacterial or fungal origin. Chemically or genetically modified mutants are included in some embodiments. Suitable cellulases include, but are not limited to *Humicola insolens* cellulases (*See e.g.,* U.S. Pat. No. 4,435,307). Especially suitable cellulases are the cellulases having color care benefits (*See e.g.,* EP 0 495 257). Commercially available cellulases that find use in the present include, but are not limited to CELLUZYME®, CAREZYME® (Novozymes), and KAC-500(B)™ (Kao Corporation). In some embodiments, cellulases are incorporated as portions or fragments of mature wild-type or variant cellulases, wherein a portion of the N-terminus is deleted (*See e.g.,* U.S. Pat. No. 5,874,276). In some embodiments, the cleaning compositions of the present invention further comprise cellulases at a level from about 0.00001 % to about 10% of additional cellulase by weight of the composition and the balance of cleaning adjunct materials by weight of composition. In some other embodiments of the present invention, the cleaning compositions of the present invention also comprise cellulases at a level of about 0.0001 % to about 10%, about 0.001% to about 5%, about 0.001% to about 2%, about 0.005% to about 0.5% cellulase by weight of the composition.

Any mannanase suitable for use in detergent compositions also finds use in the present invention. Suitable mannanases include, but are not limited to those of bacterial or fungal origin. Chemically or genetically modified mutants are included in some embodiments. Various mannanases are known which find use in the present invention (*See e.g.,* U.S. Pat. No. 6,566,114, U.S. Pat. No.6,602,842, and US Patent No. 6,440,991,). In some embodiments, the cleaning compositions of the present invention further comprise mannanases at a level from about 0.00001 % to about 10% of additional mannanase by weight of the composition and the balance of cleaning adjunct materials by weight of composition. In some embodiments of the present invention, the cleaning compositions of the present invention also comprise mannanases at a level of about 0.0001 % to about 10%, about 0.001% to about 5%, about 0.001% to about 2%, about 0.005% to about 0.5% mannanase by weight of the composition.

In some embodiments, peroxidases are used in combination with hydrogen peroxide or a source thereof (*e.g.,* a percarbonate, perborate or persulfate) in the compositions of the present invention. In some alternative embodiments, oxidases are used in combination with oxygen. Both types of enzymes are used for "solution bleaching" (*i.e.,* to prevent transfer of a textile dye from a dyed fabric to another fabric when the fabrics are washed together in a wash liquor), preferably together with an enhancing agent (*See e.g.,* WO 94/12621 and WO 95/01426). Suitable peroxidases/oxidases include, but are not limited to those of plant, bacterial or fungal origin. Chemically or genetically modified mutants are included in some embodiments. In some embodiments, the cleaning compositions of the present invention further comprise peroxidase and/or oxidase enzymes at a level from about 0.00001 % to about 10% of additional peroxidase and/or oxidase by weight of the composition and the balance of cleaning adjunct materials by weight of composition. In some other embodiments of the present invention, the cleaning compositions of the present invention also comprise, peroxidase and/or oxidase enzymes at a level of about 0.0001 % to about 10%, about 0.001% to about 5%, about 0.001% to about 2%, about 0.005% to about 0.5% peroxidase and/or oxidase enzymes by weight of the composition.

In some embodiments, additional enzymes find use, including but not limited to perhydrolases (*See e.g.,* WO 05/056782). In addition, in some embodiments, mixtures of the above mentioned enzymes are encompassed herein, in particular one or more additional protease, amylase, lipase, mannanase, and/or at least one cellulase. Indeed, it is contemplated that various mixtures of these enzymes will find use in the present invention. It is also contemplated that the varying levels of the variant protease(s) and one or more additional enzymes may both independently range to about 10%, the balance of the cleaning composition being cleaning adjunct materials. The specific selection of cleaning adjunct materials are readily made by considering the surface, item, or fabric to be cleaned, and the desired form of the composition for the cleaning conditions during use (*e.g.,* through the wash detergent use).

Examples of suitable cleaning adjunct materials include, but are not limited to, surfactants, builders, bleaches, bleach activators, bleach catalysts, other enzymes, enzyme stabilizing systems, chelants, optical brighteners, soil release polymers, dye transfer agents, dye transfer inhibiting agents, catalytic materials, hydrogen peroxide, sources of hydrogen peroxide, preformed peracids, polymeric dispersing agents, clay soil removal agents, structure elasticizing agents, dispersants, suds suppressors, dyes, perfumes, colorants, filler salts, hydrotropes, photoactivators, fluorescers, fabric conditioners, fabric softeners, carriers, hydrotropes, processing aids, solvents, pigments, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents (*See e.g.,* U.S. Pat. Nos. 6,610,642, 6,605,458, 5,705,464, 5,710,115, 5,698,504, 5,695,679, 5,686,014 and 5,646,101, ). Embodiments of specific cleaning composition materials are exemplified in detail below. In embodiments in which the cleaning adjunct materials are not compatible with the variant proteases of the present invention in the cleaning compositions, then suitable methods of keeping the cleaning adjunct materials and the protease(s) separated (*i.e.,* not in contact with each other) until combination of the two components is appropriate are used. Such separation methods include any suitable method known in the art (*e.g.,* gelcaps, encapsulation, tablets, physical separation, etc.).

In some embodiments, an effective amount of one or more variant protease(s) provided herein is included in compositions useful for cleaning a variety of surfaces in need of proteinaceous stain removal. Such cleaning compositions include cleaning compositions for such applications as cleaning hard surfaces, fabrics, and dishes. Indeed, in some embodiments, the present invention provides fabric cleaning compositions, while in other embodiments, the present invention provides non-fabric cleaning compositions. Notably, the present invention also provides cleaning compositions suitable for personal care, including oral care (including dentrifices, toothpastes, mouthwashes, etc., as well as denture cleaning compositions), skin, and hair cleaning compositions. It is intended that the present invention encompass detergent compositions in any form (*i.e.,* liquid, granular, bar, semi-solid, gels, emulsions, tablets, capsules, etc.).

By way of example, several cleaning compositions wherein the variant proteases of the present invention find use are described in greater detail below. In some embodiments in which the cleaning compositions of the present invention are formulated as compositions suitable for use in laundry machine washing method(s), the compositions of the present invention preferably contain at least one surfactant and at least one builder compound, as well as one or more cleaning adjunct materials preferably selected from organic polymeric compounds, bleaching agents, additional enzymes, suds suppressors, dispersants, lime-soap dispersants, soil suspension and anti-redeposition agents and corrosion inhibitors. In some embodiments, laundry compositions also contain softening agents (*i.e.,* as additional cleaning adjunct materials). The compositions of the present invention also find use detergent additive products in solid or liquid form. Such additive products are intended to supplement and/or boost the performance of conventional detergent compositions and can be added at any stage of the cleaning process. In some embodiments, the density of the laundry detergent compositions herein ranges from about 400 to about 1200 g/liter, while in other embodiments, it ranges from about 500 to about 950 g/liter of composition measured at 20°C.

In embodiments formulated as compositions for use in manual dishwashing methods, the compositions of the invention preferably contain at least one surfactant and preferably at least one additional cleaning adjunct material selected from organic polymeric compounds, suds enhancing agents, group II metal ions, solvents, hydrotropes and additional enzymes.

In some embodiments, various cleaning compositions such as those provided in U.S, Pat. No. 6,605,458, find use with the variant proteases of the present invention. Thus, in some embodiments, the compositions comprising at least one variant protease of the present invention is a compact granular fabric cleaning composition, while in other embodiments, the composition is a granular fabric cleaning composition useful in the laundering of colored fabrics, in further embodiments, the composition is a granular fabric cleaning composition which provides softening through the wash capacity, in additional embodiments, the composition is a heavy duty liquid fabric cleaning composition. In some embodiments, the compositions comprising at least one variant protease of the present invention are fabric cleaning compositions such as those described in U.S. Pat. Nos. 6,610,642 and 6,376,450. In addition, the variant proteases of the present invention find use in granular laundry detergent compositions of particular utility under European or Japanese washing conditions (*See e.g.,* U.S. Pat. No. 6,610,642).

In some alternative embodiments, the present invention provides hard surface cleaning compositions comprising at least one variant protease provided herein. Thus, in some embodiments, the compositions comprising at least one variant protease of the present invention is a hard surface cleaning composition such as those described in U.S. Pat. Nos. 6,610,642, 6,376,450, and 6,376,450.

In yet further embodiments, the present invention provides dishwashing compositions comprising at least one variant protease provided herein. Thus, in some embodiments, the compositions comprising at least one variant protease of the present invention is a hard surface cleaning composition such as those in U.S. Pat. Nos. 6,610,642 and 6,376,450. In some still further embodiments, the present invention provides dishwashing compositions comprising at least one variant protease provided herein. In some further embodiments, the compositions comprising at least one variant protease of the present invention comprise oral care compositions such as those in U.S. Pat. No. 6,376,450, and 6,376,450. The formulations and descriptions of the compounds and cleaning adjunct materials contained in the aforementioned US Pat. Nos. 6,376,450, 6,605,458, 6,605,458, and 6,610,642, find use with the variant proteases provided herein.

The cleaning compositions of the present invention are formulated into any suitable form and prepared by any process chosen by the formulator, non-limiting examples of which are described in U.S. Pat. Nos. 5,879,584, 5,691,297, 5,574,005, 5,569,645, 5,565,422, 5,516,448, 5,489,392, and 5,486,303,. When a low pH cleaning composition is desired, the pH of such composition is adjusted via the addition of a material such as monoethanolamine or an acidic material such as HCl.

While not essential for the purposes of the present invention, the non-limiting list of adjuncts illustrated hereinafter are suitable for use in the instant cleaning compositions. In some embodiments, these adjuncts are incorporated for example, to assist or enhance cleaning performance, for treatment of the substrate to be cleaned, or to modify the aesthetics of the cleaning composition as is the case with perfumes, colorants, dyes or the like. It is understood that such adjuncts are in addition to the variant proteases of the present invention. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments. In addition to the disclosure below, suitable examples of such other adjuncts and levels of use are found in U.S. Patent Nos. 5,576,282, 6,306,812, and 6,326,348. The aforementioned adjunct ingredients may constitute the balance of the cleaning compositions of the present invention.

In some embodiments, the cleaning compositions according to the present invention comprise at least one surfactant and/or a surfactant system wherein the surfactant is selected from nonionic surfactants, anionic surfactants, cationic surfactants, ampholytic surfactants, zwitterionic surfactants, semi-polar nonionic surfactants and mixtures thereof. In some low pH cleaning composition embodiments (*e.g.,* compositions having a neat pH of from about 3 to about 5), the composition typically does not contain alkyl ethoxylated sulfate, as it is believed that such surfactant may be hydrolyzed by such compositions the acidic contents. In some embodiments, the surfactant is present at a level of from about 0.1% to about 60%, while in alternative embodiments the level is from about 1% to about 50%, while in still further embodiments the level is from about 5% to about 40%, by weight of the cleaning composition.

In some embodiments, the cleaning compositions of the present invention comprise one or more detergent builders or builder systems. In some embodiments incorporating at least one builder, the cleaning compositions comprise at least about 1%, from about 3% to about 60% or even from about 5% to about 40% builder by weight of the cleaning composition. Builders include, but are not limited to, the alkali metal, ammonium and alkanolammonium salts of polyphosphates, alkali metal silicates, alkaline earth and alkali metal carbonates, aluminosilicates, polycarboxylate compounds, ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof. Indeed, it is contemplated that any suitable builder will find use in various embodiments of the present invention.

In some embodiments, the builders form water-soluble hardness ion complexes (e.g., sequestering builders), such as citrates and polyphosphates (*e.g*., sodium tripolyphosphate and sodium tripolyphospate hexahydrate, potassium tripolyphosphate, and mixed sodium and potassium tripolyphosphate, etc.). It is contemplated that any suitable builder will find use in the present invention, including those known in the art (*See e.g.,* EP 2 100 949).

In some embodiments, the cleaning compositions of the present invention contain at least one chelating agent. Suitable chelating agents include, but are not limited to copper, iron and/or manganese chelating agents and mixtures thereof. In embodiments in which at least one chelating agent is used, the cleaning compositions of the present invention comprise from about 0.1% to about 15% or even from about 3.0% to about 10% chelating agent by weight of the subject cleaning composition.

In some still further embodiments, the cleaning compositions provided herein contain at least one deposition aid. Suitable deposition aids include, but are not limited to, polyethylene glycol, polypropylene glycol, polycarboxylate, soil release polymers such as polytelephthalic acid, clays such as kaolinite, montmorillonite, atapulgite, illite, bentonite, halloysite, and mixtures thereof.

As indicated herein, in some embodiments, anti-redeposition agents find use in some embodiments of the present invention. In some embodiments, non-ionic surfactants find use. For example, in automatic dishwashing embodiments, non-ionic surfactants find use for surface modification purposes, in particular for sheeting, to avoid filming and spotting and to improve shine. These non-ionic surfactants also find use in preventing the re-deposition of soils. In some embodiments, the anti-redeposition agent is a non-ionic surfactant as known in the art (*See e.g.,* EP 2 100 949).

In some embodiments, the cleaning compositions of the present invention include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. In embodiments in which at least one dye transfer inhibiting agent is used, the cleaning compositions of the present invention comprise from about 0.0001% to about 10%, from about 0.01% to about 5%, or even from about 0.1% to about 3% by weight of the cleaning composition.

In some embodiments, silicates are included within the compositions of the present invention. In some such embodiments, sodium silicates (*e.g.,* sodium disilicate, sodium metasilicate, and crystalline phyllosilicates) find use. In some embodiments, silicates are present at a level of from about 1% to about 20%. In some embodiments, silicates are present at a level of from about 5% to about 15% by weight of the composition.

In some still additional embodiments, the cleaning compositions of the present invention also contain dispersants. Suitable water-soluble organic materials include, but are not limited to the homo- or co-polymeric acids or their salts, in which the polycarboxylic acid comprises at least two carboxyl radicals separated from each other by not more than two carbon atoms.

In some further embodiments, the enzymes used in the cleaning compositions are stabilized by any suitable technique. In some embodiments, the enzymes employed herein are stabilized by the presence of water-soluble sources of calcium and/or magnesium ions in the finished compositions that provide such ions to the enzymes. In some embodiments, the enzyme stabilizers include oligosaccharides, polysaccharides, and inorganic divalent metal salts, including alkaline earth metals, such as calcium salts. It is contemplated that various techniques for enzyme stabilization will find use in the present invention. For example, in some embodiments, the enzymes employed herein are stabilized by the presence of water-soluble sources of zinc (II), calcium (II) and/or magnesium (II) ions in the finished compositions that provide such ions to the enzymes, as well as other metal ions (*e.g*., barium (II), scandium (II), iron (II), manganese (II), aluminum (III), Tin (II), cobalt (II), copper (II), nickel (II), and oxovanadium (IV). Chlorides and sulfates also find use in some embodiments of the present invention. Examples of suitable oligosaccharides and polysaccharides (*e.g*., dextrins) are known in the art (*See e.g.,* WO 07/145964). In some embodiments, reversible protease inhibitors also find use, such as boron-containing compounds (*e.g*., borate, 4-formyl phenyl boronic acid) and/or a tripeptide aldehyde find use to further improve stability, as desired.

In some embodiments, bleaches, bleach activators and/or bleach catalysts are present in the compositions of the present invention. In some embodiments, the cleaning compositions of the present invention comprise inorganic and/or organic bleaching compound(s). Inorganic bleaches include, but are not limited to perhydrate salts (*e.g.,* perborate, percarbonate, perphosphate, persulfate, and persilicate salts). In some embodiments, inorganic perhydrate salts are alkali metal salts. In some embodiments, inorganic perhydrate salts are included as the crystalline solid, without additional protection, although in some other embodiments, the salt is coated. Any suitable salt known in the art finds use in the present invention (*See e.g.,* EP 2 100 949).

In some embodiments, bleach activators are used in the compositions of the present invention. Bleach activators are typically organic peracid precursors that enhance the bleaching action in the course of cleaning at temperatures of 60°C and below. Bleach activators suitable for use herein include compounds which, under perhydrolysis conditions, give aliphatic peroxoycarboxylic acids having preferably from about 1 to about 10 carbon atoms, in particular from about 2 to about 4 carbon atoms, and/or optionally substituted perbenzoic acid. Additional bleach activators are known in the art and find use in the present invention (*See e.g.,* EP 2 100 949).

In addition, in some embodiments and as further described herein, the cleaning compositions of the present invention further comprise at least one bleach catalyst. In some embodiments, the manganese triazacyclononane and related complexes find use, as well as cobalt, copper, manganese, and iron complexes. Additional bleach catalysts find use in the present invention (*See e.g.,* US 4,246,612, 5,227,084, 4,810410, WO 99/06521, and EP 2 100 949).

In some embodiments, the cleaning compositions of the present invention contain one or more catalytic metal complexes. In some embodiments, a metal-containing bleach catalyst finds use. In some embodiments, the metal bleach catalyst comprises a catalyst system comprising a transition metal cation of defined bleach catalytic activity, (*e.g.,* copper, iron, titanium, ruthenium, tungsten, molybdenum, or manganese cations), an auxiliary metal cation having little or no bleach catalytic activity (*e.g.,* zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof are used (*See e.g.,* US Patent No. 4,430,243). In some embodiments, the cleaning compositions of the present invention are catalyzed by means of a manganese compound. Such compounds and levels of use are well known in the art (*See e.g.,* US Patent No. 5,576,282). In additional embodiments, cobalt bleach catalysts find use in the cleaning compositions of the present invention. Various cobalt bleach catalysts are known in the art (*See e.g.,* US Patent Nos. 5,597,936 and 5,595,967) and are readily prepared by known procedures.

In some additional embodiments, the cleaning compositions of the present invention include a transition metal complex of a macropolycyclic rigid ligand (MRL). As a practical matter, and not by way of limitation, in some embodiments, the compositions and cleaning processes provided by the present invention are adjusted to provide on the order of at least one part per hundred million of the active MRL species in the aqueous washing medium, and in some embodiments, provide from about 0.005 ppm to about 25 ppm, more preferably from about 0.05 ppm to about 10 ppm, and most preferably from about 0.1 ppm to about 5 ppm, of the MRL in the wash liquor.

In some embodiments, transition-metals in the instant transition-metal bleach catalyst include, but are not limited to manganese, iron and chromium. MRLs also include, but are not limited to special ultra-rigid ligands that are cross-bridged (*e.g.,* 5,12-diethyl-1,5,8,12-tetraazabicyclo[6.6.2]hexadecane). Suitable transition metal MRLs are readily prepared by known procedures (*See e.g.,* WO 2000/32601, and US Patent No. 6,225,464).

In some embodiments, the cleaning compositions of the present invention comprise metal care agents. Metal care agents find use in preventing and/or reducing the tarnishing, corrosion, and/or oxidation of metals, including aluminum, stainless steel, and non-ferrous metals (*e.g*., silver and copper). Suitable metal care agents include those described in EP 2 100 949, WO 9426860 and WO 94/26859). In some embodiments, the metal care agent is a zinc salt. In some further embodiments, the cleaning compositions of the present invention comprise from about 0.1% to about 5% by weight of one or more metal care agent.

In some embodiments, the cleaning composition is a high density liquid (HDL) composition having a variant LG12 protease. The HDL liquid laundry detergent can comprise a detersive surfactant (10%-40%) comprising anionic detersive surfactant (selected from a group of linear or branched or random chain, substituted or unsubstituted alkyl sulphates, alkyl sulphonates, alkyl alkoxylated sulphate, alkyl phosphates, alkyl phosphonates, alkyl carboxylates, and/or mixtures thereof); and optionally non-ionic surfactant (selected from a group of linear or branched or random chain, substituted or unsubstituted alkyl alkoxylated alcohol, for example a C₈-C₁₈ alkyl ethoxylated alcohol and/or C₆-C₁₂ alkyl phenol alkoxylates), optionally wherein the weight ratio of anionic detersive surfactant (with a hydrophilic index (HIc) of from 6.0 to 9) to non-ionic detersive surfactant is greater than 1: 1.

The composition can comprise optionally, a surfactancy boosting polymer consisting of amphiphilic alkoxylated grease cleaning polymers (selected from a group of alkoxylated polymers having branched hydrophilic and hydrophobic properties, such as alkoxylated polyalkylenimines in the range of 0.05wt%-10wt%) and/or random graft polymers (typically comprising of hydrophilic backbone comprising monomers selected from the group consisting of: unsaturated C₁-C₆ carboxylic acids, ethers, alcohols, aldehydes, ketones, esters, sugar units, alkoxy units, maleic anhydride, saturated polyalcohols such as glycerol, and mixtures thereof; and hydrophobic side chain(s) selected from the group consisting of: C₄-C₂₅ alkyl group, polypropylene, polybutylene, vinyl ester of a saturated C₁-C₆ mono-carboxylic acid, C₁-C₆ alkyl ester of acrylic or methacrylic acid, and mixtures thereof.

The composition can comprise additional polymers such as soil release polymers (include anionically end-capped polyesters, for example SRP1, polymers comprising at least one monomer unit selected from saccharide, dicarboxylic acid, polyol and combinations thereof, in random or block configuration, ethylene terephthalate-based polymers and co-polymers thereof in random or block configuration, for example Repel-o-tex SF, SF-2 and SRP6, Texcare SRA100, SRA300, SRN100, SRN170, SRN240, SRN300 and SRN325, Marloquest SL), anti-redeposition polymers (0.1 wt% to 10wt%, include carboxylate polymers, such as polymers comprising at least one monomer selected from acrylic acid, maleic acid (or maleic anhydride), fumaric acid, itaconic acid, aconitic acid, mesaconic acid, citraconic acid, methylenemalonic acid, and any mixture thereof, vinylpyrrolidone homopolymer, and/or polyethylene glycol, molecular weight in the range of from 500 to 100,000 Da); cellulosic polymer (including those selected from alkyl cellulose, alkyl alkoxyalkyl cellulose, carboxyalkyl cellulose, alkyl carboxyalkyl cellulose examples of which include carboxymethyl cellulose, methyl cellulose, methyl hydroxyethyl cellulose, methyl carboxymethyl cellulose, and mixures thereof) and polymeric carboxylate (such as maleate/acrylate random copolymer or polyacrylate homopolymer).

The composition can further comprise saturated or unsaturated fatty acid, preferably saturated or unsaturated C₁₂-C₂₄ fatty acid (0 wt% to 10 wt%); deposition aids (examples for which include polysaccharides, preferably cellulosic polymers, poly diallyl dimethyl ammonium halides (DADMAC), and co-polymers of DAD MAC with vinyl pyrrolidone, acrylamides, imidazoles, imidazolinium halides, and mixtures thereof, in random or block configuration, cationic guar gum, cationic cellulose such as cationic hydoxyethyl cellulose, cationic starch, cationic polyacylamides, and mixtures thereof.

The composition can further comprise dye transfer inhibiting agents examples of which include manganese phthalocyanine, peroxidases, polyvinylpyrrolidone polymers, polyamine N-oxide polymers, copolymers of N-vinylpyrrolidone and N-vinylimidazole, polyvinyloxazolidones and polyvinylimidazoles and/or mixtures thereof; chelating agents examples of which include ethylenediamine-tetraacetic acid (EDTA); diethylene triamine penta methylene phosphonic acid (DTPMP); hydroxy-ethane diphosphonic acid (HEDP); ethylenediamine N,N'-disuccinic acid (EDDS); methyl glycine diacetic acid (MGDA); diethylene triamine penta acetic acid (DTPA); propylene diamine tetracetic acid (PDT A); 2-hydroxypyridine-N-oxide (HPNO); or methyl glycine diacetic acid (MGDA); glutamic acid N,N-diacetic acid (N,N-dicarboxymethyl glutamic acid tetrasodium salt (GLDA); nitrilotriacetic acid (NTA); 4,5-dihydroxy-m-benzenedisulfonic acid; citric acid and any salts thereof; N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP) and derivatives thereof.

The composition can further comprise enzymes (0.01 wt% active enzyme to 0.03wt% active enzyme) selected from a group of proteases; amylases; lipases; cellulases; choline oxidases; peroxidases/oxidases; pectate lyases; mannanases; cutinases; laccases; phospholipases; lysophospholipases; acyltransferase; perhydrolase; arylesterase and any mixture thereof. The composition may comprise an enzyme stabilizer (examples of which include polyols such as propylene glycol or glycerol, sugar or sugar alcohol, lactic acid, reversible protease inhibitor, boric acid, or a boric acid derivative, e.g., an aromatic borate ester, or a phenyl boronic acid derivative such as 4-formylphenyl boronic acid).

The composition can further comprise silicone or fatty-acid based suds suppressors; heuing dyes, calcium and magnesium cations, visual signaling ingredients, anti-foam (0.001 wt% to about 4.0wt%), and/or structurant/thickener (0.01 wt% to 5wt%, selected from the group consisting of diglycerides and triglycerides, ethylene glycol distearate, microcrystalline cellulose, cellulose based materials, microfiber cellulose, biopolymers, xanthan gum, gellan gum, and mixtures thereof).

Suitable detersive surfactants also include cationic detersive surfactants (selected from a group of alkyl pyridinium compounds, alkyl quarternary ammonium compounds, alkyl quarternary phosphonium compounds, alkyl ternary sulphonium compounds, and/or mixtures thereof); zwitterionic and/or amphoteric detersive surfactants (selected from a group of alkanolamine sulpho-betaines); ampholytic surfactants; semi-polar non-ionic surfactants and mixtures thereof.

The composition can be any liquid form, for example a liquid or gel form, or any combination thereof. The composition may be in any unit dose form, for example a pouch.

In some embodiments, the cleaning composition is a high density powder (HDD) composition having a variant LG12 protease. The HDD powder laundry detergent can comprise a detersive surfactant including anionic detersive surfactants (selected from a group of linear or branched or random chain, substituted or unsubstituted alkyl sulphates, alkyl sulphonates, alkyl alkoxylated sulphate, alkyl phosphates, alkyl phosphonates, alkyl carboxylates and/or mixtures thereof), non-ionic detersive surfactant (selected from a group of linear or branched or random chain, substituted or unsubstituted C₈-C₁₈ alkyl ethoxylates, and/or C₆-C₁₂ alkyl phenol alkoxylates), cationic detersive surfactants (selected from a group of alkyl pyridinium compounds, alkyl quaternary ammonium compounds, alkyl quaternary phosphonium compounds, alkyl ternary sulphonium compounds, and mixtures thereof), zwitterionic and/or amphoteric detersive surfactants (selected from a group of alkanolamine sulpho-betaines); ampholytic surfactants; semi-polar non-ionic surfactants and mixtures thereof; builders (phosphate free builders [for example zeolite builders examples of which include zeolite A, zeolite X, zeolite P and zeolite MAP in the range of 0wt% to less than 10wt%]; phosphate builders [examples of which include sodium tri-polyphosphate in the range of 0wt% to less than 10wt%]; citric acid, citrate salts and nitrilotriacetic acid or salt thereof in the range of less than 15 wt%); silicate salt (sodium or potassium silicate or sodium meta-silicate in the range of 0wt% to less than 10wt%, or layered silicate (SKS-6)); carbonate salt (sodium carbonate and/or sodium bicarbonate in the range of 0 wt% to less than 10 wt%); and bleaching agents (photobleaches, examples of which include sulfonated zinc phthalocyanines, sulfonated aluminum phthalocyanines, xanthenes dyes, and mixtures thereof; hydrophobic or hydrophilic bleach activators (examples of which include dodecanoyl oxybenzene sulfonate, decanoyl oxybenzene sulfonate, decanoyl oxybenzoic acid or salts thereof, 3,5,5-trimethy hexanoyl oxybenzene sulfonate, tetraacetyl ethylene diamine-TAED, and nonanoyloxybenzene sulfonate-NOBS, nitrile quats, and mixtures thereof; hydrogen peroxide; sources of hydrogen peroxide (inorganic perhydrate salts examples of which include mono or tetra hydrate sodium salt of perborate, percarbonate, persulfate, perphosphate, or persilicate); preformed hydrophilic and/or hydrophobic peracids (selected from a group consisting of percarboxylic acids and salts, percarbonic acids and salts, perimidic acids and salts, peroxymonosulfuric acids and salts) & mixtures thereof and/or bleach catalyst (such as imine bleach boosters examples of which include iminium cations and polyions; iminium zwitterions; modified amines; modified amine oxides; N-sulphonyl imines; N-phosphonyl imines; N-acyl imines; thiadiazole dioxides; perfluoroimines; cyclic sugar ketones and mixtures thereof; metal-containing bleach catalyst for example copper, iron, titanium, ruthenium, tungsten, molybdenum, or manganese cations along with an auxiliary metal cations such as zinc or aluminum and a sequestrate such as ethylenediaminetetraacetic acid, ethylenediaminetetra(methylenephosphonic acid) and water-soluble salts thereof).

The composition can further comprise enzymes selected from a group of proteases; amylases; lipases; cellulases; choline oxidases; peroxidases/oxidases; pectate lyases; mannanases; cutinases; laccases; phospholipases; lysophospholipases; acyltransferase; perhydrolase; arylesterase and any mixture thereof.

The composition can further comprise additional detergent ingredients including perfume microcapsules, starch encapsulated perfume accord, hueing agents, additional polymers including fabric integrity and cationic polymers, dye lock ingredients, fabric-softening agents, brighteners (for example C.I. Fluorescent brighteners), flocculating agents, chelating agents, alkoxylated polyamines, fabric deposition aids, and/or cyclodextrin.

In some embodiments, the cleaning composition is an automatic dishwashing (ADW) detergent composition having a variant LG12 protease. The ADW detergent can comprise two or more non-ionic surfactants selected from a group of ethoxylated non-ionic surfactants, alcohol alkoxylated surfactants, epoxy-capped poly(oxyalkylated) alcohols, or amine oxide surfactants present in amounts from 0 to 10% by weight; builders in the range of 5-60% comprising either phosphate (mono-phosphates, di-phosphates, tri-polyphosphates or oligomeric-poylphosphates, preferred sodium tripolyphosphate-STPP or phosphate-free builders [amino acid based compounds, examples of which include MGDA (methyl-glycine-diacetic acid), and salts and derivatives thereof, GLDA (glutamic-N,Ndiacetic acid) and salts and derivatives thereof, IDS (iminodisuccinic acid) and salts and derivatives thereof, carboxy methyl inulin and salts and derivatives thereof and mixtures thereof, nitrilotriacetic acid (NTA), diethylene triamine penta acetic acid (DTPA), B-alaninediacetic acid (B-ADA) and their salts], homopolymers and copolymers of polycarboxylic acids and their partially or completely neutralized salts, monomeric polycarboxylic acids and hydroxycarboxylic acids and their salts in the range of 0.5% to 50% by weight; sulfonated/carboxylated polymers (provide dimensional stability to the product) in the range of about 0.1 % to about 50% by weight; drying aids in the range of about 0.1 % to about 10% by weight (selected from polyesters, especially anionic polyesters optionally together with further monomers with 3 to 6 functionalities which are conducive to polycondensation, specifically acid, alcohol or ester functionalities, polycarbonate-, polyurethane- and/or polyurea-polyorganosiloxane compounds or precursor compounds thereof of the reactive cyclic carbonate and urea type); silicates in the range from about 1 % to about 20% by weight (sodium or potassium silicates for example sodium disilicate, sodium meta-silicate and crystalline phyllosilicates); bleach-inorganic (for example perhydrate salts such as perborate, percarbonate, perphosphate, persulfate and persilicate salts) and organic (for example organic peroxyacids including diacyl and tetraacylperoxides, especially diperoxydodecanedioc acid, diperoxytetradecanedioc acid, and diperoxyhexadecanedioc acid); bleach activators- organic peracid precursors in the range from about 0.1 % to about 10% by weight; bleach catalysts (selected from manganese triazacyclononane and related complexes, Co, Cu, Mn and Fe bispyridylamine and related complexes, and pentamine acetate cobalt(III) and related complexes); metal care agents in the range from about 0.1% to 5% by weight (selected from benzatriazoles, metal salts and complexes, and/or silicates); enzymes in the range from about 0.01 to 5.0mg of active enzyme per gram of automatic dishwashing detergent composition (selected from a group of proteases; amylases; lipases; cellulases; choline oxidases; peroxidases/oxidases; pectate lyases; mannanases; cutinases; laccases; phospholipases; lysophospholipases; acyltransferase; perhydrolase; arylesterase and any mixture thereof); and enzyme stabilizer components (selected from oligosaccharides, polysaccharides and inorganic divalent metal salts).

Representative detergent formulations that beneficially include a serine protease polypeptide of the present invention include the detergent formulations found in WO2013063460, pages 78-152, and in particular the tables of pages 94 to 152. The serine proteases are normally incorporated into the detergent composition at a level of from 0.00001% to 10% of enzyme protein by weight of the composition. In some embodiments, the detergent composition comprises more than 0.0001%, 0.001%, 0.01%, or 0.1% of the serine protease by weight of the composition. In some embodiments, the detergent composition comprises less than 1%, 0.1%, 0.01%, or 0.001% of the serine protease by weight of the composition.

The cleaning compositions of the present invention are formulated into any suitable form and prepared by any process chosen by the formulator, non-limiting examples of which are described in U.S. Pat. Nos. 5,879,584, 5,691,297, 5,574,005, 5,569,645, 5,516,448, 5,489,392, and 5,486,303. In some embodiments in which a low pH cleaning composition is desired, the pH of such composition is adjusted via the addition of an acidic material such as HCl.

The cleaning compositions disclosed herein of find use in cleaning a *situs* (*e.g.,* a surface, item, dishware, or fabric). Typically, at least a portion of the situs is contacted with an embodiment of the present cleaning composition, in neat form or diluted in a wash liquor, and then the situs is optionally washed and/or rinsed. For purposes of the present invention, "washing" includes but is not limited to, scrubbing, and mechanical agitation. In some embodiments, the cleaning compositions are typically employed at concentrations of from about 500 ppm to about 15,000 ppm in solution. When the wash solvent is water, the water temperature typically ranges from about 5°C to about 90°C and, when the situs comprises a fabric, the water to fabric mass ratio is typically from about 1:1 to about 30:1.

### Processes of Making and Using Cleaning Compositions

The cleaning compositions of the present invention are formulated into any suitable form and prepared by any suitable process chosen by the formulator, (*See e.g.,* US Patent Nos. 5,879,584, 5,691,297, 5,574,005, 5,569,645, 5,565,422, 5,516,448, 5,489,392, 5,486,303, 4,515,705, 4,537,706, 4,515,707, 4,550,862, 4,561,998, 4,597,898, 4,968,451, 5,565,145, 5,929,022, 6,294,514 and 6,376,445).

In some embodiments, the cleaning compositions of the present invention are provided in unit dose form, including tablets, capsules, sachets, pouches, and multi-compartment pouches. In some embodiments, the unit dose format is designed to provide controlled release of the ingredients within a multi-compartment pouch (or other unit dose format). Suitable unit dose and controlled release formats are known in the art (*See e.g.,* EP 2 100 949, WO 02/102955, US Pat. Nos. 4,765,916 and 4,972,017, and WO 04/111178 for materials suitable for use in unit dose and controlled release formats). In some embodiments, the unit dose form is provided by tablets wrapped with a water-soluble film or water-soluble pouches. Various formats for unit doses are provided in EP 2 100 947, and are known in the art.

### Methods of Use

In some embodiments, the cleaning compositions of the present invention find use in cleaning surfaces (e.g., dishware), laundry, hard surfaces, contact lenses, etc. In some embodiments, at least a portion of the surface is contacted with at least one embodiment of the cleaning compositions of the present invention, in neat form or diluted in a wash liquor, and then the surface is optionally washed and/or rinsed. For purposes of the present invention, "washing" includes, but is not limited to, scrubbing, and mechanical washing. In some embodiments, the cleaning compositions of the present invention are used at concentrations of from about 500 ppm to about 15,000 ppm in solution. In some embodiments in which the wash solvent is water, the water temperature typically ranges from about 5 °C to about 90 °C.

The present invention provides methods for cleaning or washing an item or surface (*e.g.,* hard surface) in need of cleaning, including, but not limited to methods for cleaning or washing a dishware item, a tableware item, a fabric item, a laundry item, personal care item, etc., or the like, and methods for cleaning or washing a hard or soft surface (*e.g.,* a hard surface of an item).

In some embodiments, the present invention provides a method for cleaning an item, object, or surface in need of cleaning, the method comprising contacting the item or surface (or a portion of the item or surface desired to be cleaned) with at least one variant LG12 protease of the present invention or a composition of the present invention for a sufficient time and/or under conditions suitable and/or effective to clean the item, object, or surface to a desired degree. Some such methods further comprise rinsing the item, object, or surface with water. For some such methods, the cleaning composition is a dishwashing detergent composition and the item or object to be cleaned is a dishware item or tableware item. As used herein, a "dishware item" is an item generally used in serving or eating food. A dishware item can be, but is not limited to for example, a dish, plate, cup, bowl, etc., and the like. As used herein, "tableware" is a broader term that includes, but is not limited to for example, dishes, cutlery, knives, forks, spoons, chopsticks, glassware, pitchers, sauce boats, drinking vessels, serving items, etc. It is intended that "tableware item" includes any of these or similar items for serving or eating food. For some such methods, the cleaning composition is an automatic dishwashing detergent composition or a hand dishwashing detergent composition and the item or object to be cleaned is a dishware or tableware item. For some such methods, the cleaning composition is a laundry detergent composition (*e.g.,* a power laundry detergent composition or a liquid laundry detergent composition), and the item to be cleaned is a fabric item. In some other embodiments, the cleaning composition is a laundry pre-treatment composition.

In some embodiments, the present invention provides methods for cleaning or washing a fabric item optionally in need of cleaning or washing, respectively. In some embodiments, the methods comprise providing a composition comprising the variant protease, including but not limited to fabric or laundry cleaning composition, and a fabric item or laundry item in need of cleaning, and contacting the fabric item or laundry item (or a portion of the item desired to be cleaned) with the composition under conditions sufficient or effective to clean or wash the fabric or laundry item to a desired degree.

In some embodiments, the present invention provides a method for cleaning or washing an item or surface (*e.g*., hard surface) optionally in need of cleaning, the method comprising providing an item or surface to be cleaned or washed and contacting the item or surface (or a portion of the item or surface desired to be cleaned or washed) with at least one LG12 variant of the invention or a composition of the invention comprising at least one such LG12 variant for a sufficient time and/or under conditions sufficient or effective to clean or wash the item or surface to a desired degree. Such compositions include, but are not limited to for example, a cleaning composition or detergent composition of the invention (*e.g.*, a hand dishwashing detergent composition, hand dishwashing cleaning composition, laundry detergent or fabric detergent or laundry or fabric cleaning composition, liquid laundry detergent, liquid laundry cleaning composition, powder laundry detergent composition, powder laundry cleaning composition, automatic dishwashing detergent composition, laundry booster cleaning or detergent composition, laundry cleaning additive, and laundry pre-spotter composition, etc.). In some embodiments, the method is repeated one or more times, particularly if additional cleaning or washing is desired. For example, in some instance, the method optionally further comprises allowing the item or surface to remain in contact with the at least one variant protease or composition for a period of time sufficient or effective to clean or wash the item or surface to the desired degree. In some embodiments, the methods further comprise rinsing the item or surface with water and/or another liquid. In some embodiments, the methods further comprise contacting the item or surface with at least one variant protease of the invention or a composition of the invention again and allowing the item or surface to remain in contact with the at least one variant protease or composition for a period of time sufficient to clean or wash the item or surface to the desired degree. In some embodiments, the cleaning composition is a dishwashing detergent composition and the item to be cleaned is a dishware or tableware item. In some embodiments of the present methods, the cleaning composition is an automatic dishwashing detergent composition or a hand dishwashing detergent composition and the item to be cleaned is a dishware or tableware item. In some embodiments of the methods, the cleaning composition is a laundry detergent composition and the item to be cleaned is a fabric item.

The present invention also provides methods of cleaning a tableware or dishware item in an automatic dishwashing machine, the method comprising providing an automatic dishwashing machine, placing an amount of an automatic dishwashing composition comprising at least one LG12 variant of the present invention or a composition of the invention sufficient to clean the tableware or dishware item in the machine (*e.g.,* by placing the composition in an appropriate or provided detergent compartment or dispenser in the machine), putting a dishware or tableware item in the machine, and operating the machine so as to clean the tableware or dishware item (*e.g.,* as per the manufacturer's instructions). In some embodiments, the methods include any automatic dishwashing composition described herein, which comprises, but is not limited to at least one LG12 variant provided herein. The amount of automatic dishwashing composition to be used can be readily determined according to the manufacturer's instructions or suggestions and any form of automatic dishwashing composition comprising at least one variant protease of the invention (*e.g.,* liquid, powder, solid, gel, tablet, etc.), including any described herein, may be employed.

The present invention also provides methods for cleaning a surface, item or object optionally in need of cleaning, the method comprises contacting the item or surface (or a portion of the item or surface desired to be cleaned) with at least one variant LG12 of the present invention or a cleaning composition of the invention in neat form or diluted in a wash liquor for a sufficient time and/or under conditions sufficient or effective to clean or wash the item or surface to a desired degree. The surface, item, or object may then be (optionally) washed and/or rinsed if desired. For purposes of the present invention, "washing" includes, but is not limited to for example, scrubbing and mechanical agitation. In some embodiments, the cleaning compositions are employed at concentrations of from about 500 ppm to about 15,000 ppm in solution (*e.g.,* aqueous solution). When the wash solvent is water, the water temperature typically ranges from about 5°C to about 90°C.

The present invention also provides methods of cleaning a laundry or fabric item in an washing machine, the method comprising providing an washing machine, placing an amount of a laundry detergent composition comprising at least one variant LG12 of the invention sufficient to clean the laundry or fabric item in the machine (*e.g.,* by placing the composition in an appropriate or provided detergent compartment or dispenser in the machine), placing the laundry or fabric item in the machine, and operating the machine so as to clean the laundry or fabric item (*e.g.,* as per the manufacturer's instructions). The methods of the present invention include any laundry washing detergent composition described herein, comprising but not limited to at least one of any variant LG12 provided herein. The amount of laundry detergent composition to be used can be readily determined according to manufacturer's instructions or suggestions and any form of laundry detergent composition comprising at least one variant protease of the invention (*e.g.,* solid, powder, liquid, tablet, gel, etc.), including any described herein, may be employed.

### LG-12 clade protease polypeptides of the present invention for use in Animal Feed

LG-12 clade protease polypeptides of the present invention can be used as a compontent of an animal feed composition, animal feed additive and/or pet food comprising a LG-12 clade protease and variants thereof. Disclosed herein is a method for preparing such an animal feed composition, animal feed additive composition and/or pet food comprising mixing the LG-12 clade protease polypeptide with one or more animal feed ingredients and/or animal feed additive ingredients and/or pet food ingredients. Furthermore, disclosed herein is the use of the LG-12 clade protease polypeptide in the preparation of an animal feed composition and/or animal feed additive composition and/or pet food.

The term "animal" includes all non-ruminant and ruminant animals. In a particular embodiment, the animal is a non-ruminant animal, such as a horse and a mono-gastric animal. Examples of mono-gastric animals include, but are not limited to, pigs and swine, such as piglets, growing pigs, sows; poultry such as turkeys, ducks, chicken, broiler chicks, layers; fish such as salmon, trout, tilapia, catfish and carps; and crustaceans such as shrimps and prawns. In a further embodiment the animal is a ruminant animal including, but not limited to, cattle, young calves, goats, sheep, giraffes, bison, moose, elk, yaks, water buffalo, deer, camels, alpacas, llamas, antelope, pronghorn and nilgai.

In the present context, it is intended that the term "pet food" is understood to mean a food for a household animal such as, but not limited to, dogs, cats, gerbils, hamsters, chinchillas, fancy rats, guinea pigs; avian pets, such as canaries, parakeets, and parrots; reptile pets, such as turtles, lizards and snakes; and aquatic pets, such as tropical fish and frogs.

The terms "animal feed composition," "feedstuff' and "fodder" are used interchangeably and can comprise one or more feed materials selected from the group comprising a) cereals, such as small grains (e.g., wheat, barley, rye, oats and combinations thereof) and/or large grains such as maize or sorghum; b) by products from cereals, such as corn gluten meal, Distillers Dried Grain Solubles (DDGS) (particularly corn based Distillers Dried Grain Solubles (cDDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

### LG-12 clade protease polypeptides of the present invention for use in Textile Desizing

Also contemplated are compositions and methods of treating fabrics (*e.g.,* to desize a textile) using a LG-12 clade protease polypeptide of the present invention. Fabric-treating methods are well known in the art (*see, e.g.,* U.S. Patent No. 6,077,316). For example, the feel and appearance of a fabric can be improved by a method comprising contacting the fabric with a LG-12 clade protease in a solution. The fabric can be treated with the solution under pressure.

A LG-12 clade protease of the present invention can be applied during or after the weaving of a textile, or during the desizing stage, or one or more additional fabric processing steps. During the weaving of textiles, the threads are exposed to considerable mechanical strain. Prior to weaving on mechanical looms, warp yarns are often coated with sizing starch or starch derivatives to increase their tensile strength and to prevent breaking. A LG-12 clade protease of the present invention can be applied during or after the weaving to remove these sizing starch or starch derivatives. After weaving, the LG-12 clade protease can be used to remove the size coating before further processing the fabric to ensure a homogeneous and wash-proof result.

A LG-12 clade protease of the present invention can be used alone or with other desizing chemical reagents and/or desizing enzymes to desize fabrics, including cotton-containing fabrics, as detergent additives, e.g., in aqueous compositions. An amylase also can be used in compositions and methods for producing a stonewashed look on indigo-dyed denim fabric and garments. For the manufacture of clothes, the fabric can be cut and sewn into clothes or garments, which are afterwards finished. In particular, for the manufacture of denim jeans, different enzymatic finishing methods have been developed. The finishing of denim garment normally is initiated with an enzymatic desizing step, during which garments are subjected to the action of proteolytic enzymes to provide softness to the fabric and make the cotton more accessible to the subsequent enzymatic finishing steps. The LG-12 clade protease can be used in methods of finishing denim garments (*e.g.,* a "bio-stoning process"), enzymatic desizing and providing softness to fabrics, and/or finishing process.

### LG-12 clade protease polypeptides of the present invention for use in Paper Pulp Bleaching

The LG-12 clade protease polypeptides described herein find further use in the enzyme aided bleaching of paper pulps such as chemical pulps, semi-chemical pulps, kraft pulps, mechanical pulps or pulps prepared by the sulfite method. In general terms, paper pulps are incubated with a LG-12 clade protease polypeptide of the present invention under conditions suitable for bleaching the paper pulp.

In some embodiments, the pulps are chlorine free pulps bleached with oxygen, ozone, peroxide or peroxyacids. In some embodiments, the LG-12 clade protease polypeptides are used in enzyme aided bleaching of pulps produced by modified or continuous pulping methods that exhibit low lignin contents. In some other embodiments, the LG-12 clade protease polypeptides are applied alone or preferably in combination with xylanase and/or endoglucanase and/or alpha-galactosidase and/or cellobiohydrolase enzymes.

### LG-12 clade protease polypeptides of the present invention for use in Protein degradation

The LG-12 clade protease polypeptides described herein find further use in the enzyme aided removal of proteins from animals and their subsequent degradation or disposal, such as feathers, skin, hair, hide, and the like. In some instances, immersion of the animal carcass in a solution comprising a LG-12 clade protease polypeptide of the present invention can act to protect the skin from damage in comparison to the traditional immersion in scalding water or the defeathering process. In one embodiment, feathers can be sprayed with an isolated metalloprotase polypeptide of the present invention under conditions suitable for digesting or initiating degradation of the plumage. In some embodiments, a LG-12 clade protease of the present invention can be used, as above, in combination with an oxidizing agent.

In some embodiments, removal of the oil or fat associated with raw feathers is assisted by using a LG-12 clade protease polypeptide of the present invention. In some embodiments, the LG-12 clade protease polypeptides are used in compositions for cleaning the feathers as well as to sanitize and partially dehydrate the fibers. In some other embodiments, the LG-12 clade protease polypeptides are applied in a wash solution in combination with 95% ethanol or other polar organic solvent with or without a surfactant at about 0.5% (v/v).

In yet other embodiments, the disclosed LG-12 clade protease polypeptides find use in recovering protein from plumage. In some embodiments, the recovered protein can be subsequently used in animal or fish feed.

### LG-12 clade protease polypeptides of the present invention for use in Tissue Debridement

The LG-12 clade protease polypeptides described herein find further use in the enzyme aided debridement of tissue. This involves the removal of dead or damaged tissue, for example, removal from wounds to aid in healing.

### LG-12 clade protease polypeptides of the present invention for use in tissue culture

The LG-12 clade protease polypeptides described herein find further use in tissue culture. In particular, LG-12 clade proteases of the present invention can be used to suspend or resuspend cells adherent to a cell culture wall, such as during the process of harvesting cells. LG-12 clade proteases of the present invention can be used to cleave protein bonds between cultured cells and the dish, allowing cells to become suspended in solution.

### LG-12 clade protease polypeptides of the present invention in food applications

The LG-12 clade protease polypeptides described herein find further use as a food additive, a digestive aide or a food processing aid.

### LG-12 clade protease polypeptides of the present invention for use in leather processing

The LG-12 clade protease polypeptides described herein find further use in leather processing by removing hair from animal hides, soaking, degreasing, or bating, which is a process involving degradation of non-structural proteins during leather making.

### EXPERIMENTAL

### EXAMPLE 1

### Assays

The following assays are standard assays used in the examples described below. Occasionally specific protocols call for deviations from these standard assays. In those cases, deviations from these standard assay protocols below are identified in the examples.

### Performance Index

The performance index (PI) of an enzyme compares the performance of the variant (measured value) with the parent enzyme (theoretical value or measured value) at the same protein concentration. Theoretical concentrations for the parent enzyme can be calculated using the parameters extracted from a Langmuir fit of a standard curve of the parent enzyme. A performance index (PI) that is greater than 1 (PI>1) indicates improved performance by a variant as compared to the parent (e.g. LG12 sprC mature protein, SEQ ID NO: 3), while a PI of 1 (PI=1) identifies a variant that performs the same as the parent, and a PI that is less than 1 (PI<1) identifies a variant that performs worse than the parent.

### Protein Determination Assay

Protein determination was performed to determine levels of protein expression using supernatants from cultures grown in 96-well micro-titer plates (MTPs), by High Performance Liquid Chromatography (HPLC) method. An Agilent 1200 or 1260 HPLC equipped with an Acquity UPLC BEH 125 SEC (Waters) size exclusion column was used. Sample was eluted from the column using 25 mM sodium phosphate buffer pH 6.8 containing 250 mM sodium chloride. Absorbance was measured at 220 nm, and peaks integrated using ChemStation software (Agilent Technologies). The protein concentration of samples was determined based on a standard curve of purified wild type protein (parent LG12-clade subtilisin enzyme, sprC of SEQ ID NO:3).

### Protease Activity

The protease activity of LG12 sprC protease and variants thereof was tested by measuring the hydrolysis of N-suc-AAPF-pNA colorimetric substrate. The reagent solutions used were: 100 mM Tris/HCl pH 8.6, containing 0.005% TWEEN®-80 (Tris dilution buffer); 100 mM Tris buffer pH 8.6, containing 10 mM CaCl₂ and 0.005% TWEEN®-80 (Tris/Ca buffer); and 160 mM suc-AAPF-pNA in DMSO (suc-AAPF-pNA stock solution) (Sigma: S-7388). To prepare a substrate working solution, 1 ml suc-AAPF-pNA stock solution was added to 100 ml Tris/Ca buffer and mixed well. An enzyme sample was added to a MTP plate (Greiner 781101) containing 1 mg/ suc-AAPF-pNA working solution and assayed for activity at 405 nm over 3 minutes using a SpectraMax plate reader in kinetic mode at RT. The protease activity was expressed as mOD·min⁻¹.

### General sample set-up for Stability Assays

Variants were tested for stability under various stress conditions (buffers and detergents as indicated on the Table 1 below) by measuring the residual activity following incubation at elevated temperature. The elevated temperature was set to obtain approximately 30% residual activity Diluted enzyme sample was mixed in stressor and unstressed protease activity was measured. The diluted sample in stressor was incubated at elevated temperature and after incubation the stressed protease activity was measured.

The stability assay conditions are described in the table below, detergents were inactivated prior to use in stability assays:

**Table 1 - Conditions used for protein stability assays**

| **Condition** | **Stress temperature (°C)** |
|---|---|
| 50mM Tris pH9; 2mM CaCl₂ | 72.5 |
| 50mM Tris pH9; 2mM CaCl₂ | 50 |
| 50mM Tris pH9; 1mM EDTA | 51 |
| 0.02% LAS, 2.1mM EDTA in 50mM Hepes pH8, 0,005% Tween | 52 |
| GSM-B pH 10.5 | 68.5 |
| GSM-C pH10.5 | 62 |
| OMO Klein & Krachtig | 51 |

For the unstressed condition, enzyme was assayed immediately for activity on AAPF (see assay above). For the stressed condition, the PCR plate was sealed and incubated at elevated temperature for 5 minutes using a Eppendorf 384 Thermocycler, then assayed for activity.

Stressed and unstressed activity was measured by hydrolysis of the synthetic substrate AAPF as described above. % Residual activities were calculated by taking a ratio of the stressed to unstressed activity and multiplying by 100. Stability PIs were obtained by dividing the residual activity of a variant by that of the wild type.

### Cleaning performance in Laundry and Automatic Dish detergents

Variants were tested for cleaning performance relative to wildtype LG12 sprC on BMI (blood/milk/ink on cotton) microswatches (EMPA-116) for laundry based applications, and on egg yolk (egg yolk on polyacryl fabric, aged and colored with carbon black dye) microswatches (PAS-38) for dish based applications.

Pre-punched (to fit on MTP), rinsed, and filled swatch-containing plates (Corning 3641) were prepared by Center for Testmaterials BV, Vlaardingen, The Netherlands. The microswatch plates were filled with detergent prior to enzyme addition. Commercial detergents were heat-inactivated to remove enzyme and dosed as described on Table 2. In addition, the compositions of GSM-B and GSM-C ADW detergents are shown in Table 2.1.

Detergent treatments for enzyme inactivation were as follows. Each ADW detergent tablet was dissolved in 700 mL 21GH water and enzyme activity was inactivated by pitting the solution for 30-45 minutes at 65°C. The enzyme inactivated solution was diluted 10 fold in 21GH water prior to use in the assays.

HDL laundry detergents were inactivated by heating to 95°C for 16 hours in a water bath. Protease activity was assayed following inactivation using the AAPF substrate to ensure complete inactivation. HDD laundry detergents were inactivated by preparing a 10X concentrated solution relative to what is used in the final cleaning assay and heating for 16 hours at 95°C. Protease activity was assayed following inactivation using the AAPF substrate. After 16 hour heating of both HDD and HDL detergents, protease activity was non-existent. Ten x concentration were prepared of ADW tablets and laundry HDD's, laundry HDL has been treated undiluted. The solutions were incubated at elevated temperature to inactivate enzyme activity. Protease activity was assayed following inactivation using AAPF-pNA substrate.

**Table 2 - List of detergent conditions used for performance assays**

| **Detergent** | **Type** | **Final Wash Conc, (g/L)** | **Hardness Conc. (ppm)** | **Buffer** | ***Set pH** |
|---|---|---|---|---|---|
| Kirkland Ultra | HDD | 1.09 | 150 | 2 mM NaCO₃ | 10.6 |
| OMO Color | HDD | 5.3 | 250 | 2 mM NaCO₃ | 10.6 |
| OMO Klein & Krachtig | HDL | 2.8 | 250 | 5 mM sodium HEPES | 8.2 |
| Blue Moon | HDL | 6.6 | 250 | 5 mM sodium HEPES | 6.5 |
| SUN All in One | ADW | One tablet dissolved and diluted 10X | 374ppm | Not buffered | ND |
| Finish Quantum | ADW | One tablet dissolved and diluted 10X | 374ppm | Not buffered | ND |
| GSM-B | ADW | 3 | 374ppm | Not buffered | ND |
| GSM-C | ADW | 3 | 374ppm | Not buffered | ND |
| GSM-B 9 | ADW | 3 | 374ppm | 1M citrate added | 9 |

| | | | | | |
|---|---|---|---|---|---|
| *pH was set for those detergents where value is provided and not for those marked ND | | | | | |

Detergent sources: Kirkland Ultra (Sun Products), OMO Color (Unilever) OMO Klein & Krachtig (Unilever) and Blue Monn (Guangzhou Blue Moon) were purchased from local supermarkets in 2012.Finish All in One (Reckitt Benckiser), Finish Quantum (Reckitt Benckiser), and SUN All in One (Unilever) detergent tablets were purchased in December 2012 from a local supermarket.

**Table 2.1 - Composition of GSM-B and GSM-C ADW detergents (purchased from WFK Testgewebe GmbH, Brüggen, Deutschland, www.testgewebe.de)**

| **GSM-B** | | **GSM-C** | |
|---|---|---|---|
| **Phosphate-Free Detergent** | | **Phosphate-Containing Detergent** | |
| **Component** | **wt %** | **Component** | **wt %** |
| Sodium citrate dehydrate | 30.0 | Sodium tripolyphosphate | 23.0 |
| Maleic acid/ acrylic acid copolymer sodium Salt (SOKALAN® CP5; BASF) | 12.0 | Sodium citrate dehydrate | 22.3 |
| Sodium perborate monohydrate | 5.0 | Maleic acid/ Acrylic Acis Copolymer Sodium Salt | 4.0 |
| TAED | 2.0 | Sodium perborate monohydrate | 6.0 |
| Sodium disilicate: Protil A (Cognis) | 25.0 | TAED | 2.0 |
| Linear fatty alcohol ethoxylate | 2.0 | Sodium disilicate: Protil A (Cognis) | 5.0 |
| Sodium carbonate anhydrous | add to 100 | Linear Fatty Alcohol Ethoxylate | 2.0 |
| | | Sodium Carbonate anhydrous | add to 100 |

Aliquots of enzyme were added to a detergent-filled microswatch plate to reach a final volume of 200 uL for laundry assays, to attain 5 to 0.5 ppm final enzyme concentration. Laundry cleaning assays with HDL or HDD formulas was carried out at 25°C for 15-20 minutes, while automatic dish (ADW) assays were carried out at 40°C for 30 minutes.

Following incubation, 100 uL of supernatant was transferred to a fresh MTP (Costar 9017) and absorbance was read at 600 nm for EMPA-116 swatches, or at 405 nm for PAS-38 swatches, using the SpectraMax plate reader. Absorbance results were obtained by subtracting a the value for a blank control (no enzyme) from each sample value. The cleaning PI for each assay condition was obtained by dividing the absorbance values for a given variant by that of the predicted wild type at the same concentration. The wildtype value was determined by fitting the standard curve of the parent to a Langmuir fit.

### EXAMPLE 2

### Generation of a site evaluation library (SEL) of Bacillus sp LG12 sprC protease

The LG12 sprC protease was produced in B. subtilis using an expression cassette consisting of the B. subtilis aprE promoter, a hybrid aprE-BPN' signal peptide, a hybrid BPN'-LG12 sprC protease pro-peptide, the mature LG12 sprC protease and a BPN' terminator. This cassette was cloned into the pHYT replicating shuttle vector as an EcoRI-BamHI fragment. The pHYT vector was derived from pHY300PLK (Takara) by adding a terminator after the tetracycline resistance gene using the BstEII and EcoRI sites (SEQ ID NO:21 terminator sequence, GGTTACCTTGAATGTATATAAACATTCTCAAAGGGATTTCTAATAAAAAACGCTCGGTTGCC GCCGGGCGTTTTTTATGCATCGATGGAATTC). The HindIII site in pHY300PLK was also removed using a linker cloned into the BamHI and HindIII sites (SEQ ID NO:22 new linker sequence, GGATCCTGACTGCCTGAGCTT).

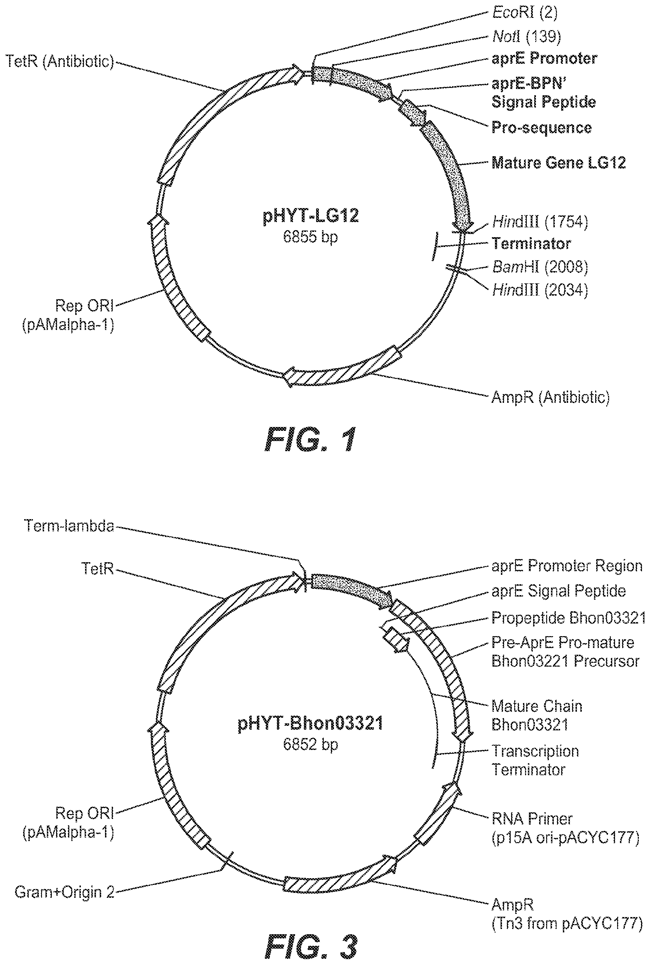

A map of the pHYT vector containing the LG12 sprC gene (pHYT-LG12) is shown in Figure 1. Top10 E.coli competent cells were transformed with plasmid pHYT-LG12 sprC and the transformed cells were plated onto LB agar plates containing 50ppm carbenicillin. Following an overnight incubation at 37C, cultures were set up in 5ml Luria broth containing 50ppm carbenicillin and grown at 37C overnight with shaking. Plasmid DNA was isolated from the cultures the following day and sequenced. Sequencing of plasmid DNA across regions manipulated was performed for confirmation, and plasmids with correct sequences were then used to transform *Bacillus subtilis* cells. Transformed cells were plated on 1.6% skim milk containing LB agar plates with 10ppm tetracycline.

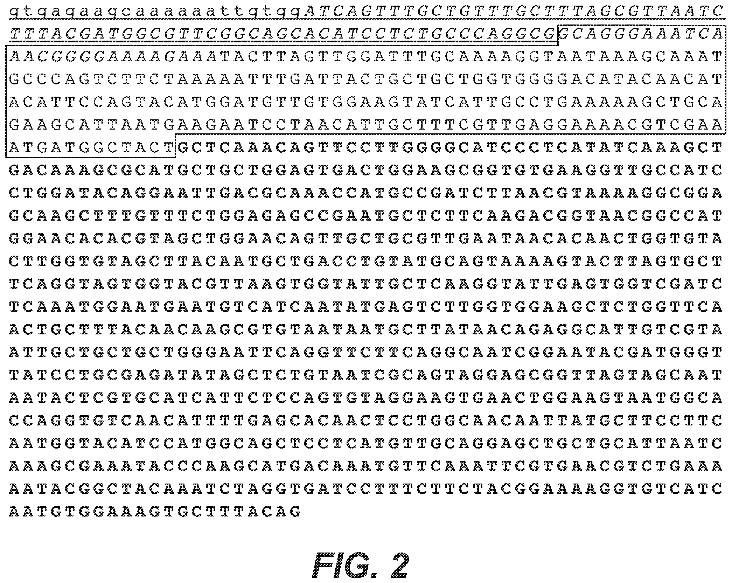

A positional library at each of the 275 sites in the LG12 sprC mature serine protease [B. Schmidt et al 1995, Applied and Environmental Microbiology 61:4490-4493] was generated.
The variants consisted of transformed *B. subtilis* cells containing the expression plasmid encoding LG12 sprC variant sequences. The variants were arrayed in a 96 well plate, one variant per well. The *B. subtilis* transformants containing LG12 sprC substitution variants were grown overnight in 96 well plates in Tryptic Soy Broth (TSB) with 12.5ppm tetracycline, and 10 uL of this pre-culture was added to square well MTPs (Enzyscreen) containing 340uL of cultivation media (described below) supplemented with 25ppm tetracycline. The plates were incubated for 2 days at 32°C, at 80% humidity with constant rotational mixing at 300 rpm. Cells were harvested by centrifugation at 2500 rpm for 10 minutes and filtered through Millipore Multiscreen filterplate using a Millipore vacuum system. The culture supernatants were used for assays. The cultivation media was an enriched semi-defined media based on MOPs buffer, with urea as major nitrogen source, glucose as the main carbon source, and supplemented with 1% soytone for robust cell growth. Figure 2 shows the sequence of the precursor to LG12 sprC protein as encoded in plasmid pHYT-LG12. Signal peptide (Signal peptide: 7^{th} amino acid (Trp) of AprE fused to 8^{th} amino acid (Ile) of BPN') is underlined (Sequences from *Bacillus subtilis* are shown in lower case, Pro peptide: 9^{th} amino acid (Lys) of BPN' fused to 13^{th} amino acid (Tyr) of LG12, sequences from *Bacillus amyloliquefaciens* are shown in *italics*, LG12 sequences are shown in normal text), Pro peptide sequence is highlighted, mature LG12 sequence is shown in bold.

The nucleotide sequence of the open reading frame encoding the LG12 sprC gene and the N-terminal signal and pro-peptide regions used for expression in pHYT-LG12 plasmid is shown below.
SEQ ID NO:1 sets forth the nucleotide sequence encoding the precursor to LG12 sprC protein as encoded in plasmid pHYT-LG12. 
GTGAGAAGCAAAAAATTGTGGATCAGTTTGCTGTTTGCTTTAGCGTTAATCTT
TACGATGGCGTTCGGCAGCACATCCTCTGCCCAGGCGGCAGGGAAATCAAAC
GGGGAAAAGAAATACTTAGTTGGATTTGCAAAAGGTAATAAAGCAAATGCC
CAGTCTTCTAAAAATTTGATTACTGCTGCTGGTGGGGACATACAACATACAT
TCCAGTACATGGATGTTGTGGAAGTATCATTGCCTGAAAAAGCTGCAGAAGC
ATTAATGAAGAATCCTAACATTGCTTTCGTTGAGGAAAACGTCGAAATGATG
GCTACTGCTCAAACAGTTCCTTGGGGCATCCCTCATATCAAAGCTGACAAAG
CGCATGCTGCTGGAGTGACTGGAAGCGGTGTGAAGGTTGCCATCCTGGATAC
AGGAATTGACGCAAACCATGCCGATCTTAACGTAAAAGGCGGAGCAAGCTT
TGTTTCTGGAGAGCCGAATGCTCTTCAAGACGGTAACGGCCATGGAACACAC
GTAGCTGGAACAGTTGCTGCGTTGAATAACACAACTGGTGTACTTGGTGTAG
CTTACAATGCTGACCTGTATGCAGTAAAAGTACTTAGTGCTTCAGGTAGTGG
TACGTTAAGTGGTATTGCTCAAGGTATTGAGTGGTCGATCTCAAATGGAATG
AATGTCATCAATATGAGTCTTGGTGGAAGCTCTGGTTCAACTGCTTTACAAC
AAGCGTGTAATAATGCTTATAACAGAGGCATTGTCGTAATTGCTGCTGCTGG
GAATTCAGGTTCTTCAGGCAATCGGAATACGATGGGTTATCCTGCGAGATAT
AGCTCTGTAATCGCAGTAGGAGCGGTTAGTAGCAATAATACTCGTGCATCAT
TCTCCAGTGTAGGAAGTGAACTGGAAGTAATGGCACCAGGTGTCAACATTTT
GAGCACAACTCCTGGCAACAATTATGCTTCCTTCAATGGTACATCCATGGCA
GCTCCTCATGTTGCAGGAGCTGCTGCATTAATCAAAGCGAAATACCCAAGCA
TGACAAATGTTCAAATTCGTGAACGTCTGAAAAATACGGCTACAAATCTAGG
TGATCCTTTCTTCTACGGAAAAGGTGTCATCAATGTGGAAAGTGCTTTACAG
SEQ ID NO:2 sets forth the nucleotide sequence coding for mature LG12 sprC protein (825 nucleotides) 
GCTCAAACAGTTCCTTGGGGCATCCCTCATATCAAAGCTGACAAAGCGCATG
CTGCTGGAGTGACTGGAAGCGGTGTGAAGGTTGCCATCCTGGATACAGGAAT
TGACGCAAACCATGCCGATCTTAACGTAAAAGGCGGAGCAAGCTTTGTTTCT
GGAGAGCCGAATGCTCTTCAAGACGGTAACGGCCATGGAACACACGTAGCT
GGAACAGTTGCTGCGTTGAATAACACAACTGGTGTACTTGGTGTAGCTTACA
ATGCTGACCTGTATGCAGTAAAAGTACTTAGTGCTTCAGGTAGTGGTACGTT
AAGTGGTATTGCTCAAGGTATTGAGTGGTCGATCTCAAATGGAATGAATGTC
ATCAATATGAGTCTTGGTGGAAGCTCTGGTTCAACTGCTTTACAACAAGCGT
GTAATAATGCTTATAACAGAGGCATTGTCGTAATTGCTGCTGCTGGGAATTC
AGGTTCTTCAGGCAATCGGAATACGATGGGTTATCCTGCGAGATATAGCTCT
GTAATCGCAGTAGGAGCGGTTAGTAGCAATAATACTCGTGCATCATTCTCCA
GTGTAGGAAGTGAACTGGAAGTAATGGCACCAGGTGTCAACATTTTGAGCAC
AACTCCTGGCAACAATTATGCTTCCTTCAATGGTACATCCATGGCAGCTCCTC
 
ATGTTGCAGGAGCTGCTGCATTAATCAAAGCGAAATACCCAAGCATGACAA
ATGTTCAAATTCGTGAACGTCTGAAAAATACGGCTACAAATCTAGGTGATCC
TTTCTTCTACGGAAAAGGTGTCATCAATGTGGAAAGTGCTTTACAG
SEQ ID NO:3 sets forth the amino acid sequence of the mature LG12 sprC protein (275 amino acids) 
AQTVPWGIPHIKADKAHAAGVTGSGVKVAILDTGIDANHADLNVKGGASFVSG
EPNALQDGNGHGTHVAGTVAALNNTTGVLGVAYNADLYAVKVLSASGSGTLS
GIAQGIEWSISNGMNVINMSLGGSSGSTALQQACNNAYNRGIVVIAAAGNSGSS
GNRNTMGYPARYSSVIAVGAVSSNNTRASFSSVGSELEVMAPGVNILSTTPGNN
YASFNGTSMAAPHVAGAAALIKAKYPSMTNVQIRERLKNTATNLGDPFFYGKG
VINVESALQ
SEQ ID NO: 4 sets forth the amino acid sequence of the full-length LG12 sprC protein (Signal sequence is underlined, pro-peptide is shown in italics, 3 residue addition AGK is shown in bold, mature LG12 sequence is shown in normal font).

VRSKKLWISLLFALALIFTMAFGSTSSAQAAGKSNGEKKYLVGFAKGNKANAQSSK
NLITAAGGDIQHTFQYMDVVEVSLPEKAAEALMKNPNIAFVEENVEMMATAQTVPW
GIPHIKADKAHAAGVTGSGVKVAILDTGIDANHADLNVKGGASFVSGEPNALQD
GNGHGTHVAGTVAALNNTTGVLGVAYNADLYAVKVLSASGSGTLSGIAQGIE
WSISNGMNVINMSLGGSSGSTALQQACNNAYNRGIVVIAAAGNSGSSGNRNTM
GYPARYSSVIAVGAVSSNNTRASFSSVGSELEVMAPGVNILSTTPGNNYASFNGT
SMAAPHVAGAAALIKAKYPSMTNVQIRERLKNTATNLGDPFFYGKGVINVESAL
Q

### EXAMPLE 3

### Productive Positions and Combinable Mutations

Productive positions are described as those positions within a molecule that are most useful for making combinatorial variants exhibiting an improved characteristic, where the position itself allows for at least one combinable mutation. Combinable mutations can be described as those substitutions in a molecule that can be used to make combinatorial variants. Combinable mutations are ones that improve at least one desired property of the molecule, while not significantly decreasing either: expression, activity, or stability.

Combinable mutations are ones that improve at least one desired property of the molecule, while not significantly decreasing either: expression, activity, or stability. Combinable mutations in LG12 sprC were determined using performance index (PI) values resulting from the assays described in Example 1 and listed below.
Protein Determination (expression) is shown in Example 1.
Protease activity (AAPF assay) is shown in Example 1.
Cleaning activity in the following detergents: Kirkland Ultra HDD pH 10.6, OMO Color HDD pH 10.6, OMO Klein & Krachtig HDL pH 8.2, Blue Moon HDL pH6.5, GSM-B, GSM-B pH 9, GSM-C, Sun All in One, Finish Quantum tablet.
Stability assays in the following detergent and buffers: OMO Klein & Krachtig HDL pH 8.2 at 51°C, GSM-B at 68.5°C, GSM-C at 62°C, LAS-EDTA buffer at 52°C, Tris EDTA buffer at 51°C, Tris-calcium buffer at 72.5°C.

Combinable mutations have been grouped according to the following criteria:
A variant where the minimum performance indices (PI) relative to LG12 sprC parent for expression, protease activity, activity in one of more of the cleaning activity assays, and one or more of the stability assays are greater than or equal to 0.9, and in addition have a PI for any one of these tests that is greater than or equal to 1.0 (**Group A**).
A variant where the minimum performance indices (PI) relative to LG12 sprC parent for expression, protease activity, activity in one of more of the cleaning activity assays, and one or more of the stability assays are greater than or equal to 0.8, and in addition have a PI for any one of these tests that is greater than or equal to 1.2 (**Group B**).
A variant where the minimum performance indices (PI) relative to LG12 sprC parent for expression, protease activity, activity in one of more of the cleaning activity assays, and one or more of the stability assays are greater than or equal to 0.5, and in addition have a PI for any one of these tests that is greater than or equal to 1.5 (**Group C**).

The properties of combinable mutations are summarized in Table 3

| **Table 3. Properties for each group of combinable mutations** | | | | | |
|---|---|---|---|---|---|
| | **Performance Index (PI)** | | | | |
| **Group** | **Expression** | **Cleaning (pH 6, or 8, or 9, or 10)** | **Stability (detergent or buffer)** | **Activity in AAPF assay** | **Minimum PI in one or more tests** |
| A | ≥ 0.9 | ≥ 0.9 | ≥ 0.9 | ≥ 0.9 | X ≥ 1.0 |
| B | ≥ 0.8 | ≥ 0.8 | ≥ 0.8 | ≥ 0.8 | X ≥ 1.2 |
| C | ≥ 0.5 | ≥ 0.5 | ≥ 0.5 | ≥ 0.5 | X ≥ 1.5 |

Groups A, B, and C further contain amino acid positions that have differing degrees of tolerance for multiple substitutions. To identify productive positions, we measure the degree of substitutions tolerated at each position and assign a Productivity Score to each position. The Productivity Score was assigned according to the percentage of substitutions within each position that fall within groups A, B, or C, using the criteria set forth below.

Productive positions are defined as the positions which have shown a certain degree of tolerance for multiple substitutions, while at the same time meeting a set of criteria for combinability as set forth below.

The criteria to determine the Productivity Score for productive positions are as follows:
Positions where less than 15% of the substitutions at a given position fall within groups A, B, or C are given a Productivity Score of **"1".**
Positions where less than 30%, but greater than, or equal to 15% of the substitutions at a given position fall within groups A, B, or C are given a Productivity Score of **"2".**
Positions where less than 50%, but greater than, or equal to 30% of the substitutions at a given position fall within groups A, B, or C are given a Productivity Score of **"3".**
Positions where 50% or more of the substitutions at a given position fall within groups A, B, or C are given a Productivity Score of **"4".**
**Positions with Productivity Score of "1" are listed below:**
4, 7, 8, 12, 14, 16, 17, 29, 33, 42, 46, 51, 56, 65, 69, 73, 81, 83, 84, 88, 94, 104, 109, 111, 114, 120, 122, 125, 128, 132, 138, 141, 143, 146, 151, 163, 165, 167, 175, 176, 195, 203, 204, 205, 206, 211, 212, 215, 220, 224, 227, 230, 232, 238, 250, 260, 262, 268, 273, and 274.
**Positions with Productivity Score of "2" are listed below:**
1, 3, 18, 19, 20, 22, 24, 25, 26, 28, 30, 31, 44, 50, 53, 55, 57, 58, 61, 67, 72, 76, 77, 79, 80, 85, 86, 87, 89, 90, 91, 93, 96, 100, 101, 102, 106, 107, 108, 113, 116, 121, 124, 126, 127, 136, 137, 140, 144, 147, 150, 152, 153, 160, 162, 170, 172, 173, 183, 185, 187, 188, 194, 213, 216, 217, 218, 222, 225, 228, 231, 234, 235, 241, 243, 244, 246, 252, 255, 265, and 272.
**Positions with Productivity Score of "3" are listed below:**
2, 9, 15, 38, 45, 48, 52, 59, 62, 75, 97, 99, 103, 117, 118, 119, 123, 129, 133, 145, 148, 149, 156, 159, 161, 181, 209, 236, 240, 248, 256, 267, 271, and 275.
**Positions with Productivity Score of "4" are listed below:**
27, 40, 43, 78, 98, 130, 131, 158, 166, 182, 237, 239, 242, 245, and 251.

Combinable mutations in LG12 sprC were determined using performance index (PI) values resulting from the assays described in Example 1: Protein Determination (expression), AAPF activity, Cleaning activity in the following detergents (Kirkland Ultra HDD pH 10.6, OMO Color HDD pH 10.6, OMO Klein & Krachtig HDL pH 8.2, Blue Moon HDL pH6.5, GSM-B, GSM-B pH 9, GSM-C, Sun All in One, and Finish Quantum tablet), and Stability assays in the following detergent and buffers (OMO Klein & Krachtig HDL detergent pH 8.2, GSM-B detergent, GSM-C detergent, LAS-EDTA buffer, Tris EDTA buffer, and Tris-calcium buffer at 72.5°C).

Combinable mutations were assigned to groups A, B or C according to criteria set forth in Example 4. These substitutions are further assigned a Suitability Score based on the group(s) (A, B, C) where the substitution appears, and where a higher suitability scores represents a substitution more suitable for use in making combinatorial variants. Suitability scores are defined in Table 4. Suitability scores for individual substitutions of LG12 sprC that fit the above criteria are reported in Table 4.

**Table 3.2: Definitions of suitability scores**

| **Substitutions Occur in Group(s):** | **Suitability Score** |
|---|---|
| A, B and C | +++++ |
| A and B | ++++ |
| A or (B and C) | +++ |
| B | ++ |
| C | + |

| **Table 4: Suitability scores of individual substitutions in LG12 sprC** | | | | | |
|---|---|---|---|---|---|
| | VARIANTS SUITABILITY SCORE | | | | |
| POS | (+) | (++) | (+++) WT AA 1ST | (++++) | (+++++) |
| 1 | | L | AHK | GR | |
| 2 | | | QE | | FKPRSWY |
| 3 | A | | TGH | EY | IV |
| 4 | C | | V | | |
| 7 | | | G | N | |
| 8 | | | IL | | |
| 9 | Y | | P | HQ | AGIMRSTV |
| 12 | | | K | GR | |
| 14 | L | | D | | |
| 15 | | MQT | KG | HRVY | A |
| 16 | | | AGV | | |
| 17 | A | | H | | |
| 18 | D | FS | AQ | NRT | |
| 19 | | F | A | KLR | MW |
| 20 | | HQ | GE | | N |
| 22 | | | T | DS | E |
| 24 | | G | SFT | HIRV | |
| 25 | | | GHI | AM | |
| 26 | ER | | V | NS | |
| 27 | | HN | K | ADEGLPQRST | M |
| 28 | | NS | V | ET | |
| 29 | | | AT | S | |
| 30 | T | | IAE | V | |
| 31 | | I | LMQ | T | |
| 33 | D | | T | | |
| 38 | | HY | N | AQT | DEKS |
| 40 | I | | AQ | LTW | DEHKMNRVY |
| 42 | D | | L | | |
| 43 | | V | NI | AFGHKLMQSWY | DE |
| 44 | AI | | V | F | |
| 45 | | L | K | DEFQRSW | A |
| 46 | | | GN | | |
| 48 | | | AW | DEGHKNQ | |
| 50 | | | FRS | AKQY | |
| 51 | | | VI | | |
| 52 | E | | STV | DHLMNQR | F |
| 53 | Y | H | G | ADNR | E |
| 55 | I | | P | KNR | |
| 56 | | | NE | | |
| 57 | | | AGW | P | |
| 58 | | | LF | D | K |
| 59 | FM | HNW | QT | I | V |
| 61 | T | | GA | EY | M |
| 62 | EIV | A | NF | KL | PW |
| 65 | T | | G | V | |
| 67 | | | HG | AI | P |
| 69 | | | AG | T | |
| 72 | | M | V | AGST | |
| 73 | | | A | G | |
| 75 | AN | HK | L | FQRW | G |
| 76 | FP | | NA | QSY | D |
| 77 | Q | | NGT | | |
| 78 | C | | TS | IKLV | ADEFGHRW |
| 79 | EM | | TA | KQ | IL |
| 80 | CP | FHM | G | Y | |
| 81 | H | | V | | M |
| 83 | | | G | S | |
| 84 | | | V | A | |
| 85 | | | ATV | S | |
| 86 | | | YA | P | F |
| 87 | | Q | NM | HLS | |
| 88 | C | G | A | | |
| 89 | | AR | DF | | |
| 90 | DS | | LGQ | | ENP |
| 91 | | | YH | IMNTVW | |
| 93 | | GI | V | LT | |
| 94 | G | | K | | |
| 96 | GKRY | | L | I | EH |
| 97 | | | SD | GHLMN | AI |
| 98 | Y | V | ADFKNW | HMQST | |
| 99 | ADNQR | | S | IKLV | |
| 100 | FKVY | | G | | AE |
| 101 | | M | S | ATV | R |
| 102 | V | | GF | | EY |
| 103 | E | AY | TG | HKNRS | D |
| 104 | Y | | L | | V |
| 106 | A | I | G | ENSTV | |
| 107 | | | IHQ | V | T |
| 108 | | S | AI | C | |
| 109 | | | Q | K | |
| 111 | | | IV | | |
| 113 | | Y | WF | H | |
| 114 | | T | S | G | |
| 116 | | | S | EF | M |
| 117 | MQ | | NHKR | EGSTY | |
| 118 | | T | GKRY | DEHQS | |
| 119 | NV | | M | AFHILQST | |
| 120 | | | NH | E | |
| 121 | T | S | V | A | I |
| 122 | | M | I | | |
| 123 | IS | | N | | ADEGHQ |
| 124 | RSY | | M | | A |
| 125 | G | | S | | |
| 126 | HW | | LIQ | G | K |
| 127 | EHN | | G | | |
| 128 | E | | G | | N |
| 129 | EIR | Y | SKQ | AGH | D |
| 130 | IR | | S | HLNQTW | EFVY |
| 131 | IKLT | HN | GA | PRS | DE |
| 132 | | A | S | | |
| 133 | G | H | T | AFLMNQV | DI |
| 136 | | L | QHW | FR | D |
| 137 | MV | I | Q | HT | E |
| 138 | | | A | ES | |
| 140 | I | F | N | Y | DQ |
| 141 | | | NG | | |
| 143 | | D | Y | | E |
| 144 | DF | | N | HMVWY | |
| 145 | DFGLV | | RM | WY | N |
| 146 | | N | G | | R |
| 147 | | R | IK | S | AHM |
| 148 | DILMQ | | VN | | GS |
| 149 | | | V | APS | EGLNQ |
| 150 | | | I | AS | DNTV |
| 151 | | | A | | H |
| 152 | | G | A | | PV |
| 153 | M | | A | GS | |
| 156 | CE | G | SDHQ | AM | KN |
| 158 | EGHILQ | | SK | A | DMTV |
| 159 | CFT | | S | MNY | DEKQ |
| 160 | N | HM | G | | |
| 161 | | | NFKW | MS | DERY |
| 162 | KMV | | R | | |
| 163 | S | | N | | D |
| 165 | I | | M | V | |
| 166 | KW | | GC | FIL | ADEHMNQSTY |
| 167 | G | | Y | | |
| 170 | P | | R | KL | |
| 172 | C | NT | SG | H | |
| 173 | | A | ST | | D |
| 175 | | | IS | | Q |
| 176 | G | | A | | |
| 181 | T | | SDEQ | AHK | N |
| 182 | CQ | PW | s | AHIMRY | DE |
| 183 | G | AQ | N | | DE |
| 185 | CW | | T | QR | M |
| 187 | C | | A | G | DHPVW |
| 188 | CLN | T | SH | Y | |
| 194 | | M | ST | FKPR | D |
| 195 | | Q | E | | |
| 203 | C | | V | | |
| 204 | | | NE | | |
| 205 | | | IV | | |
| 206 | | | L | Y | |
| 209 | I | | TKM | V | FHWY |
| 211 | C | | G | | |
| 212 | GY | | N | | |
| 213 | VW | GM | NR | KQ | |
| 215 | | | A | DE | |
| 216 | C | | S | FIT | A |
| 217 | CHKL | | FE | | R |
| 218 | CH | | N | | D |
| 220 | AG | | T | | |
| 222 | C | | M | A | HLQ |
| 224 | | | A | S | |
| 225 | AGV | | P | | |
| 227 | | A | VM | | |
| 228 | CR | | A | S | |
| 230 | | | AS | | |
| 231 | C | GS | A | | |
| 232 | | S | A | | |
| 234 | MQ | | I | V | |
| 235 | INS | | KG | DEF | |
| 236 | V | | A | FGN | CDEQS |
| 237 | LMTY | FS | KD | GHR | ANQ |
| 238 | | W | Y | F | |
| 239 | | | PY | CEFGHINRSTW | ADKLMV |
| 240 | | | SHQ | FILPVW | M |
| 241 | | HK | M | ILQSW | |
| 242 | | GH | TL | IKNVW | AEPQS |
| 243 | | | NAT | G | |
| 244 | AE | | V | | Y |
| 245 | C | | QAFHMNRTW | DSY | |
| 246 | | N | IV | AT | |
| 248 | Q | KLNTW | ED | CHMY | |
| 250 | | | L | IM | |
| 251 | CGHY | W | KFL | R | ADMNSTV |
| 252 | D | Q | N | F | E |
| 255 | | Q | T | DEI | |
| 256 | | V | NA | DKMQW | EPR |
| 260 | Y | | P | | |
| 262 | | | FE | | |
| 265 | R | | K | | DS |
| 267 | H | | VN | A | FIKSTY |
| 268 | F | | I | | |
| 271 | DL | MQ | E | HK | AFWY |
| 272 | PV | M | S | FNQ | K |
| 273 | | | A | | S |
| 274 | | I | L | | |
| 275 | | LTY | QGHW | ENR | |

### Evaluation of beneficial LG12-clade subtilisin mutations for certain cleaning applications

The SEL variants were further grouped in various categories based on their performance in different assay conditions as described below. For this analysis, the SEL variants for which the HPLC PI values were less than 0.75 were excluded. In addition, for each assay, PI values were not considered if the corresponding CV value were less than 0 or larger than 100.
To obtain best mutations for each category of performance, in addition to HPLC (relative protein expression) PI values the following measurements were considered:
1) HDD PI value 1.01 or greater in: Kirkland Ultra HDD pH 10.6, or OMO Color HDD pH 10.6
2) HDL (pH 8) PI value 1.01 or greater in: OMO Klein & Krachtig HDL pH 8.2
3) HDL (pH 6) PI value 1.01 or greater in: Blue Moon pH6.5
4) Laundry stability PI value 1.01 or greater in detergent and buffers: OMO Klein & Krachtig HDL pH 8.2 at 51 °C or LAS-EDTA buffer at 52°C.
5) ADW PI value 1.01 or greater in detergents: GSM-B, GSM-B pH 9, GSM-C, Sun All in One, or Finish Quantum tablet.
6) ADW stability PI value 1.01 or greater in detergent and buffers: GSM-B at 68.5°C, GSM-C at 62°C, Tris EDTA buffer at 51 °C, Tris-calcium buffer at 72.5°C.
Criteria for grouping in each category: PI > 1.01 for the specific category as listed on Table 7, and PI >0.75 for expression.

### List of most beneficial mutations in laundry stability

A029T, I030A, I030V, A040D, A040E, A040H, A040K, A040L, A040N, A040R, A040T, A040V, A040W, A040Y, S101R, T103D, T103E, T103G, T103K, T103R, S052D, S052F, S052N, S052R, N183E, V093T, L031T, N117E, N117F, N117S, N117T, M124S, L126D, L126G, L126I, L126K, L126Q, N123D, S125G, S129A, S129D, S129K, S129Q, S130E, S130F, S130H, S130L, S130N, S130Q, S130T, S130V, S130W, S130Y, G131D, G131E, G131R, Q137E, S099K, G102E, G102F, G102Y, I107H, I107Q, N120E, V121G, S182D, S182E, T003E, T003I, T003V, A224P, A224S, K015V, P225G, T185Q, K027E, K027L, K027M, K027S, K027T, N043A, N043D, N043E, N043F, N043L, N043W, Q136D, Q136H, Q136R, Q136W, N163D, E248C, E248D, A019M, T022D, T022E, R145N, R145T, R145W, S158D, L090P, G100A, G100E, G100F, I147A, I147H, I147S, V148N, V148S, Q245M, Q245T, Q245V, I246N, N243G, S024H, S024T, S024V, K045D, K045E, K045F, K045L, K045Q, K045R, K045S, K045W, Q275E, Q275N, A048D, A048E, A048G, A048H, A048Q, A048Y, T078A, T078D, T078E, T078F, T078G, T078H, T078K, T078L, T078S, T078W, N087S, Y091H, Y091N, M241Q, M241S, M241W, V244Y, N252E, K265D, K265S, S272M, S272Q, N038A, N038D, N038E, N038T, K251A, K251C, K251F, K251M, K251N, K251R, K251S, K251T, K251V, F050Q, F050R, F050Y, V051I, N076D, N076S, T079L, T079Q, L075G, G166A, G166C, G166D, G166E, G166F, G166H, G166L, G166M, G166N, G166Q, G166S, G166T, G166Y, G053D, G053E, G053Q, G053R, Y143D, Y143E, S181A, S181H, S181N, S181Q, G154F, G154H, G154I, G154K, G154T, S194K, S194P, S194R, K237A, K237N, A215D, A215E, Y171G, Y171Q, K012G, V072A, V072T, S156A, S156D, S156H, S156K, S156M, S156N, S156Q, T255D, N256D, N256E, N256P, N256W, F262E, G106E, T133D, T133F, T133M, T133N, T133V, N161D, N161E, R170K, R170L, A108C, A108I, S114G, S114T, G118D, G118E, G118Q, G118S, G118T, G146N, A138E, A138S, N140D, V149A, V149E, V149G, V149L, V149N, V149Q, V149S, I150A, I150N, I150S, I150T, I150V, A152P, A152V, S216I, S216T, A228S, P239A, P239C, P239D, P239E, P239F, P239G, P239H, P239M, P239N, P239T, P239W, F217R, T220G, S159D, S159E, S159N, S159Q, G128N, T209F, T209V, T209W, T209Y, A236C, A236D, A236E, A236N, A236Q, A236S, S240P, S240Q, T242E, S173D, P009A, P009I, P009M, P009Q, P009S, P009T, P009V, L096E, L096H, L096I, L096R, L096Y, S116E, V081M, K235D, K235E, K235F, K235G, V267F, V267I, V267R, V267S, V267T, V267Y, M222A, M222H, and M222Q

### List of most beneficial mutations in HDL pH6 performance

I030A, A040E, A040L, A040T, A040Y, T103D, T103E, S052D, S052F, S052N, N183E, L031T, N117E, N117F, M124S, N123D, S129D, S130E, Q137E, G102E, I107Q, N120E, V121G, S182D, S182E, T003E, A224S, K015V, K027E, K027L, K027M, K027S, K027T, N043A, N043D, N043E, N043F, N043W, Q136D, N163D, T022D, T022E, R145N, R145T, R145W, S158D, L090P, G100A, G100E, I147A, I147H, I147S, V148S, Q245T, Q245V, I246N, S024T, S024V, K045D, K045E, K045F, K045L, K045Q, K045R, K045S, K045W, Q275E, Q275N, A048D, A048E, A048Q, T078D, T078E, T078F, T078L, T078W, N087S, Y091H, M241S, M241W, K265D, K265S, S272M, N038A, N038D, N038E, N038T, K251A, K251C, K251F, K251M, K251N, K251R, K251S, K251T, K251V, F050Q, T079L, G166D, G166E, G166H, G166N, G166Y, G053D, G053E, Y143D, Y143E, S181H, K237A, K237N, A215D, A215E, K012G, V072T, S156D, S156H, S156N, S156Q, T255D, N256D, N256E, F262E, G106E, T133D, N161D, N161E, R170L, A108C, S114G, S114T, G118D, G118E, G118Q, G118S, G118T, G146N, A138E, A138S, N140D, V149A, V149E, V149G, V149L, V149N, I150A, I150N, I150T, I150V, S216I, S216T, A228S, P239C, P239D, P239E, P239F, P239H, P239M, P239N, P239T, P239W, S159D, S159E, S159N, T209F, T209V, A236D, A236E, A236N, A236Q, A236S, T242E, S173D, P009A, P009I, P009M, P009Q, P009S, P009T, P009V, L096E, K235D, K235E, and V267S

### List of most beneficial mutations in HDL pH8 performance

A029G, A029T, T033N, T033V, A040D, A040E, G065A, A069G, K094A, K094D, K094E, K094H, K094L, K094M, K094N, K094S, K094T, V095A, V095S, G110S, I111M, Y086D, Y086E, Y086I, Y086P, Y086S, Y086T, S101A, T103A, T103D, T103E, T103N, L104I, L104T, S052D, S052F, S052G, S052H, S052L, L058V, N183E, G083A, V084E, V093I, L031A, L031E, L031N, L031Q, G034A, G034E, A037D, A037E, A037F, A037H, A037L, A037M, A037N, A037Q, A037T, A037W, V068D, V068Q, M124S, L126Q, M119A, M119F, M119H, M119I, M119L, N123A, N123D, N123E, N123G, N123H, N123I, N123Q, A187D, A187H, N062A, N062K, N062L, N062P, S129D, S130E, S130P, Q137E, N144M, S097G, S097H, S097I, S097L, S097M, S097Y, G102E, I107Q, N120P, S182D, S182E, A001G, A001H, A001L, T003E, V004E, D014E, E197D, L206Y, I011A, I011S, I011T, A013S, K015H, K015M, K015N, K015P, K015Q, K015S, K015T, K015V, K015Y, T185M, T185Q, P005D, P005T, K027D, K027E, K027G, K027L, K027M, K027N, K027Q, K027T, K027Y, N043D, N043E, S049A, S049D, S049E, S049G, S049H, S049I, S049K, S049N, S049Q, S049T, S049V, S049W, Q136D, N163D, N163E, G211D, G211Q, G211T, W006A, W006D, W006H, W006L, W006M, W006N, W006P, W006Q, W006S, A019K, A019W, T022G, T022L, G061E, R145N, R145T, R145W, S158D, S158H, S158Q, L090E, L090G, L090N, L090Q, G100A, G100E, I147H, I147M, I147S, V148A, V148D, V148E, V148F, V148G, V148L, V148Q, T164D, T164E, N184A, N184H, F189A, F189D, F189E, F189I, F189L, F189S, F189V, Q245M, Q245N, Q245P, Q245T, Q245V, I246A, I246C, L274D, L274E, L274G, N243D, H017I, V021D, L257C, L257G, L257I, S024G, K045E, K045L, K045W, Q275L, Q275T, A048D, A048E, A048G, A048H, A048L, E054F, E054G, E054H, E054I, E054L, E054M, E054N, E054P, E054R, E054T, E054V, E054W, A073I, T078D, T078E, T078F, T078G, T078H, T078W, N087F, N087H, N087P, N087Q, N087S, A088D, A088G, A088N, A088P, A088Q, D089F, D089K, D089L, Y091T, Y091V, Y091W, G020E, G020H, G020L, G020V, G020Y, G046E, G046L, G046M, N252E, N252F, N252I, N252L, N252Q, N252S, F261Y, K265D, K265V, S272F, S272M, S272Q, S272V, N056A, N056E, N056H, N056I, N056L, N056M, N056Q, N056S, N056V, A074G, N077D, N077G, N077P, T208A, A231F, A231L, A018F, A018K, A018L, A018N, A018P, A018Q, A018S, A018T, A018V, A018W, A018Y, N038D, N038E, N038H, N038I, N038K, N038P, N038Q, N038T, N038Y, K251A, K251C, K251F, K251M, K251N, K251R, K251S, K251T, K251V, E271A, E271D, E271F, E271M, E271W, F050I, F050S, F050Y, V051D, V051E, V051F, V051G, V051K, V051L, V051M, V051N, V051P, V051Q, V051R, V051T, V051W, V051Y, A057C, A057D, A057E, A057I, A057K, A057L, A057M, A057N, A057Q, A057R, A057S, A057T, A057V, A057Y, L075A, L075D, L075F, L075N, L075Q, L075S, L075T, L075V, L075W, G166A, G166C, G166D, G166E, G166H, G166L, G166M, G166N, G166Q, G166S, I175A, I175F, I175Q, I175S, I175T, I175V, G053A, G053D, G053E, G053I, G053L, G053P, P055A, P055G, P055M, P055W, P055Y, S194G, A232H, A232I, A232N, A232Q, A232S, A232T, I234M, I234N, I234S, I234T, I234V, I234W, K237A, K237D, K237F, K237L, K237M, K237N, K237T, K237V, K237W, A215D, A215E, A215M, V203A, V203D, V203E, V203G, V203I, V203L, L233G, L233H, L233N, L233T, L250I, K012D, K012E, K012G, K012H, K012L, K012M, K012T, K012Y, V072A, V072D, V072E, V072F, V072G, V072I, V072T, Y167D, Y167E, Y167F, Y167P, S156D, S156N, S172N, Y238A, Y238C, Y238E, Y238F, Y238G, Y238S, Y238W, T255L, N256D, N256E, I035A, I035F, I035G, I035L, I035M, I035T, V227F, V227S, V227Y, F262E, F262I, G106E, G106T, T133D, N161D, N161E, N161F, N161S, N161W, N161Y, R170I, R170L, R170M, A108C, A108I, S114T, G118D, G118E, G118Q, G118S, G118T, G146H, G146N, G146Q, D060E, D060N, D060S, W113F, A138E, N140D, N140G, V149D, V149F, V149G, V149L, I150A, N212I, N212L, N212Q, N212W, S216D, A228G, P239C, P239G, P239H, P239L, P239N, Q002E, Q002S, F217D, F217E, F217I, F217T, H226D, H226E, H226L, H226Q, H226T, A230F, A230H, A230I, A230M, A230Q, Q059I, A016H, A016W, P210D, P210E, P210W, S159D, S159E, S159N, G063D, G063E, G063L, G063M, G063N, G063S, G063T, G063V, A098F, A098S, I122L, V198A, V198C, V198F, V198T, T209M, T209Q, T209V, N213D, N213E, A236C, A236D, A236E, A236F, A236L, S240F, S240P, T242E, T242Q, P260C, G258D, N141G, S173D, Y214M, P009A, P009G, P009I, P009T, H010L, L096E, L096H, L096I, S116E, S116F, G025A, G025F, V081P, H039G, H039T, K235D, K235E, V267L, V267N, A151H, G160F, G160H, G160M, G160V, M222L, and M222Y

### List of most beneficial mutations HDD performance

A029G, A029S, A029T, I030S, I030V, I030Y, T033N, T033V, A040D, A040E, A040G, A040H, A040K, A040L, A040N, A040P, A040Y, G065A, T066S, H067A, H067E, H067F, H067G, H067K, H067L, H067M, H067N, H067P, H067Q, H067S, H067T, H067V, H067Y, A069G, K094A, K094D, K094E, K094H, K094L, K094M, K094N, K094S, K094T, V095A, V095S, V095T, G110S, I111M, I111V, Y086D, Y086E, Y086I, Y086K, Y086M, Y086P, Y086Q, Y086T, Y086W, S101A, S101M, S101T, S101V, T103A, T103D, T103E, T103G, T103L, T103N, T103S, T103Y, L104I, S052D, S052F, S052G, S052H, S052L, S052M, S052N, S052Q, S052T, S052V, L058A, L058D, L058H, L058I, L058R, L058T, L058V, N183E, N183I, N183M, N183P, N183Y, G083A, G083S, V084L, V084S, V084T, A085I, A085S, V093G, V093I, V093M, V093T, L031A, L031E, L031N, L031Q, L031T, G034A, G034E, A037D, A037E, A037F, A037H, A037L, A037M, A037N, A037Q, A037R, A037S, A037T, A037W, V068D, V068I, V068M, V068Q, N117E, N117F, N117K, N117M, N117S, N117T, N117Y, M124S, L126G, L126I, L126Q, M119A, M119F, M119H, M119L, M119Q, M119S, M119T, N123A, N123D, N123E, N123G, N123H, N123I, N123Q, A187D, A187F, A187G, A187H, A187N, A187P, A187W, N062A, N062K, N062L, N062P, N062W, S129A, S129D, S129G, S129Q, S129T, S129Y, S130E, S130L, S130N, S130P, S130Q, S130T, G131D, G131P, G131Q, G131S, S132A, Q137E, Q137H, Q137I, Q137T, N144H, N144V, N144Y, A153S, S097G, S097H, S097I, S097K, S097L, S097M, S097N, S097Y, S099I, S099L, S099V, G102E, I107Q, N120G, N120P, N120T, V121A, V121F, V121G, V121S, V177I, V177T, A179G, S182A, S182D, S182E, S182F, S182L, S182M, S182N, S182W, A001G, A001K, A001L, A001R, T003E, T003H, T003I, T003V, T003Y, V004E, V004F, V004G, V004H, V004P, V004S, V004Y, D014E, D014F, D014G, D014H, D014T, D014Y, E197D, I205A, I205C, I205M, I205N, L206F, L206K, L206M, L206N, L206R, L206S, L206V, L206W, L206Y, A224S, I011A, I011M, I011T, I011V, A013S, K015H, K015M, K015N, K015P, K015Q, K015S, K015T, K015V, K015Y, A092V, T185F, T185G, T185H, T185M, T185Q, P005D, P005I, P005M, P005S, P005T, P005V, G007Y, K027D, K027E, K027G, K027H, K027L, K027M, K027N, K027P, K027Q, K027R, K027S, K027T, K027Y, N043A, N043D, N043E, N043F, N043G, N043H, N043I, N043K, N043L, N043M, N043Q, N043S, N043V, N043W, S049A, S049D, S049E, S049G, S049H, S049I, S049K, S049N, S049Q, S049R, S049T, S049V, S049W, Q136D, Q136G, Q136H, Q136L, Q136N, Q136V, N163D, N163E, N204L, G211H, G211K, G211Q, G211T, E248A, E248H, I008A, I008G, I008H, I008N, I008P, I008T, I008V, I008Y, V028E, V028F, V028H, V028I, V028L, V028M, V028N, V028Q, V028S, V028T, L042I, L042M, L042N, L042Q, L042T, L042V, W006A, W006D, W006H, W006K, W006L, W006M, W006N, W006P, W006Q, W006S, W006Y, A019F, A019K, A019L, A019M, A019R, A019W, A019Y, T022E, T022G, T022I, T022L, T022M, T022Q, T022R, T022S, T022V, T022W, V026E, V026F, V026H, V026L, V026N, V026Q, V026R, V026S, G061A, G061E, G061I, G061M, G061P, G061S, G061T, G061Y, R145N, R145T, S158D, S158G, S158H, S158M, S158N, S158Q, S158T, S158V, L090E, L090F, L090G, L090N, L090P, L090Q, L090R, L090Y, G100A, G100E, I147A, I147H, I147M, I147S, V148D, V148E, V148G, V148L, V148M, V148Q, V148S, N184A, N184S, F189A, F189D, F189E, F189G, F189H, F189I, F189K, F189L, F189M, F189N, F189P, F189Q, F189S, F189T, F189V, P201L, P201S, P201T, Q245A, Q245F, Q245M, Q245N, Q245P, Q245R, Q245S, Q245T, Q245W, Q245Y, I246A, I246F, I246M, I246V, L274F, L274M, L274Y, N243D, N243K, N243L, N243P, N243R, T253F, T253G, T253K, T253N, A254G, A254T, H017I, H017L, H017P, H017S, H017T, H017V, V021D, V021F, V021G, V021H, V021K, V021M, V021R, L257A, L257C, L257G, L257H, L257I, L257M, L257P, L257Q, L257V, V270I, V270L, V270M, V270Q, V270T, V270W, V270Y, S024G, S024H, S024I, S024P, S024R, S024T, S024V, K045D, K045L, K045Q, K045R, K045S, K045W, Q275K, Q275L, Q275N, Q275R, Q275W, Q275Y, A048D, A048E, A048G, A048H, A048I, A048K, A048L, A048N, A048Q, A048W, E054D, E054F, E054G, E054H, E054I, E054K, E054L, E054M, E054N, E054P, E054R, E054S, E054T, E054V, E054W, A073G, A073I, A073K, A073L, A073T, A073V, T078A, T078F, T078H, T078I, T078L, T078R, T078V, T078W, N087F, N087H, N087P, N087Q, N087S, A088D, A088G, A088N, A088P, A088Q, D089F, D089K, D089L, D089R, D089T, D089W, Y091H, Y091I, Y091M, Y091N, Y091V, Y091W, G020E, G020H, G020L, G020N, G020Q, G020T, G020V, G020Y, M241H, M241I, M241Q, M241S, V244L, V244Q, N252F, N252I, N252L, N252Q, N252R, N252S, F261Y, K265S, K265V, S272F, S272H, S272K, S272M, S272N, S272P, S272Q, S272T, S272V, N056A, N056E, N056I, N056L, N056M, N056P, N056Q, N056S, N056V, A074G, A074S, N077G, L135I, L196M, T208A, T208I, T208L, T208N, T208V, A231G, A231L, A231S, A231Y, R249K, R249W, A018F, A018K, A018L, A018N, A018S, A018T, A018W, A018Y, N038A, N038D, N038E, N038I, N038K, N038P, N038T, N038Y, K251A, K251C, K251F, K251M, K251N, K251R, K251S, K251T, K251V, E271A, E271D, E271F, E271L, E271M, E271Q, E271W, E271Y, F050A, F050I, F050K, F050Q, F050S, F050W, F050Y, V051A, V051D, V051E, V051F, V051G, V051H, V051I, V051K, V051L, V051M, V051N, V051P, V051Q, V051R, V051T, V051W, V051Y, N076D, N076E, N076I, N076K, N076Q, N076R, N076S, N076T, N076Y, T079K, T079L, T079Q, A057C, A057D, A057E, A057I, A057K, A057L, A057M, A057N, A057P, A057Q, A057R, A057S, A057T, A057V, A057Y, L075A, L075D, L075E, L075F, L075G, L075H, L075I, L075K, L075N, L075Q, L075R, L075S, L075T, L075V, L075W, G166A, G166C, G166D, G166E, G166F, G166H, G166I, G166M, G166N, G166Q, G166S, G166Y, I175A, I175F, I175Q, I175S, I175T, I175V, G053A, G053D, G053E, G053I, G053L, G053N, G053P, G053Q, P055A, P055G, P055K, P055M, P055N, P055R, P055S, P055W, P055Y, Y143E, Y143L, Y143M, Y143N, Y143Q, Y143T, Y143V, S181A, T071A, T071G, T071M, T071N, T071P, T071S, T071V, S194G, S194I, S194M, S194V, A232H, A232I, A232L, A232M, A232N, A232Q, A232S, A232T, I234A, I234F, I234M, I234N, I234Q, I234S, I234V, I234W, I234Y, K237F, K237G, K237I, K237L, K237M, K237N, K237Q, K237R, K237S, K237T, K237V, K237W, A215D, A215E, A215F, A215G, A215H, A215K, A215L, A215M, A215N, A215Y, A169S, V203A, V203D, V203E, V203G, V203L, V203T, L233A, L233D, L233G, L233H, L233M, L233T, L250I, L250T, K012D, K012E, K012G, K012H, K012L, K012M, K012R, K012T, K012Y, D036A, D036E, D036W, V072A, V072D, V072E, V072F, V072K, V072P, V072Q, V072T, Y167A, Y167D, Y167E, Y167F, V180A, V180N, V180S, V180T, G080F, G080M, G080Y, S156A, S156D, S156H, S156I, S156L, S156M, S156N, S156Q, S156V, S172H, S172I, S172L, S172M, S172N, S172T, S172V, S172Y, S188G, S188H, S188T, S188Y, Y238A, Y238C, Y238D, Y238F, Y238G, Y238I, Y238K, Y238L, Y238M, Y238Q, Y238T, Y238V, Y238W, T255A, T255D, T255F, T255G, T255H, T255I, T255K, T255L, T255M, T255N, T255Q, T255S, T255W, T255Y, N256D, N256E, N256K, N256L, N256M, N256P, N256Q, N256R, N256V, N256W, I035A, I035F, I035G, I035L, I035M, I035T, V227F, V227S, V227T, V227Y, F262E, F262S, G106E, G106I, G106N, G106S, T133A, T133D, T133F, T133L, T133M, T133N, T133Q, T133V, N161D, N161E, N161F, N161M, N161Q, N161W, N161Y, R170K, R170L, R170M, A108C, A108I, A108L, S114G, S114T, G118E, G118H, G118Q, G118R, G118S, G118T, G118Y, G146Q, G146R, D060A, D060E, D060F, D060H, D060K, D060L, D060M, D060N, D060P, D060Q, D060S, D060T, D060V, D060Y, W113F, W113H, W113Y, A138E, A138S, N140D, N140F, N140G, N140I, N140L, N140M, N140Q, N140T, N140V, N140W, N140Y, V149A, V149D, V149E, V149F, V149G, V149H, V149I, V149L, V149N, V149P, V149Q, V149S, V149T, I150A, I150M, I150N, I150T, I150V, N212F, N212G, N212L, N212P, N212W, S216A, A228S, P239F, P239I, P239K, P239T, P239V, P239W, Q002E, Q002K, Q002P, Q002S, Q002W, Q002Y, S207T, S207V, F217D, F217E, F217H, F217I, F217R, F217T, F217V, H226D, H226E, H226F, H226G, H226L, H226Q, H226T, A230D, A230F, A230H, A230M, A230Q, A230Y, Q059I, Q059N, A016E, A016G, A016H, A016I, A016K, A016L, A016Q, A016T, A016W, G023A, A200S, P210D, P210E, P210F, P210I, P210L, P210Q, P210S, P210T, P210V, P210W, S159D, S159E, S159N, S159Q, S159T, G063A, G063D, G063E, G063F, G063H, G063L, G063M, G063N, G063P, G063R, G063S, G063T, G063V, G063W, G063Y, A098H, A098L, A098M, A098P, A098Q, A098S, A098T, A098V, Q109K, I122L, I122M, G128N, V198A, V198C, V198F, T209E, T209M, T209Q, T209R, T209S, T209V, T209W, N213A, N213D, N213E, N213F, N213G, N213M, A236C, A236F, A236G, A236L, A236N, A236Q, A236V, S240F, S240I, S240M, S240P, S240Q, S240V, S240W, T242A, T242E, T242G, T242H, T242I, T242K, T242L, T242P, T242Q, T242S, T242V, T242Y, P260C, P260I, P260L, P260M, P260N, P260S, P260V, G258D, Y263T, Y263W, N141G, N141T, S173A, S173D, S173N, S173T, Y214F, Y214G, Y214I, Y214M, Y214N, P009A, P009G, P009H, P009M, P009Q, P009R, P009T, P009V, H010A, H010L, L096H, L096I, S116F, S116M, G025A, V044A, V044I, V081G, V081L, V081M, V081P, V081Y, N269K, N269T, N269V, H039G, H039S, H039T, K235A, K235D, K235E, V267A, V267E, V267F, V267G, V267I, V267K, V267L, V267N, V267S, V267T, V267Y, A151H, A151I, A151Q, A151S, A151T, G160F, G160H, G160M, G160V, M222E, M222H, M222L, and M222Y

### List of most beneficial mutations laundry stability + HDL pH6

I030A, A040E, A040L, A040T, A040Y, T103D, T103E, S052D, S052F, S052N, N183E, L031T, N117E, N117F, M124S, N123D, S129D, S130E, Q137E, G102E, I107Q, N120E, V121G, S182D, S182E, T003E, A224S, K015V, K027E, K027L, K027M, K027S, K027T, N043A, N043D, N043E, N043F, N043W, Q136D, N163D, T022D, T022E, R145N, R145T, R145W, S158D, L090P, G100A, G100E, I147A, I147H, I147S, V148S, Q245T, Q245V, I246N, S024T, S024V, K045D, K045E, K045F, K045L, K045Q, K045R, K045S, K045W, Q275E, Q275N, A048D, A048E, A048Q, T078D, T078E, T078F, T078L, T078W, N087S, Y091H, M241S, M241W, K265D, K265S, S272M, N038A, N038D, N038E, N038T, K251A, K251C, K251F, K251M, K251N, K251R, K251S, K251T, K251V, F050Q, T079L, G166D, G166E, G166H, G166N, G166Y, G053D, G053E, Y143D, Y143E, S181H, K237A, K237N, A215D, A215E, K012G, V072T, S156D, S156H, S156N, S156Q, T255D, N256D, N256E, F262E, G106E, T133D, N161D, N161E, R170L, A108C, S114G, S114T, G118D, G118E, G118Q, G118S, G118T, G146N, A138E, A138S, N140D, V149A, V149E, V149G, V149L, V149N, I150A, I150N, I150T, I150V, S216I, S216T, A228S, P239C, P239D, P239E, P239F, P239H, P239M, P239N, P239T, P239W, S159D, S159E, S159N, T209F, T209V, A236D, A236E, A236N, A236Q, A236S, T242E, S173D, P009A, P009I, P009M, P009Q, P009S, P009T, P009V, L096E, K235D, K235E, V267S, and M222Q

### List of most beneficial mutations laundry stability + HDL pH8

A029T, A040D, A040E, T103D, T103E, S052D, S052F, N183E, M124S, L126Q, N123D, S129D, S130E, Q137E, G102E, I107Q, S182D, S182E, T003E, K015V, T185Q, K027E, K027L, K027M, K027T, N043D, N043E, Q136D, N163D, R145N, R145T, R145W, S158D, G100A, G100E, I147H, I147S, Q245M, Q245T, Q245V, K045E, K045L, K045W, A048D, A048E, A048G, A048H, T078D, T078E, T078F, T078G, T078H, T078W, N087S, N252E, K265D, S272M, S272Q, N038D, N038E, N038T, K251A, K251C, K251F, K251M, K251N, K251R, K251S, K251T, K251V, F050Y, G166A, G166C, G166D, G166E, G166H, G166L, G166M, G166N, G166Q, G166S, G053D, G053E, K237A, K237N, A215D, A215E, K012G, V072A, V072T, S156D, S156N, N256D, N256E, F262E, G106E, T133D, N161D, N161E, R170L, A108C, A108I, S114T, G118D, G118E, G118Q, G118S, G118T, G146N, A138E, N140D, V149G, V149L, I150A, P239C, P239G, P239H, P239N, S159D, S159E, S159N, T209V, A236C, A236D, A236E, S240P, T242E, S173D, P009A, P009I, P009T, L096E, L096H, L096I, S116E, K235D, and K235E

### List of most beneficial mutations laundry stability + HDD

A029T, I030V, A040D, A040E, A040H, A040K, A040L, A040N, A040Y, T103D, T103E, T103G, S052D, S052F, S052N, N183E, V093T, L031T, N117E, N117F, N117S, N117T, M124S, L126G, L126I, L126Q, N123D, S129A, S129D, S129Q, S130E, S130L, S130N, S130Q, S130T, G131D, Q137E, G102E, I107Q, V121G, S182D, S182E, T003E, T003I, T003V, A224S, K015V, T185Q, K027E, K027L, K027M, K027S, K027T, N043A, N043D, N043E, N043F, N043L, N043W, Q136D, Q136H, N163D, A019M, T022E, R145N, R145T, S158D, L090P, G100A, G100E, I147A, I147H, I147S, V148S, Q245M, Q245T, S024H, S024T, S024V, K045D, K045L, K045Q, K045R, K045S, K045W, Q275N, A048D, A048E, A048G, A048H, A048Q, T078A, T078F, T078H, T078L, T078W, N087S, Y091H, Y091N, M241Q, M241S, K265S, S272M, S272Q, N038A, N038D, N038E, N038T, K251A, K251C, K251F, K251M, K251N, K251R, K251S, K251T, K251V, F050Q, F050Y, V051I, N076D, N076S, T079L, T079Q, L075G, G166A, G166C, G166D, G166E, G166F, G166H, G166M, G166N, G166Q, G166S, G166Y, G053D, G053E, G053Q, Y143E, S181A, K237N, A215D, A215E, K012G, V072A, V072T, S156A, S156D, S156H, S156M, S156N, S156Q, T255D, N256D, N256E, N256P, N256W, F262E, G106E, T133D, T133F, T133M, T133N, T133V, N161D, N161E, R170K, R170L, A108C, A108I, S114G, S114T, G118E, G118Q, G118S, G118T, A138E, A138S, N140D, V149A, V149E, V149G, V149L, V149N, V149Q, V149S, I150A, I150N, I150T, I150V, A228S, P239F, P239T, P239W, F217R, S159D, S159E, S159N, S159Q, G128N, T209V, T209W, A236C, A236N, A236Q, S240P, S240Q, T242E, S173D, P009A, P009M, P009Q, P009T, P009V, L096H, L096I, V081M, K235D, K235E, V267F, V267I, V267S, V267T, V267Y, and M222H

### List of most beneficial mutations for laundry Stability + HDL pH8 + HDD

A029T, A040D, A040E, T103D, T103E, S052D, S052F, N183E, M124S, L126Q, N123D, S129D, S130E, Q137E, G102E, I107Q, S182D, S182E, T003E, K015V, T185Q, K027E, K027L, K027M, K027T, N043D, N043E, Q136D, N163D, R145N, R145T, S158D, G100A, G100E, I147H, I147S, Q245M, Q245T, K045L, K045W, A048D, A048E, A048G, A048H, T078F, T078H, T078W, N087S, S272M, S272Q, N038D, N038E, N038T, K251A, K251C, K251F, K251M, K251N, K251R, K251S, K251T, K251V, F050Y, G166A, G166C, G166D, G166E, G166H, G166M, G166N, G166Q, G166S, G053D, G053E, K237N, A215D, A215E, K012G, V072A, V072T, S156D, S156N, N256D, N256E, F262E, G106E, T133D, N161D, N161E, R170L, A108C, A108I, S114T, G118E, G118Q, G118S, G118T, A138E, N140D, V149G, V149L, I150A, S159D, S159E, S159N, T209V, A236C, S240P, T242E, S173D, P009A, P009T, L096H, L096I, K235D, and K235E

### List of most beneficial mutations for laundry stability + HDD + HDL pH6 + HDL pH8

A040E, T103D, T103E, S052D, S052F, N183E, M124S, N123D, S129D, S130E, Q137E, G102E, I107Q, S182D, S182E, T003E, K015V, K027E, K027L, K027M, K027T, N043D, N043E, Q136D, N163D, R145N, R145T, S158D, G100A, G100E, I147H, I147S, Q245T, K045L, K045W, A048D, A048E, T078F, T078W, N087S, S272M, N038D, N038E, N038T, K251A, K251C, K251F, K251M, K251N, K251R, K251S, K251T, K251V, G166D, G166E, G166H, G166N, G053D, G053E, K237N, A215D, A215E, K012G, V072T, S156D, S156N, N256D, N256E, F262E, G106E, T133D, N161D, N161E, R170L, A108C, S114T, G118E, G118Q, G118S, G118T, A138E, N140D, V149G, V149L, I150A, S159D, S159E, S159N, T209V, T242E, S173D, P009A, P009T, K235D, and K235E

### List of most beneficial mutations in ADW Stability

A001G, A001K, A001R, Q002F, Q002K, Q002P, Q002R, Q002S, Q002W, T003G, T003I, T003V, T003Y, G007N, P009A, P009G, P009H, P009I, P009M, P009Q, P009R, P009S, P009T, P009V, A018N, A019M, T022E, S024H, S024R, S024T, S024V, G025A, G025H, V026N, K027P, K027R, K027S, K027T, A029S, I030V, T033E, N038A, N038Q, N038T, A040D, A040E, A040H, A040K, A040L, A040N, A040R, A040T, A040V, N043A, N043D, N043E, N043F, N043H, N043K, N043L, N043M, N043S, K045D, K045Q, K045R, K045S, A048E, A048H, A048N, A048Q, A048W, F050A, F050Q, F050R, F050S, V051I, S052D, S052M, S052N, S052Q, G053D, G053E, G053N, G053Q, P055N, A057P, Q059I, H067A, H067G, H067P, V072A, V072G, V072T, A073G, L075F, L075G, L075R, N076Q, N076S, N077T, T078A, T078D, T078E, T078F, T078G, T078H, T078K, T078L, T078R, T078S, T078V, T079K, T079L, T079Q, G080M, V081M, Y086P, N087H, D089F, L090P, Y091I, Y091M, Y091V, L096E, L096N, S097N, A098N, A098Q, A098S, G100A, G100E, S101M, S101T, G102F, G102Y, T103D, T103E, T103G, T103S, G106E, G106N, G106T, I107Q, I107V, W113H, S114G, S114T, S116E, S116F, S116M, N117S, N117T, G118D, G118E, G118H, G118Q, G118R, G118S, G118T, G118Y, M119Q, V121A, V121G, L126G, L126I, L126K, G128N, S129D, S130E, S130H, S130N, S130Q, S130T, S130V, G131H, T133A, T133D, T133F, T133L, T133V, Q136H, Q137E, Q137T, A138S, N140D, N144H, N144Y, R145W, G146N, G146R, I147A, I147H, I147S, V149A, V149E, V149G, V149L, V149N, V149P, V149Q, V149S, I150N, I150V, A151H, A152P, A152V, S156A, S156H, S156K, S156M, S156N, S156Q, S158V, S159D, S159E, S159N, S159Q, N161M, N161R, M165V, G166A, G166D, G166E, G166F, G166H, G166I, G166L, G166M, G166N, G166Q, G166S, G166T, G166Y, S181A, S181H, S181N, S182A, S182E, S182M, S182R, T185M, T185Q, T185R, A187W, S188H, S194P, T209F, T209V, T209W, T209Y, N213K, S216F, S216I, S216T, F217R, M222L, A224S, A228S, I234V, A236C, A236D, A236E, A236N, A236Q, A236S, K237A, Y238F, P239A, P239E, P239F, P239G, P239H, P239K, P239N, P239R, P239S, P239W, S240F, S240M, S240P, M241W, T242S, N243G, V244F, Q245A, Q245N, Q245S, Q245T, I246N, L250I, L250M, K251R, N252E, T255I, N256E, N256K, N256P, N256Q, N256R, N256W, K265S, V267F, V267K, V267N, V267S, V267T, V267Y, E271W, S272F, S272K, S272N, S272Q, and Q275E

### List of most beneficial mutations in ADW performance

A001G, A001H, A001K, A001R, Q002F, Q002K, Q002P, Q002R, Q002S, Q002W, Q002Y, T003E, T003G, T003H, T003I, T003V, T003Y, G007N, P009A, P009G, P009H, P009I, P009M, P009Q, P009R, P009S, P009T, P009V, K012G, K012R, K015H, K015V, K015Y, A018F, A018N, A018Q, A018R, A018T, A019K, A019L, A019M, A019R, A019W, G020N, T022D, T022E, T022S, S024H, S024I, S024R, S024T, S024V, G025A, V026N, V026S, K027D, K027E, K027G, K027L, K027M, K027P, K027Q, K027R, K027S, K027T, V028E, V028T, A029S, A029T, I030A, I030E, I030V, L031Q, L031T, N038A, N038Q, N038T, A040D, A040E, A040H, A040K, A040L, A040N, A040R, A040T, A040V, A040W, A040Y, N043A, N043D, N043E, N043F, N043G, N043H, N043I, N043K, N043L, N043M, N043Q, N043S, N043W, N043Y, K045D, K045E, K045F, K045L, K045Q, K045R, K045S, K045W, G046N, A048D, A048E, A048G, A048H, A048K, A048N, A048Q, A048W, F050A, F050K, F050Q, F050R, F050S, F050Y, S052D, S052H, S052L, S052M, S052N, S052Q, S052R, S052T, S052V, G053A, G053D, G053E, G053N, G053Q, G053R, P055K, P055N, P055R, A057P, L058D, Q059I, G061E, G061M, G061Y, N062K, N062L, N062P, N062W, H067A, H067G, A069G, V072A, V072G, V072T, A073G, L075F, L075G, L075Q, L075R, L075W, N076D, N076Q, N076S, N076Y, N077T, T078A, T078D, T078E, T078F, T078G, T078H, T078I, T078K, T078L, T078R, T078S, T078V, T079K, T079L, T079Q, G080M, G080Y, V081M, G083S, A085S, Y086P, N087H, N087S, D089F, L090E, L090N, L090P, Y091H, Y091I, Y091M, Y091N, Y091T, Y091V, Y091W, V093T, L096I, L096N, S097G, S097H, S097I, S097L, S097M, S097N, A098F, A098H, A098K, A098M, A098N, A098Q, A098S, A098T, S099I, S099K, S099L, S099V, G100A, G100E, S101A, S101M, S101R, S101T, S101V, G102E, T103D, T103E, T103H, T103K, T103N, T103R, T103S, G106E, G106N, G106S, G106T, G106V, I107Q, I107T, I107V, A108C, A108I, Q109K, I111V, W113F, W113H, S114G, S114T, S116E, S116F, S116M, N117G, N117K, N117S, N117T, N117Y, G118D, G118E, G118H, G118Q, G118R, G118S, G118T, G118Y, M119A, M119F, M119H, M119I, M119L, M119Q, M119S, M119T, N120E, V121A, N123A, N123D, N123E, N123G, N123H, N123Q, G128N, S129A, S129D, S129G, S129H, S129Q, S129T, S130E, S130F, S130H, S130L, S130N, S130Q, S130T, S130V, G131A, G131D, G131H, G131P, G131R, G131S, S132A, T133A, T133D, T133F, T133L, T133M, T133N, T133Q, T133V, Q136D, Q136F, Q136H, Q136R, Q136W, Q137E, Q137H, Q137T, A138E, A138S, N140D, N140Q, N140Y, N141T, Y143E, N144H, N144M, N144V, N144W, N144Y, R145W, G146N, G146R, I147A, I147H, I147M, I147S, V148G, V148S, V149A, V149E, V149G, V149L, V149N, V149P, V149Q, V149S, I150A, I150N, I150S, I150T, I150V, A151H, A152V, A153G, A153S, S156A, S156H, S156K, S156M, S156N, S158D, S158M, S158T, S158V, S159D, S159E, S159K, S159M, S159N, S159Q, S159Y, G160F, N161D, N161E, N161K, N161M, N161R, N161S, N163D, M165V, G166A, G166D, G166E, G166F, G166H, G166I, G166L, G166M, G166N, G166Q, G166S, G166T, G166Y, R170K, R170L, S172H, I175Q, I175S, S181A, S181H, S181K, S181N, S181Q, S182A, S182D, S182E, S182H, S182I, S182M, S182R, S182Y, N183E, T185M, T185Q, T185R, A187D, A187G, A187H, A187P, A187Q, A187V, A187W, S188H, S188Y, S194F, S194K, S194P, S194R, L206Y, T209F, T209V, T209W, T209Y, N213K, A215D, A215E, S216A, S216F, S216I, S216T, F217R, M222L, A224S, A228S, I234V, K235D, K235E, A236C, A236D, A236E, A236F, A236G, A236N, A236Q, A236S, K237A, K237G, K237N, K237Q, K237R, Y238F, P239A, P239C, P239D, P239E, P239F, P239G, P239H, P239I, P239K, P239L, P239M, P239N, P239R, P239S, P239T, P239V, P239W, S240F, S240I, S240M, S240P, S240Q, S240V, S240W, M241I, M241Q, M241S, M241W, T242A, T242E, T242I, T242K, T242P, T242Q, T242S, T242V, N243G, V244F, Q245A, Q245F, Q245M, Q245N, Q245S, Q245T, Q245W, Q245Y, I246A, I246N, I246V, E248D, E248H, E248K, E248M, E248Y, L250I, L250M, K251F, K251R, N252E, N252F, T255D, T255I, N256D, N256E, N256K, N256M, N256P, N256Q, N256R, N256W, F262E, K265D, K265S, V267A, V267F, V267K, V267N, V267S, V267T, V267Y, E271A, E271F, E271H, E271K, E271W, E271Y, S272F, S272K, S272N, S272Q, Q275E, Q275G, Q275N, and Q275R

### List of most beneficial mutations in ADW stability + ADW performance

A001G, A001K, A001R, Q002F, Q002K, Q002P, Q002R, Q002S, Q002W, T003G, T003I, T003V, T003Y, G007N, P009A, P009G, P009H, P009I, P009M, P009Q, P009R, P009S, P009T, P009V, A018N, A019M, T022E, S024H, S024R, S024T, S024V, G025A, V026N, K027P, K027R, K027S, K027T, A029S, I030V, N038A, N038Q, N038T, A040D, A040E, A040H, A040K, A040L, A040N, A040R, A040T, A040V, N043A, N043D, N043E, N043F, N043H, N043K, N043L, N043M, N043S, K045D, K045Q, K045R, K045S, A048E, A048H, A048N, A048Q, A048W, F050A, F050Q, F050R, F050S, S052D, S052M, S052N, S052Q, G053D, G053E, G053N, G053Q, P055N, A057P, Q059I, H067A, H067G, V072A, V072G, V072T, A073G, L075F, L075G, L075R, N076Q, N076S, N077T, T078A, T078D, T078E, T078F, T078G, T078H, T078K, T078L, T078R, T078S, T078V, T079K, T079L, T079Q, G080M, V081M, Y086P, N087H, D089F, L090P, Y091I, Y091M, Y091V, L096N, S097N, A098N, A098Q, A098S, G100A, G100E, S101M, S101T, T103D, T103E, T103S, G106E, G106N, G106T, I107Q, I107V, W113H, S114G, S114T, S116E, S116F, S116M, N117S, N117T, G118D, G118E, G118H, G118Q, G118R, G118S, G118T, G118Y, M119Q, V121A, G128N, S129D, S130E, S130H, S130N, S130Q, S130T, S130V, G131H, T133A, T133D, T133F, T133L, T133V, Q136H, Q137E, Q137T, A138S, N140D, N144H, N144Y, R145W, G146N, G146R, I147A, I147H, I147S, V149A, V149E, V149G, V149L, V149N, V149P, V149Q, V149S, I150N, I150V, A151H, A152V, S156A, S156H, S156K, S156M, S156N, S158V, S159D, S159E, S159N, S159Q, N161M, N161R, M165V, G166A, G166D, G166E, G166F, G166H, G166I, G166L, G166M, G166N, G166Q, G166S, G166T, G166Y, S181A, S181H, S181N, S182A, S182E, S182M, S182R, T185M, T185Q, T185R, A187W, S188H, S194P, T209F, T209V, T209W, T209Y, N213K, S216F, S216I, S216T, F217R, M222L, A224S, A228S, I234V, A236C, A236D, A236E, A236N, A236Q, A236S, K237A, Y238F, P239A, P239E, P239F, P239G, P239H, P239K, P239N, P239R, P239S, P239W, S240F, S240M, S240P, M241W, T242S, N243G, V244F, Q245A, Q245N, Q245S, Q245T, I246N, L250I, L250M, K251R, N252E, T255I, N256E, N256K, N256P, N256Q, N256R, N256W, K265S, V267F, V267K, V267N, V267S, V267T, V267Y, E271W, S272F, S272K, S272N, S272Q, and Q275E

| **Table 7: Summary of number of mutations in each category** | |
|---|---|
| **Category** | **Number of mutations** |
| Laundry stability | 329 |
| HDL pH 6 performance | 183 |
| HDL pH 8 performance | 609 |
| HDD performance | 1258 |
| Laundry stability + HDL pH6 | 184 |
| Laundry stability + HDL pH8 | 137 |
| Laundry stability +HDD | 226 |
| Laundry stability + HDD + HLD pH 8 | 116 |
| Laundry stability + HDD + HLD pH 8 + HDL pH6 | 95 |
| ADW stability | 314 |
| ADW performance | 564 |
| ADW stability + ADW performance | 299 |

### EXAMPLE 4

### Comparison of LG12 sprC protease to Related Molecules Identification of Homologous Proteases

Homologs were identified by a BLAST search (Altschul et al., Nucleic Acids Res, 25:3389-402, 1997) against the NCBI non-redundant protein database and the Genome Quest Patent database with search parameters set to default values using the mature protein amino acid sequences for LG12 sprC protease (SEQ ID NO:3) as the query sequence. Percent identity (PID) for both search sets is defined as the number of identical residues divided by the number of aligned residues in the pairwise alignment. Value labeled "sequence length" on tables corresponds to the length (in amino acids) for the proteins referenced with the listed Accession numbers, while "aligned length" refers to sequence used for alignment and PID calculation . Table 5 provides a list of sequences with the percent identity to LG12 sprC from the NCBI non-redundant protein database, and Table 6 provides a list of sequences from the Genome Quest patent database.

| **Table 5: List of sequences with percent identity to LG12 sprC protein identified from the NCBI non-redundant protein database** | | | | |
|---|---|---|---|---|
| **Accession #** | **PID** | **Organism** | **Sequence Length** | **Alignment Length** |
| AAC43580 | 100.0 | *Bacillus sp.* | 378 | 275 |
| WP_010192403 | 92.0 | *Bacillus sp. m3-13* | 381 | 275 |
| BAD11988 | 78.5 | *Bacillus sp. KSM-LD1* | 376 | 275 |
| BAD21128 | 77.9 | *Bacillus sp. KSM-LD1* | 377 | 276 |
| AAC43581 | 77.2 | *Bacillus sp.* | 379 | 276 |
| WP_010192405 | 76.8 | *Bacillus sp. m3-13* | 379 | 276 |
| WP_021837258 | 71.3 | *Bacilluslicheniformis CG-B52* | 379 | 275 |
| WP_006636716 | 71.3 | *Bacillus sonorensis L12* | 378 | 275 |
| BAE92942 | 71.3 | *Bacillus licheniformis* | 378 | 275 |
| AAG31027 | 71.1 | *Bacillus licheniformis* | 374 | 270 |
| AAG31028 | 71.1 | *Bacillus licheniformis* | 374 | 270 |
| WP_023856571 | 70.9 | *Bacillus sp. CPSM8* | 378 | 275 |
| AAG00494 | 70.9 | *Bacillus licheniformis* | 310 | 275 |
| AAG00493 | 70.9 | *Bacillus licheniformis* | 310 | 275 |
| WP_003180337 | 70.9 | *Bacilluslicheniformis WX-02* | 379 | 275 |
| ABY65723 | 70.9 | *Bacillus subtilis* | 354 | 275 |
| CAJ70731 | 70.9 | *Bacillus licheniformis* | 379 | 275 |
| AAT75303 | 70.9 | *Bacillus mojavensis* | 379 | 275 |
| AAY82467 | 70.9 | *Bacillus licheniformis* | 379 | 275 |
| YP_078307 | 70.9 | *Bacillus licheniformis* | 379 | 275 |
| ADK11044 | 70.9 | *Bacillus licheniformis* | 379 | 275 |
| P00780 | 70.9 | *Bacillus licheniformis* | 379 | 275 |
| AAG31026 | 70.7 | *Bacillus licheniformis* | 374 | 270 |
| AEQ38580 | 70.7 | *Bacillus licheniformis* | 370 | 276 |
| ABU68339 | 70.5 | *Bacillus licheniformis* | 379 | 275 |
| YP_008077337 | 70.5 | *Bacillus licheniformis 9945A* | 379 | 275 |
| AAG10033 | 70.5 | *Bacillus licheniformis* | 310 | 275 |
| AAB34259 | 70.5 | *Bacillus licheniformis* | 379 | 275 |
| AEU12640 | 69.8 | *Bacillus licheniformis* | 379 | 275 |
| AEU17777 | 69.8 | *Bacillus licheniformis* | 349 | 275 |
| CAA62668 | 69.3 | *Bacillus licheniformis* | 379 | 274 |
| AER41028 | 68.8 | *Bacillus subtilis* | 234 | 234 |
| YP_003972439 | 68.4 | *Bacillus atrophaeus UCMB-5137* | 382 | 275 |
| CAA62667 | 68.2 | *Bacillus licheniformis* | 379 | 274 |
| ABD33463 | 68.2 | *Bacillus sp. hr08* | 355 | 255 |
| CAA62666 | 67.5 | *Bacillus licheniformis* | 379 | 274 |
| WP_003239459 | 67.3 | *Bacillus subtilis subsp. inaquosorum KCTC 13429* | 381 | 275 |
| ACJ07037 | 67.3 | *Bacillus subtilis* | 381 | 275 |
| ADI24411 | 67.1 | *Bacillus subtilis* | 347 | 240 |
| ACM47735 | 67.0 | *Bacillus pumilus* | 383 | 273 |
| ADV15999 | 66.9 | *Bacillus licheniformis* | 273 | 251 |
| BAN09118 | 66.9 | *Bacillus subtilis* | 381 | 275 |
| YP_007661807 | 66.9 | *Bacillus subtilis subsp. subtilis str. BAB-1* | 381 | 275 |
| YP_007426221 | 66.9 | *Bacillus subtilis XF-1* | 381 | 275 |
| AAX53176 | 66.9 | *Bacillus subtilis* | 381 | 275 |
| ADK11996 | 66.7 | *Bacillus pumilus* | 383 | 273 |
| AAU88064 | 66.7 | *Bacillus pumilus* | 383 | 273 |
| YP_005556107 | 66.5 | *Bacillus subtilis subsp. subtilis str. RO-NN-1* | 381 | 275 |
| WP_010333625 | 66.5 | *Bacillus mojavensis* | 381 | 275 |
| YP_006230926 | 66.5 | *Bacillus subtilis* | 381 | 275 |
| AGL34969 | 66.5 | *Bacillus subtilis* | 361 | 254 |
| ACJ06132 | 66.3 | *Bacillus subtilis subsp. natto* | 362 | 273 |
| WP_008359041 | 66.3 | *Bacillus sp. HYC-10* | 381 | 273 |
| AER52006 | 66.2 | *Bacillus subtilis* | 324 | 275 |
| ACM 17223 | 66.2 | *Recombining vector pCRBAF1* | 358 | 275 |
| ACL37472 | 66.2 | *Bacillus subtilis* | 381 | 275 |
| YP_007210274 | 66.2 | *Bacillus subtilis subsp. subtilis str. BSP1* | 381 | 275 |
| AAO65246 | 66.2 | *Bacillus subtilis* | 381 | 275 |
| AFC89901 | 66.2 | *Bacillus subtilis* | 381 | 275 |
| YP_004876616 | 66.2 | *Bacillus subtilis subsp. spizizenii TU-B-10* | 381 | 275 |
| ABU93240 | 66.2 | *Bacillus sp. CN* | 352 | 275 |
| YP_004207041 | 66.2 | *Bacillus sp. EGD-AK10* | 381 | 275 |
| ABJ98766 | 66.2 | *Bacillus subtilis* | 374 | 275 |
| ABJ98765 | 66.2 | *Bacillus subtilis* | 381 | 275 |
| CAC41625 | 66.2 | *Bacillus subtilis* | 275 | 275 |
| WP_018922084 | 66.1 | *Salsuginibacillus kocurii* | 373 | 274 |
| AAW83000 | 65.9 | *Bacillus subtilis subsp. natto* | 362 | 273 |
| ACJ48969 | 65.9 | *Bacillus subtilis* | 362 | 273 |
| AAX35771 | 65.8 | *Bacillus subtilis* | 381 | 275 |
| P00783 | 65.8 | *Bacillus subtilis subsp. amylosacchariticus* | 381 | 275 |
| AEV91244 | 65.8 | *Bacillus subtilis* | 381 | 275 |
| NP_388911 | 65.8 | *Bacillus subtilis PY79* | 381 | 275 |
| ABM97611 | 65.8 | *Bacillus subtilis* | 275 | 275 |
| P29142 | 65.8 | *Geobacillus stearothermophilus* | 381 | 275 |
| WP_017695350 | 65.8 | *Bacillus subtilis* | 381 | 275 |
| ABB92698 | 65.8 | *Bacillus subtilis* | 363 | 275 |
| WP_017417394 | 65.8 | *Bacillus amyloliquefaciens* | 382 | 275 |
| ABI93801 | 65.8 | *Bacillus sp. RH219* | 382 | 275 |
| YP_005420292 | 65.8 | *Bacillus subtilis* | 382 | 275 |
| WP_007610454 | 65.8 | *Bacillus sp. 5B6* | 382 | 275 |
| WP_021494939 | 65.8 | *Bacillus amyloliquefaciens EGD-AQ14* | 382 | 275 |
| ADK11043 | 65.8 | *Bacillus subtilis* | 381 | 275 |
| ABY83469 | 65.8 | *Bacillus subtilis* | 382 | 275 |
| WP_010329279 | 65.8 | *Bacillus vallismortis* | 381 | 275 |
| ACI32816 | 65.7 | *Bacillus subtilis* | 274 | 274 |
| WP_009966941 | 65.5 | *Bacillus subtilis* | 381 | 275 |
| AAB47045 | 65.5 | *Bacillus subtilis, RT-5, Pakistani Tharparkar desert soil isolate, Peptide, 275 aa* | 275 | 275 |
| CAA74536 | 65.5 | *Bacillus subtilis subsp. subtilis str. 168* | 381 | 275 |
| AAX35772 | 65.5 | *Bacillus subtilis* | 381 | 275 |
| YP_008627389 | 65.5 | *Bacillus amyloliquefaciens subsp. plantarum NAU-B3* | 382 | 275 |
| ABA29609 | 65.5 | *Bacillus subtilis* | 275 | 275 |
| YP_005129739 | 65.5 | *Bacillus amyloliquefaciens UASWS BA1* | 382 | 275 |
| ACT33949 | 65.5 | *Bacillus amyloliquefaciens* | 381 | 275 |
| ACA34903 | 65.5 | *Bacillus amyloliquefaciens* | 382 | 275 |
| ABU93241 | 65.5 | *Bacillus sp. SJ* | 352 | 275 |
| AAT45900 | 65.5 | *Bacillus amyloliquefaciens* | 382 | 275 |
| YP_003865404 | 65.5 | *Bacillus subtilis* | 381 | 275 |
| ACU32756 | 65.5 | *Bacillus licheniformis* | 382 | 275 |
| AFH88394 | 65.5 | *Bacillus methylotrophicus* | 382 | 275 |
| ABW91176 | 65.5 | *Micrococcus luteus* | 275 | 275 |
| WP_019713856 | 65.5 | *Bacillus subtilis* | 381 | 275 |
| YP_001420645 | 65.5 | *Bacillus amyloliquefaciens CC178* | 382 | 275 |
| BAA35011 | 65.5 | *Bacillus sp. Ace02* | 275 | 275 |
| ABY25856 | 65.5 | *Geobacillus stearothermophilus* | 382 | 275 |
| WP_017729072 | 65.5 | *Bacillus sp. L1(2012)* | 362 | 275 |
| WP_007497196 | 65.2 | *Bacillus stratosphericus LAMA 585* | 383 | 273 |
| ACM07731 | 65.2 | *Bacillus pumilus* | 383 | 273 |
| AAC63365 | 65.2 | *Bacillus subtilis* | 382 | 273 |
| ACE63521 | 65.1 | *Bacillus sp. ZLW-2* | 381 | 275 |
| YP_003919715 | 65.1 | *Bacillus amyloliquefaciens XH7* | 382 | 275 |
| CAA24990 | 65.1 | *Bacillus amyloliquefaciens* | 376 | 275 |
| ABO77900 | 65.1 | *Bacillus subtilis* | 362 | 275 |
| ABY65903 | 65.1 | *Bacillus subtilis* | 381 | 275 |
| AAV30845 | 65.1 | *Bacillus sp. B16* | 382 | 275 |
| BAD02409 | 65.0 | *Bacillus sp. KSM-LD1* | 404 | 277 |
| CAO03040 | 64.8 | *Bacillus pumilus* | 383 | 273 |
| BAA93474 | 64.8 | *Bacillus pumilus* | 383 | 273 |
| WP_017360299 | 64.8 | *Bacillus pumilus* | 383 | 273 |
| WP_019743331 | 64.8 | *Bacillus pumilus* | 383 | 273 |
| AFP23380 | 64.8 | *Bacillus lehensis* | 276 | 273 |
| WP_008348814 | 64.8 | *Bacillus sp. M 2-6* | 381 | 273 |
| ADK63096 | 64.8 | *Bacillus pumilus* | 406 | 273 |
| ACO94164 | 64.8 | *Bacillus pumilus* | 382 | 273 |
| WP_003210785 | 64.8 | *Bacillus pumilus ATCC 7061* | 356 | 273 |
| AAR19220 | 64.8 | *Bacillus pumilus* | 383 | 273 |
| AAY23643 | 64.7 | *Bacillus subtilis* | 275 | 275 |
| CAE18180 | 64.7 | *Bacillus subtilis* | 381 | 275 |
| WP_016938318 | 64.7 | *Bacillus siamensis* | 382 | 275 |
| YP_001486216 | 64.5 | *Bacillus pumilus SAFR-032* | 381 | 273 |
| ADN04910 | 64.5 | *Bacillus circulans* | 275 | 273 |
| ADD29797 | 64.5 | *Bacillus pumilus* | 406 | 273 |
| AAX14553 | 64.1 | *Bacillus pumilus* | 381 | 273 |
| P27693 | 63.7 | *Bacillus alcalophilus* | 380 | 273 |
| P41362 | 63.4 | *Bacillus alcalophilus* | 380 | 273 |
| AFR78140 | 63.0 | *synthetic construct* | 269 | 273 |
| BAA25184 | 62.9 | *Bacillus sp.* | 379 | 272 |
| AFK08970 | 62.5 | *Bacillus lehensis* | 378 | 272 |
| P20724 | 62.5 | *Bacillus subtilis* | 378 | 272 |
| YP_003426762 | 62.2 | *Bacillus pseudofirmus OF4* | 374 | 275 |
| BAA06158 | 62.2 | *Bacillus sp.* | 374 | 275 |
| WP_022628745 | 61.8 | *Bacillus marmarensis DSM 21297* | 374 | 275 |
| AGN91700 | 61.5 | *Bacillus circulans* | 383 | 273 |
| AGS78407 | 60.7 | *Bacillus gibsonii* | 375 | 272 |
| BAA05540 | 59.3 | *Bacillus sp.* | 361 | 275 |

| **Table 6: List of sequences with percent identity to LG12 sprC protein identified from the Genome Quest Patent database** | | | | |
|---|---|---|---|---|
| **Patent ID #** | **PID to LG12 sprC** | **Organism** | **Sequence Length** | **Alignment Length** |
| US5677163-0001 | 100 | *Bacillus sp.* | 275 | 275 |
| JP2004313043-0002 | 83.3 | *Bacillus sp. KSM-9865* | 275 | 275 |
| JP2004313043-0001 | 83.3 | *Bacillus sp. KSM-KP43* | 275 | 275 |
| JP2004313043-0004 | 83.3 | *Bacillus sp. KSM-9865* | 379 | 275 |
| JP2004313043-0003 | 83.3 | *Bacillus sp. KSM-KP43* | 379 | 275 |
| JP2008022828-0012 | 83.3 | *Bacillus sp. KSM-KP43* | 352 | 275 |
| US5275945-0002 | 81.8 | *Bacillus sp.* | 377 | 275 |
| JP2004065171-0001 | 78.6 | *Bacillus sp. KSM-LD1* | 376 | 275 |
| JP2004154003-0002 | 77.9 | *Bacillus sp. KSM-LD1* | 377 | 276 |
| JP2004154003-0001 | 77.9 | *Bacillus sp. KSM-LD1* | 276 | 276 |
| US5677163-0007 | 77.2 | *empty* | 276 | 276 |
| WO9830682 | 71.6 | *Bacillus sp* | 274 | 275 |
| US7510859-0014 | 71.4 | *Artificial Sequence* | 274 | 276 |
| DE10260903 | 71.4 | *Synthetic* | 379 | 276 |
| WO9628557 | 71.3 | *Synthetic* | 274 | 275 |
| WO9628557 | 71.3 | *Synthetic* | 274 | 275 |
| WO9628557 | 71.3 | *Synthetic* | 274 | 275 |
| WO8807581-0019 | 71.3 | *empty* | 274 | 275 |
| US8211428-0304 | 71.3 | *Bacillus licheniformis* | 274 | 275 |
| WO9628557 | 71 | *Synthetic* | 274 | 275 |
| JP2013500714-0098 | 71.3 | *Bacillus licheniformis* | 274 | 275 |
| WO2005124012-0018 | 71 | *Synthetic* | 274 | 276 |
| US7510859-0010 | 71 | *Synthetic* | 274 | 276 |
| DE10260903 | 71 | *Synthetic* | 379 | 276 |
| DE10260903 | 71 | *Synthetic* | 379 | 276 |
| US5719021-0004 | 70.9 | *Bacillus licheniformis* | 350 | 275 |
| US7510859-0016 | 70.9 | *Artificial Sequence* | 274 | 275 |
| JP1991072876-0002 | 70.9 | *Bacillus licheniformis* | 274 | 275 |
| US20120308543-0003 | 70.9 | *Bacillus licheniformis* | 274 | 275 |
| US20110171718-0117 | 70.9 | *Bacillus licheniformis* | 274 | 275 |
| US20120190087-0020 | 70.9 | *Bacillus licheniformis* | 274 | 275 |
| JP2013500714-0109 | 70.9 | *Bacillus licheniformis* | 274 | 275 |
| WO9628557 | 70.9 | *Synthetic* | 274 | 275 |
| WO9830682 | 70.9 | *Bacillus sp* | 274 | 275 |
| US6416756-0003 | 70.9 | *Bacillus sp.* | 274 | 275 |
| WO2013076269-0006 | 70.9 | *Bacillus licheniformis* | 377 | 275 |
| CN1412315-0001 | 70.9 | *Empty* | 379 | 275 |
| DE10260903 | 70.9 | *Bacillus licheniformis* | 379 | 275 |
| US20110110910-0002 | 70.9 | *Synthetic* | 379 | 275 |
| US20120301945-0289 | 70.9 | *Bacillus licheniformis* | 379 | 275 |
| US5275945-0004 | 70.9 | *Bacillus* | 378 | 275 |
| US8168417-5227 | 70.9 | *Bacillus licheniformis* | 379 | 275 |
| EP0701605-0026 | 70.9 | *Bacillus licheniformis* | 378 | 275 |
| US7081359-0012 | 70.9 | *Artificial Sequence* | 380 | 275 |
| US20030049619-0015 | 70.9 | *Bacillus licheniformis* | 310 | 275 |
| EP0416967 | 70.6 | *Bacillus licheniformis* | 274 | 275 |
| US20110171718-0112 | 70.6 | *Bacillus licheniformis* | 274 | 275 |

### EXAMPLE 5

### Functional comparison of LG12 sprC and Bacillus sp. m3-13, subtilisin E (1) proteases

Bacillus sp. m3-13 subtilisin E(1) (accession number WP_010192403.1, formerly ZP_07707657.1), was found to have 92% identity to LG12sprC (Example 4, Table 5). To compare it's properties to those of LG12 sprC protease, a synthetic gene (SEQ ID NO:5) encoding this protein was made and cloned into pHYT vector following the steps described in Example 2 for the cloning of LG12 sprC. Briefly, synthetic DNA was prepared corresponding to the pro-mature sequence of the protein, flanked by NheI-HindIII sites, and this resulted in fusion to the AprE signal sequence already in the pHYT vector.
SEQ ID NO: 5 sets forth the nucleotide sequence encoding the precursor to Bacillus sp. m3-13 subtilisin E(1) protein used for cloning gene into pHYT vector using NheI-HindIII sites. 
GCTAGCGCAGCAGCTTCTGGTCCGAGTGCTAATGCCAAGAAAGAGTACTTAGTGGG
ATTTGCTAAAGGAAACAAAGCGAATGCTCAGTCTTCCAAAAATTTAATTACAGCTG
CTGGTGGCGATATTCAACATACATTCCAGTACATGGATGTAGTGGAAGTGAGCTTG
CCTGAAAAAGCGGCTGAAGCATTAGCGAAAAATCCTAACATTGCATTTGTAGAGGT
AAACGCAGAAGTTCAAGCTACTGCACAAACAGTTCCTTGGGGAATTCCTCACATTA
AAGCAGACAAAGCGCACGCTTCAGGTGTAACAGGAAGTGGAGTGAAAGTTGCGGT
CCTTGATACAGGAATTGATGCAAACCATGCGGATTTGAATGTTAAAGGTGGAGCAA
GCTTTGTTTCTGGTGAACCAAATGCCCTTCAAGATGGAAACGGGCACGGAACACAC
GTAGCAGGAACGGTTGCTGCATTGAATAATACGACAGGAGTGCTTGGTGTAGCTTA
TAATGCGGATCTTTATGCAGTTAAAGTTCTTAGTGCTTCCGGAAGCGGTACATTAAG
TGGAATCGCACAAGGAATTGAGTGGTCCATTGCTAATGATATGGACGTTATCAATA
TGAGCCTTGGTGGTAGTACCGGTTCCACAGCACTTCAGCAAGCTTGTGACAATGCAT
ATGCAAGTGGAATTGTAGTGGTTGCAGCTGCTGGAAACTCCGGTTCTAAAGGTAAG
AGAAACACAATGGGTTACCCTGCTAGATACAGTTCTGTAATTGCAGTTGGTGCGGTC
GACAGCAGTAACAACCGTGCATCTTTCTCAAGTGTTGGAAGTGAGCTTGAAGTAAT
GGCTCCAGGTGTAAGTATCCTTAGCACGACTCCTGGAAACAACTACTCTTCCTTCAA
TGGTACGTCTATGGCTTCTCCACATGTTGCAGGAGCAGCTGCATTAATCAAAGCAAA
ATATCCTAGCATGACGAATGTACAAATTCGTGAAAAACTAAAAAATACTGCCACTA
ACCTTGGCGATGCGTTCTATTATGGACATGGTGTAATTAACGTTGAAAGTGCTTTGC
AATAAAAGCTT
SEQ ID NO: 6 sets forth the amino acid sequence of the full length Bacillus sp. m3-13 subtilisin E(1) protein (354 amino acids) as expressed from gene sequence SEQ ID NO:5 in pHYT plasmid.

MRSKKLWISLLFALTLIFTMAFSNMSASAAASGPSANAKKEYLVGFAKGNKANAQSSKNLI
TAAGGDIQHTFQYMDVVEVSLPEKAAEALAKNPNIAFVEVNAEVQATAQTVPWGIPHIKAD
KAHASGVTGSGVKVAVLDTGIDANHADLNVKGGASFVSGEPNALQDGNGHGTHV
AGTVAALNNTTGVLGVAYNADLYAVKVLSASGSGTLSGIAQGIEWSIANDMDVIN
MSLGGSTGSTALQQACDNAYASGIVVVAAAGNSGSKGKRNTMGYPARYSSVIAVG
AVDSSNNRASFSSVGSELEVMAPGVSILSTTPGNNYSSFNGTSMASPHVAGAAALIK
AKYPSMTNVQIREKLKNTATNLGDAFYYGHGVINVESALQ

Subsequent transformation into *Bacillus subtilis* cells and protein isolation were also as described in Example 2. Clarified supernatant samples of the mature Bacillus sp. m3-13 subtilisin E(1) protein (SEQ ID NO:7) were tested alongside samples of LG12 sprC wildtype protein in a number of assays. The methods used are described in example 1.

SEQ ID NO:7 sets forth the amino acid sequence of the mature Bacillus sp. m3-13 subtilisin E(1) protein (275 amino acids)

AQTVPWGIPHIKADKAHASGVTGSGVKVAVLDTGIDANHADLNVKGGASFVSGEPNA
LQDGNGHGTHVAGTVAALNNTTGVLGVAYNADLYAVKVLSASGSGTLSGIAQGIEWSI
ANDMDVINMSLGGSTGSTALQQACDNAYASGIVVVAAAGNSGSKGKRNTMGYPARY
SSVIAVGAVDSSNNRASFSSVGSELEVMAPGVSILSTTPGNNYSSFNGTSMASPHVAGA
AALIKAKYPSMTNVQIREKLKNTATNLGDAFYYGHGVINVESALQ

A summary of test results from MTP assays performed to measure the laundry cleaning activity on BMI swatches using either OMO or Kirkland HDL detergents, the ADW cleaning on egg swatches using GSM pH9 or Sun all in One detergents, protein stability in either OMO HDL or LAS/EDTA and protease activity on AAPF-pNA are shown on Table 7. Bacillus sp. m3-13 subtilisin E(1) (accession number WP_010192403.1, formerly ZP_07707657.1) and LG12 sprC proteases display very similar properties across the systems tested.

**Table 7. Comparison of Bacillus sp. m3-13 subtilisin E(1) and LG12 sprC proteases across various assays**

| Sample | BMI OMO cleaning | BMI Kirkland cleaning | Egg GSMpH9 cleaning | Egg Sun cleaning | OMO HDL Stabil. | LAS/EDTA Stabil. | DMC Spec Act. | AAPF Spec Act. |
|---|---|---|---|---|---|---|---|---|
| Bacillus sp. m3-13 subtilisin E(1) | +++ | +++ | +++ | ++++ | +++++ | +++++ | +++ | +++ |
| LG12sprC | ++++ | ++++ | ++++ | ++++ | ++++ | ++++ | ++++ | ++++ |

### EXAMPLE 6

### Discovery and Identification of serine protease Bhon03321

*Bacillus horikoshii* strain DSM 8719 (obtained from Leibniz-Institut DSMZ - Deutsche Sammlung von Mikroorganismen und Zellkulturen GmbH) was selected as a potential source for enzymes useful in industrial applications. To identify enzymes produced by DSM 8719 and the genes that encode these enzymes, the entire genome of DSM 8719 was sequenced using Illumina® sequencing by synthesis (SBS) technology. Genome sequencing and assembly of the sequence data was performed by BaseClear (Leiden, The Netherlands). Contigs were annotated by BioXpr (Namur, Belgium). One of genes identified this way in strain DSM 8719 encodes a protein that showed homology to serine proteases of various other bacteria. The sequence of this gene, *Bhon03321.n,* is depicted in SEQ ID NO:8.

SEQ ID NO:8 sets forth the nucleotide sequence of the Bhon03321 gene.

TTGAAGAAGTGGATGAAGAGTTTTTCAGTTATACTTATTGCAATATTGGCTTTCTCG
TTAGCTTTTGGAACTGCTCAGGCAGAGTCTTCTAATCCGAATGCTAGTGTAAAGAAA
GAATACTTAATTGGGTTTGCAAAAGGAAACAAGGCAAGTGCACAGACCTCCCAAAA
TCTGGTTACAGCAGCAGGGGGAGAAGTTCAACACACATTCCAATACATGGATGTAG
TGGAAGTTTCGTTACCTGAAAAAGCTGCAGAAGCATTGAAGAAGAATCCTAACATA
GCATTCGTAGAGGAAAACGTAGAGGTTATGGCGACTGCCCAAACAGTACCTTGGGG
CATCCCGCATATCAAAGCAGACAAAGCACATGCTGCCGGTGTGACAGGAAGCGGA
GTGAAAGTGGCAATCCTTGATACAGGGATCGACGCCAATCATGCAGACTTAAATGT
AAGAGGCGGAGCAAGTTTTGTGGCAGGTGAGCCGAATGCCCTTCAAGATGGAAATG
GACACGGAACTCACGTGGCCGGTACGGTTGCCGCATTGAATAATTCAACTGGCGTT

CTAGGGGTCGCTTACAACGCAGACCTTTATGCGGTAAAAGTTCTTAGCGCTTCAGGT
AGTGGGACTTTAAGTGGCATCGCACAAGGTATTGAATGGTCCATTGCAAATGGAAT
GGATGTAATCAATATGAGTCTTGGTGGAAGCTCTGGGTCTACTGCACTCCAACAAG
CTTGTAATAATGCCTACAATAGAGGCATTGTTGTAATTGCTGCTGGGAATTCCG
GCTCTTCAGGAAATCGCAACACTATCGGGTACCCTGCAAGATATAGTTCAGTAATC
GCAGTGGGAGCAGTGGATTCCAATAACAACCGCGCTTCTTTCTCCAGTGTTGGAAAT
GAGCTGGAAGTAATGGCGCCGGGTGCTAATATCTTGAGCACGACTCCTGGTAATAA
CTATGCATCCTTCAACGGAACGTCCATGGCAGCTCCACATGTTGCAGGAGCAGCAG
CATTGATAAAAGCAAAATATCCTAGCATGACAAATGTCCAAATTCGTGATCGTTTA
AGAAATACGGCTACAAACCTTGGTGATCCTTTCTTTTATGGAAACGGAGTAATCAAT
GTAGAAAGTGCTTTACAA

The preproenzyme encoded by the *Bhon03321.n* gene is depicted in SEQ ID NO: 9. The methionine at position 1 is encoded by the ttg start codon of the *Bhon03321.n* gene. At the N-terminus, the protein has a signal peptide with a length of 27 amino acids as predicted by SignalP-NN (Emanuelsson et al., Nature Protocols (2007) 2: 953-971). This signal peptide sequence is underlined and in bold in SEQ ID NO:9. The presence of a signal peptide indicates that this serine protease is a secreted enzyme. Like other serine proteases, the enzyme has a pro sequence which is predicted to be 79 amino acids (*in italics* in SEQ ID NO: 9). This prediction is based on pro-mature junction in homologous serine proteases like BPN' (Wells et al., Nucleic Acids Res. (1983) 11: 7911-25). The sequence of the predicted, fully processed mature chain (Bhon03321, 275 amino acids) is depicted in SEQ ID NO: 10.SEQ ID NO:9 sets forth the amino acid sequence of the serine protease precursor Bhon03321.

MKKWMKSFSVILIAILAFSLAFGTAQAESSNPNASVKKEYLIGFAKGNKASAQTSQNLVT
AAGGEVQHTFQYMDVVEVSLPEKAAEALKKNPNIAFVEENVEVMATAQTVPWGIPHIKADK
AHAAGVTGSGVKVAILDTGIDANHADLNVRGGASFVAGEPNALQDGNGHGTHVAGTV
AALNNSTGVLGVAYNADLYAVKVLSASGSGTLSGIAQGIEWSIANGMDVINMSLGGSS
GSTALQQACNNAYNRGIVVIAAAGNSGSSGNRNTIGYPARYSSVIAVGAVDSNNNRASF
SSVGNELEVMAPGANILSTTPGNNYASFNGTSMAAPHVAGAAALIKAKYPSMTNVQIR
DRLRNTATNLGDPFFYGNGVINVESALQ

SEQ ID NO:10 sets forth the amino acid sequence of the mature Bhon03321 protease (275 amino acids)

AQTVPWGIPHIKADKAHAAGVTGSGVKVAILDTGIDANHADLNVRGGASFVAGEPNAL
QDGNGHGTHVAGTVAALNNSTGVLGVAYNADLYAVKVLSASGSGTLSGIAQGIEWSI
ANGMDVINMSLGGSSGSTALQQACNNAYNRGIVVIAAAGNSGSSGNRNTIGYPARYSS
VIAVGAVDSNNNRASFSSVGNELEVMAPGANILSTTPGNNYASFNGTSMAAPHVAGAA
ALIKAKYPSMTNVQIRDRLRNTATNLGDPFFYGNGVINVESALQ

### EXAMPLE 7

### Discovery and Identification of additional B. horikoshii serine proteases

Bacillus horikoshii DSM 9711, Bacillus horikoshii DSM 9712 and Bacillus horikoshii DSM 6951 were also selected as a potential source for LG12-clade subtilisins and other enzymes useful in industrial applications. The strains were obtained from Leibniz-Institut DSMZ - Deutsche Sammlung von Mikroorganismen und Zellkulturen GmbH. To identify enzymes produced by these strains and the genes that encode these enzymes, the genomes of all three *Bacillus horikoshii* strains were sequenced using Illumina® sequencing by synthesis (SBS) technology. Genome sequencing, assembly of the sequence data, and annotation of the contigs was performed by BaseClear (Leiden, The Netherlands). One of genes identified this way in strain *Bacillus horikoshii* DSM 9711 encodes a protein that belongs to the LG12-clade subtilisins. The sequence of this gene, *DSM9711_sprC,* is depicted in SEQ ID NO: 11.
SEQ ID NO: 11 sets forth the nucleotide sequence of the *DSM9711_sprC* gene:

TTGAAGAAGTGGATGAAAGGTTTTTCGGTCATACTTATTGCAATATTGGCTTTCTCG
TTAGCATTTGGTACTGCGCAGGCAGAATCGTCCAGTCCCAATGCTAGTGCAAA
GAAAGAATACTTAGTTGGCTTTACAAAAGGAAATAAGGCAAGTGCACAATCTTCTA
AAAACTTGATTGCCGCAGCAGGTGGGGAAGTACAGCACACGTTCCAATACATGG
ATGTTGTGGAAGTGTCTTTACCTGAAAAAGCGGTAGATGCATTGAAGAAAAATCCT
AACATCGCTTTTGTAGAAGAAAACGCAGAAGTTCAAGCAACTGCACAAACTGTG
CCATGGGGTATCCCTCATATCAAGGCTGACAAAGCGCATGCCTCTGGTGTGACAGG
AAGCGGTGTCAAAGTCGCGATCCTAGATACAGGAATTGACGCAAACCATACTGA
TCTTAATGTAAAAGGCGGAGCGAGCTTTGTTTCAGGTGAGCCAAATGCCCTTCAAG
ATGGTAACGGCCATGGAACACACGTAGCAGGAACAGTTGCTGCGTTAAATAATT
CTACTGGTGTACTTGGTGTAGCTTACAATGCTGACCTTTATGCGGTGAAAGTACTTA
GTGCTTCAGGTAGTGGAACATTAAGTGGAATCGCACAAGGATTGAGTGGTCC
ATTGCAAATGGAATGAACGTCATCAATATGAGTTTAGGAGGCAGCACTGGATCAAC
TGCATTGCAACAAGCTTGTGATAATGCTTATGCAAGAGGTATTGTCGTCATTGC

GGCAGCTGGAAACTCTGGTTCTAGAGGGAAGCAAAACACAATGGGTTATCCTGCAA
GATACAGCTCCGTCATCGCAGTAGGAGCGGTAGATTCCAGTAACAACCGTGCTT
CTTTCTCCAGTGTAGGAAACGAATTGGAAGTAATGGCACCGGGTGTTAACATTTTAA
GCACAACTCCTGGTAACAACTATGCATCCTTCAATGGAACGTCCATGGCGTCT
CCACACGTTGCTGGAGCTGCTGCATTAATCAAAGCGAAGTATCCAAGCATGACAAA
TGTTCAAATCCGTGACCGTCTGAAAAATACGGCTACAAACTTAGGTGATCCTTT
CTTCTACGGTAAAGGTGTCATCAATGTGGAAAGTGCTTTACAATAA

The preproenzyme encoded by the DSM9711_sprC gene is depicted in SEQ ID NO: 12. At the N-terminus, the protein has a signal peptide with a length of 27 amino acids as predicted by SignalP-NN (Emanuelsson et al., Nature Protocols (2007) 2: 953-971). This signal peptide sequence is underlined and in bold in SEQ ID NO: 12. The presence of a signal peptide indicates that this serine protease is a secreted enzyme. The enzyme has a pro sequence which is predicted to be 79 amino acids (*in italics* in SEQ ID NO: 12). The sequence of the predicted, fully processed mature chain (DSM9711_SprC, 275 amino acids) is depicted in SEQ ID NO: 13.
SEQ ID NO: 12 sets forth the amino acid sequence of the serine protease precursor of DSM9711_SprC: 
MKKWMKGFSVILIAILAFSLAFGTAQAESSSPNASAKKEYLVGFTKGNKASAQSSKNLIA
AAGGEVQHTFQYMDVVEVSLPEKAVDALKKNPNIAFVEENAEVQATAQTVPWGIPHIKADK
AHASGVTGSGVKVAILDTGIDANHTDLNVKGGASFVSGEPNALQDGNGHGTHVAGTV
AALNNSTGVLGVAYNADLYAVKVLSASGSGTLSGIAQGIEWSIANGMNVINMSLGGST
GSTALQQACDNAYARGIVVIAAAGNSGSRGKQNTMGYPARYSSVIAVGAVDSSNNRAS
FSSVGNELEVMAPGVNILSTTPGNNYASFNGTSMASPHVAGAAALIKAKYPSMTNVQIR
DRLKNTATNLGDPFFYGKGVINVESALQ
SEQ ID NO: 13 sets forth the amino acid sequence of the mature protease DSM9711_SprC (275 amino acids):

AQTVPWGIPHIKADKAHASGVTGSGVKVAILDTGIDANHTDLNVKGGASFVSGEPNAL
QDGNGHGTHVAGTVAALNNSTGVLGVAYNADLYAVKVLSASGSGTLSGIAQGIEWSI
ANGMNVINMSLGGSTGSTALQQACDNAYARGIVVIAAAGNSGSRGKQNTMGYPARYS
SVIAVGAVDSSNNRASFSSVGNELEVMAPGVNILSTTPGNNYASFNGTSMASPHVAGA
AALIKAKYPSMTNVQIRDRLKNTATNLGDPFFYGKGVINVESALQ

In *Bacillus horikoshii* DSM 9712, another gene was identified which encodes an LG12-clade subtilisin. The sequence of this gene, *DSM9712_sprC,* is depicted in SEQ ID NO: 14.
SEQ ID NO: 14 sets forth the nucleotide sequence of the *DSM9712_sprC* gene:

TTGAAGAAGTGGATGAAAGGTTTTTCGGTCATACTTATAGCAATATTGGCTTTCTCA
TTAGCCTTCGGAACTGGAACTGCCCAGGCGGAATCTTCAGGACCGAGTGCTGG
TGCAAAGAAAGAGTACTTAATTGGATTTACGAAAGGAAATAAAGCAAGCGCACAA
TCCTCCCAAAATCTAGTCACTGCTGCAGGTGGCGATATTCAATACACATTCCAAT
ACATGGATGTAGTGGAAGTAACTTTACCTGAAAAAGCTGCTGAGGCATTAAAGAAG
AACCCTAACATTGCATTTGTAGAGGAAAACGTAGAAATGATGGCGACTGCACAG
ACAGTACCATGGGGTATTCCGCACATCAAAGCAGATAAGGCACATGCCGCTGGTGT
AACTGGAAGTGGTGTGAAGGTAGCGATCCTTGACACAGGAATTGACGCTAACCA
TGCCGACCTTAATGTAAAAGGTGGAGCAAGCTTTGTTTCTGGTGAACCAAATGCCCT
ACAAGATGGCAATGGCCATGGAACACATGTAGCGGAACGGTTGCTGCGTTAA
ATAACACAACTGGTGTGCTTGGAGTGGCTTACAATGCGGATCTTTATGCAGTTAAAG
TCCTTAACGCATCTGGTAGTGGACGTTAAGTGGCATTGCCCAAGGAATTGAG
TGGTCCATTGCAAATGGAATGGATGTAATCAATATGAGCCTTGGTGGCAGCACTGG
ATCAACTGCATTACAACAAGCTAGTAATAATGCTTACAATAGAGGCATTGTCGT
CATTGCAGCTGCCGGAAATTCAGGTTCTTTAGGCAATCGGAATACAATTGGGTATCC
TGCCAGATATAGTTCTGTTATCGCGGTAGGAGCGGTTGACTCCAACAACAACC
GTGCTTCCTTCTCTAGTGTAGGAAGTGAATTGGAAGTAATGGCACCTGGTGTAAATA
TTCTGAGCACGACTCCTGGTAATAACTATGCTTCTTTCAATGGTACATCTATG
GCAGCTCCACATGTTGCAGGAGCAGCAGCATTAATCAAGGCGAAATACCCGAATAT
GACAAACGTCCAAATTCGTGATCGCCTGAGAAATACAGCTACAAACCTTGGTGA
TCCATTCTTCTATGGAAGAGGAGTCATCAATGTGGAAAGTGCTTTACAATAA

The preproenzyme encoded by the *DSM9712_sprC* gene is depicted in SEQ ID NO: 15. At the N-terminus, the protein has a signal peptide with a length of 29 amino acids as predicted by SignalP-NN. This signal peptide sequence is underlined and in bold in SEQ ID NO: 15. The presence of a signal peptide indicates that this serine protease is a secreted enzyme. The enzyme has a pro sequence which is predicted to be 79 amino acids (*in italics* in SEQ ID NO: 15). The sequence of the predicted, fully processed mature chain (DSM9712_SprC, 275 amino acids) is depicted in SEQ ID NO: 16.
SEQ ID NO: 15 sets forth the amino acid sequence of the serine protease precursor of DSM9712_SprC: 
MKKWMKGFSVILIAILAFSLAFGTGTAQAESSGPSAGAKKEYLIGFTKGNKASAQSSQN
LVTAAGGDIQYTFQYMDVVEVTLPEKAAEALKKNPNIAFVEENVEMMATAQTVPWGIPHIK
ADKAHAAGVTGSGVKVAILDTGIDANHADLNVKGGASFVSGEPNALQDGNGHGTHVA
GTVAALNNTTGVLGVAYNADLYAVKVLNASGSGTLSGIAQGIEWSIANGMDVINMSL
GGSTGSTALQQASNNAYNRGIVVIAAAGNSGSLGNRNTIGYPARYSSVIAVGAVDSNN
NRASFSSVGSELEVMAPGVNILSTTPGNNYASFNGTSMAAPHVAGAAALIKAKYPNMT
NVQIRDRLRNTATNLGDPFFYGRGVINVESALQ
SEQ ID NO: 16 sets forth the amino acid sequence of the mature protease DSM9712_SprC (275 amino acids):

AQTVPWGIPHIKADKAHAAGVTGSGVKVAILDTGIDANHADLNVKGGASFVSGEPNAL
QDGNGHGTHVAGTVAALNNTTGVLGVAYNADLYAVKVLNASGSGTLSGIAQGIEWSI
ANGMDVINMSLGGSTGSTALQQASNNAYNRGIVVIAAAGNSGSLGNRNTIGYPARYSS
VIAVGAVDSNNNRASFSSVGSELEVMAPGVNILSTTPGNNYASFNGTSMAAPHVAGAA
ALIKAKYPNMTNVQIRDRLRNTATNLGDPFFYGRGVINVESALQ

In *Bacillus horikoshii* DSM 6951, another gene was identified encoding an LG12-clade subtilisin. The sequence of this gene, *DSM6951_sprC,* is depicted in SEQ ID NO: 17.
SEQ ID NO: 17 sets forth the nucleotide sequence of the *DSM6951_sprC* gene:

TTGAAAAAGTGGATGAAAGGTTTTTCGGTCATACTTATCGCAATATTGGCTTTCTCG
TTAGCCTTCGGAACTGCTCAGGCAGAAGCATCAAATCCGAATGCTAGTGCAAA
GAAAGAATACTTAGTTGGATTTGCAAAAGGTAATAAAGCAAATGCCCAGTCTTCTA
AAAATTTGATTACCGCTGCTGGTGGGGACATACAACATACATTCCAGTACATGG
ATGTTGTGGAAGTATCGTTGCCTGTAAAAGCTGCAGAAGCATTAATGAAGAATCCT
AACATTGCTTTCGTTGAGGAAAACGTCGAAATGATGGCTACTGCTCAAACAGTT
CCTTGGGGCATCCCTCATATCAAAGCTGACAAAGCGCATGCTGCTGGAGTGACTGG
AAGCGGTGTGAAGGTTGCCATCCTGGATACAGGAATTGACGCAAACCATGCCGA
TCTTAACGTAAAAGGCGGAGCAAGCTTTGTTTCTGGTGAGCCGAATGCTCTTCAAGA
CGGTAACGGCCATGGAACACACGTAGCTGGAACAGTTGCTGCTTTGAATAACA
CAACTGGTGTACTTGGTGTAGCTTACAATGCTGACCTATATGCAGTAAAAGTACTTA
GTGCTTCAGGTAGTGGAACATTAAGTGGCATTGCGCAAGGTATTGAGTGGTCC

GTTGCTAACGGAATGGATGTCATCAATATGAGTCTTGGTGGAAGCTCTGGATCTTCA
GCTTTACAACAAGCTTGTGATAATGCTTATGCTAGAGGAGTTGTAGTAATTGC
CGCAGCTGGGAACTCTGGTTCAAGAGGAAAGCAAAACACAATGGGGTATCCTGCTA
GATACAGTTCTGTTATCGCAGTAGGAGCGGTGGATTCCAGTAACAACCGTGCCT
CTTTCTCCAGTGTTGGGAATGAACTGGAAGTAATGGCTCCTGGAGTGAATATTTTAA
GCACGACTCCTGGTAATAACTATGCTTCTTTCAATGGTACGTCCATGGCATCT
CCGCACGTTGCAGGAGCTGCTGCATTAATCAAGGCGAAATACCCAAGCATGACAAA
TGTTCAAATTCGTGAACGTCTGAAAAATACGGCTACAAATTTAGGTGATCCTTT
CTTCTACGGAAAAGGTGTCATCAATGTGGAAAGTGCTTTACAATAA

The preproenzyme encoded by the *DSM6951_sprC* gene is depicted in SEQ ID NO: 18. At the N-terminus, the protein has a signal peptide with a length of 27 amino acids as predicted by SignalP-NN. This signal peptide sequence is underlined and in bold in SEQ ID NO: 18. The presence of a signal peptide indicates that this serine protease is a secreted enzyme. The enzyme has a pro sequence which is predicted to be 79 amino acids (*in italics* in SEQ ID NO: 18). The sequence of the predicted, fully processed mature chain (DSM6951_SprC, 275 amino acids) is depicted in SEQ ID NO: 19.
SEQ ID NO: 18 sets forth the amino acid sequence of the serine protease precursor of DSM6951_SprC: 
MKKWMKGFSVILIAILAFSLAFGTAQAEASNPNASAKKEYLVGFAKGNKANAQSSKNLIT
AAGGDIQHTFQYMDVVEVSLPVKAAEALMKNPNIAFVEENVEMMATAQTVPWGIPHIKAD
KAHAAGVTGSGVKVAILDTGIDANHADLNVKGGASFVSGEPNALQDGNGHGTHVAGT
VAALNNTTGVLGVAYNADLYAVKVLSASGSGTLSGIAQGIEWSVANGMDVINMSLGG
SSGSSALQQACDNAYARGVVVIAAAGNSGSRGKQNTMGYPARYSSVIAVGAVDSSNN
RASFSSVGNELEVMAPGVNILSTTPGNNYASFNGTSMASPHVAGAAALIKAKYPSMTN
VQIRERLKNTATNLGDPFFYGKGVINVESALQ
SEQ ID NO: 19 sets forth the amino acid sequence of the mature protease DSM6951_SprC (275 amino acids): 
AQTVPWGIPHIKADKAHAAGVTGSGVKVAILDTGIDANHADLNVKGGASFVSGEPNAL
QDGNGHGTHVAGTVAALNNTTGVLGVAYNADLYAVKVLSASGSGTLSGIAQGIEWSV
ANGMDVINMSLGGSSGSSALQQACDNAYARGVVVIAAAGNSGSRGKQNTMGYPARY
 
SSVIAVGAVDSSNNRASFSSVGNELEVMAPGVNILSTTPGNNYASFNGTSMASPHVAGA
AALIKAKYPSMTNVQIRERLKNTATNLGDPFFYGKGVINVESALQ
SEQ ID NO: 20 sets forth the amino acid sequence of the mature protease sp. KSM-LD1, SB (BAD21128) (276 amino acids):

SQTVPYGVPHIKADVAHSQNVTGNGVKVAILDTGIDAAHEDLRVVGGASFVAGEPNAL
QDGNGHGTHVAGTVAALNNQVGVLGVAYDVDLYAVKVLGADGSGTLSGIAQGIEWSI
ANNMDVINMSLGGSTGSTTLKQAADNAYNSGLVVVAAAGNSGDFFGLINTIGYPARYD
SVIAVGAVDSNNRRASFSSVGSQLEVMAPGVNILSTLPGNSYGSLNGTSMASPHVAGAA
ALLLAQDPTLTNVQVREILRDTATNLGSSFYYGNGVIDVEKALQ

### EXAMPLE 8

### Heterologous expression of B. horikoshii serine proteases

Bhon03321, DSM9711, DSM9712 and DSM6951 proteases were produced in *B*. *subtilis* using an expression cassette consisting of a DNA sequence encoding the pro-mature sequence of Bhon03321, DSM9711_SprC, DSM9712_SprC and DSM6951_SprC, preceded by the *B. subtilis aprE* promoter, the *B. subtilis aprE* signal peptide sequence, and ending with the BPN' transcription terminator sequence. The four different cassettes were cloned into the pHYT replicating shuttle vector and transformed into a suitable *B. subtilis* strain. The pHYT vector was derived from pHY300PLK (Takara) by adding a terminator after the tetracycline resistance gene using the *Bst*EII and *Eco*RI sites (SEQ ID NO:21 terminator sequence, GGTTACCTTGAATGTATATAAACATTCTCAAAGGGATTTCTAATAAAAAACGCTCGG TTGCCGCCGGGCGTTTTTTATGCATCGATGGAATTC). The *Hind*III site in pHY300PLK was also removed using a linker cloned into the *Bam*HI and *Hind*III sites (SEQ ID NO:22 new linker sequence, GGATCCTGACTGCCTGAGCTT).

A map of the pHYT vector containing the Bhon03321 gene (pHYT-Bhon03321) is shown in Figure 3. The expression plasmids for DSM971 1_SprC, DSM9712_SprC and DSM6951_SprC (not shown) are similar and differ only in the sequence encoding the pro-mature parts of the respective proteases.

To produce Bhon03321, DSM9711, DSM9712 and DSM6951 proteases, *B. subtilis* transformants containing pHYT- Bhon03321, pHYT- DSM9711_SprC, pHYT- DSM9712_SprC or DSM6951_SprC, respectively, were cultivated in an enriched semi-defined media based on MOPs buffer, with urea as major nitrogen source, glucose as the main carbon source, and supplemented with 1% soytone for robust cell growth. The media was supplemented with 25 ppm tetracycline. After incubation (2 days at 32°C), protease was detected in the growth medium of all four strains. After centrifugation and filtration, culture supernatants with Bhon03321, DSM9711, DSM9712 and DSM6951 protease were used for assays and purification.

### EXAMPLE 9

### Cleaning performance of B. horikoshii serine proteases

The cleaning performances of ***B. horikoshii*** serine proteases Bhon03321, DSM6951_SprC (DSM_6951), DSM9711_SprC (DSM_9711), and DSM9712_SprC (DSM_9712) were tested on BMI (blood/milk/ink on cotton) microswatches (EMPA-116, Center for Testmaterials, The Netherlands) for laundry based applications, and on egg yolk (egg yolk on polyacryl fabric, aged and colored with carbon black dye) microswatches (PAS-38, Center for Testmaterials, The Netherlands) for dish based applications.

Automatic dish washing (ADW) cleaning assays, (GSM-B 9 and GSM-B 10.5), were carried out at 40°C for 30 minutes. Following incubation, 100 uL of supernatant was transferred to a fresh MTP (Costar 9017) and absorbance was read at 405 nm for PAS-38 swatches, using the SpectraMax plate reader. The absorbance from a buffer-only control was subtracted and the resulting OD values at 405nm were plotted as a function of protease concentration. The data was fitted to a Sigmoidal fit.

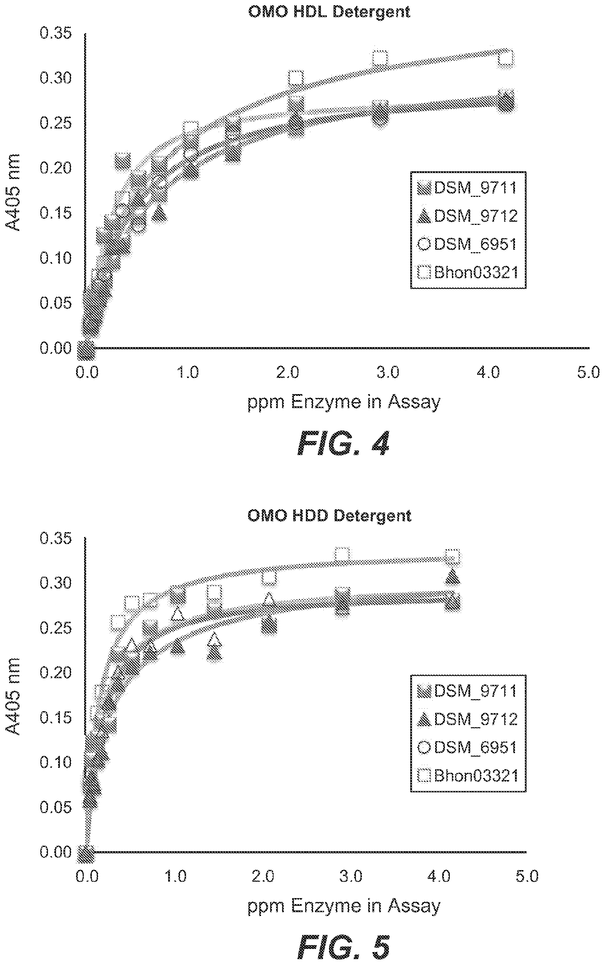

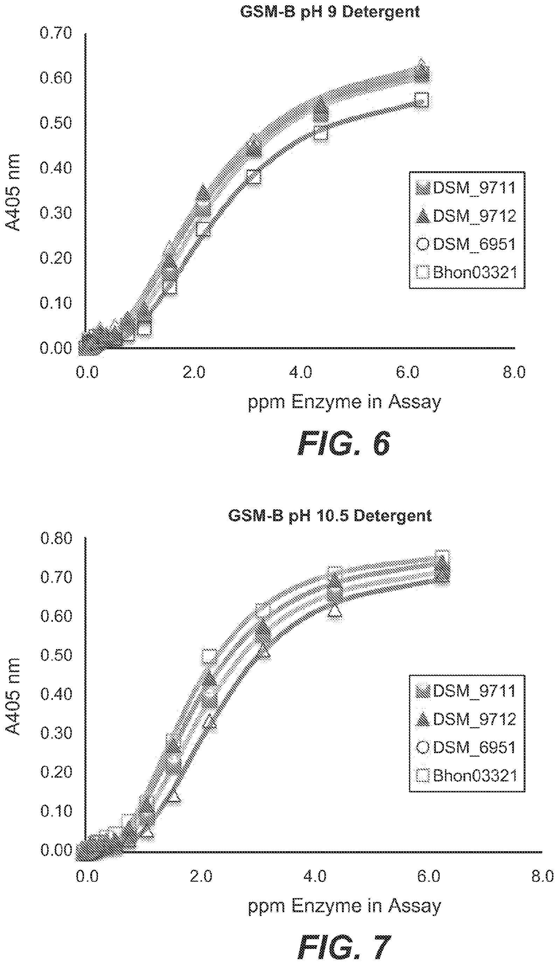

Laundry cleaning assay with commercially available HDL (OMO Klein & Krachtig, Unilever) or HDD (OMO Color, Unilever) detergent formulas was carried out at 25°C for 15 minutes. Following incubation, 100 uL of supernatant was transferred to a fresh MTP (Costar 9017) and absorbance was read at 405 nm for BMI swatches (blood milk and ink, Center for Testmaterials, The Netherlands) using the SpectraMax plate reader. The absorbance from a buffer-only control was subtracted and the resulting OD values at 405nm were plotted as a function of protease concentration. Two swatches per well were used for assays with HDL detergent, while HDD detergent assay was done using one microswatch per well. The data was fitted to a Langmuir equation. The cleaning performances for LG12 clade proteases: Bhon03321, DSM6951_SprC (DSM_6951), DSM9711_SprC (DSM_9711), and DSM9712_SprC (DSM_9712) are shown in Figures 4-7.

### EXAMPLE 10

### Stability of LG12 and homologs

Bhon03321, DSM6951_SprC (DSM_6951), DSM9711_SprC (DSM_9711), DSM9712_SprC (DSM_9712) and LG12 SprC proteases were tested for stability in 50mM Tris pH9; 2mM CaC12 over a temperature range from 40-80°C by measuring the residual activity following incubation at elevated temperatures. Diluted enzyme sample was mixed in stressor and unstressed protease activity was measured. The diluted sample in stressor was incubated at elevated temperatures and after incubation the stressed protease activity was measured. For the unstressed condition, enzyme was assayed immediately for activity by the DMC method (as described below). For the stressed condition, the PCR plate was sealed and incubated at elevated temperatures for 5 minutes using a Eppendorf 384 Thermocycler, then assayed for activity. Stressed and unstressed activity was measured by the DMC method.

For the DMC assay, the reagent solutions used were: 2.5% Dimethylcasein (DMC, Sigma) in 100 mM Sodium Carbonate pH 9.5, 0.075% TNBSA (2,4,6-trinitrobenzene sulfonic acid, Thermo Scientific) in 100 mM Sodium Carbonate pH 9.5. Dilution Solution: 10 mM NaCl, 0.1 mM CaCl2, 0.005% Tween-80, 0.02% Na-azide. MTPs (Greiner PS-microwell 384) were filled with 27.5 uL DMC substrate following the addition of 5 uL of 20 ppm protease supernatant. 27.5 uL of TNBSA solution was then added with slow mixing. Activity was measured at 405 nm over 5 minutes using a SpectraMax plate reader in kinetic mode at RT and activity was expressed as mOD/min.

The absorbance from a buffer-only control was subtracted and the resulting OD values at 405nm were plotted as a function of temperature. Where possible, the data was fitted to a 4 parameter logistic function (y=a+b/(1+(x/c)^d)). The temperature at which 50% activity for LG12, Bhon03321, DSM6951_SprC (DSM_6951), DSM9711_SprC (DSM_9711), and DSM9712_SprC (DSM_9712 proteases was retained was calculated and is shown in Table 8.

| **Table 8 Stability of LG12 and homologs** | |
|---|---|
| **Enzyme** | **T_{50%} (°C)** |
| LG12 wt | 62 |
| Bhon03321 | 72 |
| DSM9712 | 67-72 |
| DSM6951 | 75 |
| DSM9711 | 75 |

### EXAMPLE 11

### Phylogenetic Tree for mature LG12-clade and other subtilisins

A phylogenetic tree was built using the Neighbor Joining method (NJ) (Saitou, N.; and Nei, M. (1987). The neighbor-joining method: a new method for reconstructing Guide Trees. Mol Biol Evol 4, 406-425) for a subset of the mature sequences identified from NCBI searches shown in EXAMPLE 4 TABLE 5, and the newly discovered *Bacillus horikoshii* serine proteases: Bhon03321 (SEQ ID NO:10), DSM_9711 (SEQ ID NO:13), DSM_9712 (SEQ ID NO:16), and DSM_6951 (SEQ ID NO:19). The NJ method works on a matrix of distances between all pairs of sequences to be analyzed. These distances are related to the degree of divergence between the sequences. The tree construction was calculated using the following parameters: Kimura's correction for sequence distance and ignoring positions with gaps. MEGA6 program was used to display the tree shown in Figure 8. Sequences of Bhon03321, DSM_9711, DSM_9712, and DSM_6951 appear within the cluster of 10 proteases constituting the LG12-clade.The LG12-clade members so far indentified consists of: LG12 sprC (SEQ ID NO:3), DSM_9711 (SEQ ID NO:13), DSM_9712 (SEQ ID NO:16), DSM_6951 (SEQ ID NO:19), Bhon03321 (SEQ ID NO:10), Bac sp WP_010192403.1, Bac sp LG12SprD AAC43581, Bac sp WP_010192405.1, and Bac sp BAD21128.

### EXAMPLE 12

### Alignment of Homologous Sequences

The amino acid sequences of mature subtilisin sequences identified within the LG12-clade: LG12 sprC (SEQ ID NO:3), DSM_9711 (SEQ ID NO:13), DSM_9712 (SEQ ID NO:16), DSM_6951 (SEQ ID NO:19), Bhon03321 (SEQ ID NO:10), Bac sp BAD11988, Bac sp WP_010192403.1, Bac sp LG12SprD AAC43581, Bac sp WP_010192405.1, Bac sp BAD21128 were aligned to each other and the more distantly related subtilisin of *B lentus* (P29600).Protein sequence alignment was performed using CLUSTALW software (Thompson et al., Nucleic Acids Research, 22:4673-4680, 1994) with the default parameters over the region of residues 1 to 275 of LG12 sprC. The alignment of these eleven proteases is depicted in Figure 9.

### EXAMPLE 13

### Identification of unique sequences shared by LG12-clade subtilisins

A structure-based sequence alignment of LG12 sprC protease with other subtilisin proteases is shown in Figure 10. Mature protein sequences of detergent proteases *Bacillus lentus* (pdb code 1JEA), *Bacillus amyloliquefaciens* (pdb code 2ST1.A), and *Bacillus licheniformis* (pdb code 3UNX.A) were aligned with sequences of : LG12 sprC (SEQ ID NO:3, AAC43580.1) and homologs: Bacillus sp. m3-13 subtilisin E(1) (SEQ ID NO:7, WP_010192403.1, formerly ZP_07707657.1), Bacillus sp. KSM-LD1, SA (BAD11988.2), Bacillus sp. KSM-LD1, SB (BAD21128.1), Bacillus sp. m3-13 subtilisn E(2) (WP_10192405.1, formerly ZP_07707658.1), Bhon03321 (SEQ ID NO:10), DSM_9711, DSM_9712, DSM_6951 and LG12sprD (AAC43581.1).

Figure 10 shows three underlined regions where the sequences of LG12 sprC protease and it homologs differ from the sequences of other detergent proteases. One is the appearance of P, N or D, A, and L at positions 55-58 in LG12 sprC protease and its homologs, in place of a very heterologous region found in other detergent proteases such as Carlsberg, BPN' and *B. lentus.*

Another finding shared by all the LG12-clade subtilisins is the appearance of Y at position 86 followed by N or D at position 87 in LG12 sprC protease and its homologs, in place of the P and S sequence found in other commercial detergent proteases. And finally is the presence of T and L at positions 103 and 104 in LG12 sprC protease and its homologs, in place of Q/S and Y/V found in the commercial detergent proteases.

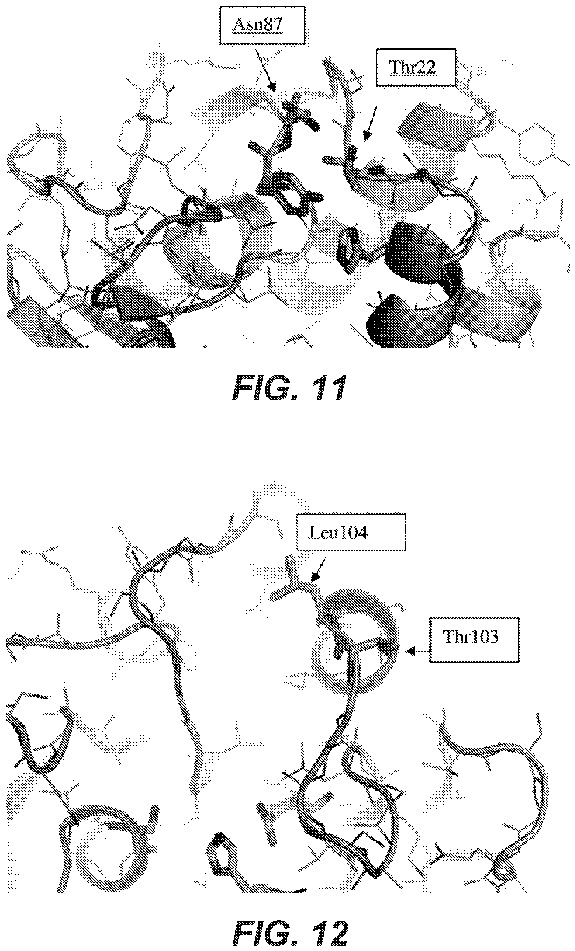

A homology model of LG12 sprC protease was prepared based on the structure of subtilisin Carlsberg *Bacillus licheniformis* (pdb code 3UNX.A) which shares the highest sequence identity of the subtilisin structures from *B. amyloliquefaciens, B. licheniformis* and *B. lentus.* The structure in the vicinity of Tyr86 and Asn87 in the homology model of LG12 sprC protease is shown in Figure 11. The side chain of Tyr86 forms a hydrogen bond to His17 and the side chain of Asn87 is in a position to form a hydrogen bond with Thr22. In the alignment on Figure 10 above it is interesting to note that His17 is conserved in all LG12-like sequences along with a preceding Ala16 and Thr22 is also conserved in all LG12 sprC-like sequences along with a preceding Val21. These hydrogen bonds would serve to stabilize loops and would be expected to represent a structural motif common to all LG12 sprC protease-like enzymes. In other known subtilisin structures, a Pro is found at the position homologous to position 86 in LG12 sprC protease and its homologs and could serve as an alternative means of stabilizing this region in the molecule.

Another common sequence motif identified in LG12 sprC protease and its homologs is the conservation of Thr103 and Leu104. These residues form a part of the substrate binding cleft as shown in Figure 12. The catalytic triad comprising Ser221, His64 and Asp32 is shown as a stick figure along with residues Thr103 and Leu104. This combination of residues found in the substrate binding cleft would be expected to contribute to the substrate specificity of LG12 sprC protease and LG12 clade proteases.

The unique sequence motifs shared by the LG12-clade wildtype enzymes include: Thr103, Leu104, and Tyr86, Asp/Asn87 in a mature sequence of 275 residues. In addition, the LG12 clade proteases display a signature motif at the C-terminus, starting at position 267: V,I,N/D,V/L,E,X,A,L,Q, where the X represents any amino acid. These sequences (V,I,N/D,V/L,E,X,A,L,Q) fromV267 to Q275 are uniquely found in the LG12 clade subtilisins.

A signature sequence motif has been identified for all the members of the LG12-clade subtilisins [LG12 sprC (SEQ ID NO:3, AAC43580.1) and homologs: Bacillus sp. m3-13 subtilisin E(1) (SEQ ID NO:7, WP_010192403.1, formerly ZP_07707657.1), Bacillus sp. KSM-LD1, SB (BAD21128.1), Bacillus sp. m3-13 subtilisn E(2) (WP_10192405.1, formerly ZP_07707658.1), Bhon03321 (SEQ ID NO:10), DSM_9711, DSM_9712, DSM_6951 and LG12sprD (AAC43581.1)]. The sequence motifs consist of: Pro55-Asp/Asn56- Ala57-Leu58 and Tyr86-Asp/Asn87 and Thr103-Leu104. The residues 55-58 confer a unique configuration in the loop observed in the structure of LG12-SprC. Residues 86-88 appear to fill a space in the overall 3 dimensional structure, while residues 103-104 are adjacent to the active site and likely influence the susbstrate binding properties of the subtilisins in this clade.

## Claims

1. An LG 12-clade variant subtilisin enzyme or an active fragment thereof comprising an amino acid modification to a parent LG12-clade subtilisin enzyme, wherein the modification is at productive position 166, wherein said variant has at least 75% identity to the amino acid sequence of a parent LG12-clade subtilisin enzyme selected from Bacillus sp.LG12sprC substilin set forth in SEQ ID NO:3; Bacillus sp. M3-13 subtilisin enzyme E(1) set forth in SEQ ID NO:7; Bacillus horikoshii strain DSM 8719, Bhon03321 set forth in SEQ ID NO:10; Bacillus horikoshii strain DSM_9711 set forth in SEQ ID NO:13; Bacillus horikoshii strain DSM_9712 set forth in SEQ ID NO:16; Bacillus horikoshii strain DSM_6951 set forth in SEQ ID NO:19; B_sp_sprD_AAC43581 as shown in Fig. 9A-C; Bacillus_sp_WP_010192405 as shown in Fig. 9A-C; Bacillus_sp_BAD 11988 as shown in Fig. 9A-C; and/or Bacillus sp. KSM-LD1, SB (BAD21128.1) set forth in SEQ ID NO:20, wherein the modification is selected from the group consisting of residues 166 C, F, I, L, A, D, E, H, M, N, Q, S, T and Y, wherein the amino acid positions of the LG12-clade subtilisin variant are numbered by correspondence with the amino acid sequence of *Bacillus sp.* LG12sprC subtilisin set forth in SEQ ID NO:3.

2. The LG12-clade variant subtilisin enzyme or an active fragment thereof of claim 1, wherein said parent LG12-clade subtilisin enzyme contains:
a catalytic triad comprising Ser221, His64, and Asp32;
a P, N/D, A, L motif from positions 55-58;
a Thr103-Leu104 motif;
a Tyr86-Asn/Asp87 motif;
an Ala16-His17 motif;
a Val21-Thr22 motif;
a V,I,N/D,V/L,E,X,A,L,Q motif from positions 267-275;
a Tyr86-Asn/Asp87 motif, and a Val21-Thr22 motif; and/or
a Tyr86-Asn/Asp87 motif, an Ala16-His17 motif, and a Val21-Thr22 motif;
wherein the amino acid positions are numbered by correspondence with the amino acid sequence of LG12 sprC protease set forth in SEQ ID NO:3.

3. A composition comprising at least one subtilisin enzyme of any one of claims 1-2, optionally wherein said composition is selected from a granular, powder, solid, bar, liquid, tablet, gel, unit dose or paste composition.

4. The composition of claim 3, wherein said composition is a cleaning composition.

5. The composition of claim 4, wherein said composition is a detergent composition, optionally wherein said detergent composition is selected from the group consisting of a laundry detergent, a fabric softening detergent, a dishwashing detergent, and a hard-surface cleaning detergent.

6. The composition of any of claims 3 to 5, wherein said composition further comprises a surfactant, optionally selected from the group consisting of an anionic surfactant, a cationic surfactant, a zwitterionic surfactant, a ampholytic surfactant, a semi-polar non-ionic surfactant, and a combination thereof; at least one stabilizer; from about 0.001 to about 10 weight % of said subtilisin enzyme; at least one bleaching agent; at least one adjunct ingredient; and/or one or more additional enzymes or enzyme derivatives selected from the group consisting of acyl transferases, alpha-amylases, beta-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-beta-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, and xylosidases, additional metallopotease enzymes and combinations thereof.

7. The composition of any of claims 3-6, wherein said cleaning composition is phosphate-free.

8. A method of cleaning, comprising contacting a surface or an item with a cleaning composition comprising at least one subtilisin enzyme of any one of claims 1-2, or a cleaning composition set forth in any one of claims 3-7; and optionally rinsing said surface or item after contacting said surface or item, respectively, with said cleaning composition.

9. The method of claim 8, wherein said item is dishware or fabric.

10. A textile processing, animal feed, leather processing, feather processing, grain processing, cellulosic ethanol processing, lens cleaning, tissue debridement, or tissue cell culture additive composition comprising the subtilisin enzyme of any one of claims 1-2.

## Patentansprüche

1. LG12-Klade-Subtilisinenzym-Variante oder aktives Fragment davon, umfassend eine Aminosäuremodifikation an einem parentalen LG12-Klade-Subtilisinenzym, wobei sich die Modifikation an produktiver Position 166 befindet, wobei die Variante zumindest 75% Identität mit der Aminosäuresequenz eines parentalen LG12-Klade-Subtilisinenzyms aufweist, das ausgewählt ist aus Bacillus sp. LG12sprC Subtilisin, aufgeführt in SEQ ID NO:3; Bacillus sp. M3-13 Subtilisinenzym E(1), aufgeführt in SEQ ID NO:7; Bacillus-horikoshii-Stamm DSM 8719, Bhon03321, aufgeführt in SEQ ID NO:10; Bacillus-horikoshii-Stamm DSM_9711, aufgeführt in SEQ ID NO:13; Bacillus-horikoshii-Stamm DSM_9712, aufgeführt in SEQ ID NO:16; Bacillus-horikoshii-Stamm DSM_6951, aufgeführt in SEQ ID NO:19; B_sp_sprD_AAC43581, wie dargestellt in Fig. 9A-C; Bacillus_sp_WP_010192405, wie dargestellt in Fig. 9A-C; Bacillus_sp_BAD11988, wie dargestellt in Fig. 9A-C; und/oder Bacillus sp. KSM-LD1, SB (BAD21128.1), aufgeführt in SEQ ID NO:20, wobei die Modifikation aus der Gruppe ausgewählt ist, die aus Resten 166 C, F, I, L, A, D, E, H, M, N, Q, S, T und Y besteht, wobei die Aminosäurepositionen der LG12-Klade-Subtilisin-Variante in Korrespondenz mit der Aminosäuresequenz von *Bacillus sp.* LG12sprC Subtilisin, aufgeführt in SEQ ID NO:3, nummeriert sind.

2. LG12-Klade-Subtilisinenzym-Variante oder aktives Fragment davon nach Anspruch 1, wobei das parentale LG12-Klade-Subtilisinenzym enthält:
eine katalytische Triade, umfassend Ser221, His64 und Asp32;
ein Motiv P, N/D, A, L von Positionen 55-58;
ein Thr103-Leu104-Motiv;
ein Tyr86-Asn/Asp87-Motiv;
ein Ala16-His17-Motiv;
ein Val21-Thr22-Motiv;
ein V,I,N/D,V/L,E,X,A,L,Q-Motiv von Positionen 267-275;
ein Tyr86-Asn/Asp87-Motiv, und ein Val21-Thr22-Motiv; und/oder
ein Tyr86-Asn/Asp87-Motiv, ein Ala16-His17-Motiv, und ein Val21-Thr22-Motiv;
wobei die Aminosäurepositionen in Korrespondenz mit der Aminosäuresequenz von LG12 sprC Protease, aufgeführt in SEQ ID NO:3, nummeriert sind.

3. Zusammensetzung, umfassend zumindest ein Subtilisinenzym nach einem der Ansprüche 1-2, wobei die Zusammensetzung gegebenenfalls aus einer granulatförmigen, pulverförmigen, festen, Riegel-, flüssigen, Tabletten-, Gel-, Dosiseinheit-, oder Pastenzusammensetzung ausgewählt ist.

4. Zusammensetzung nach Anspruch 3, wobei die Zusammensetzung eine Reinigungszusammensetzung ist.

5. Zusammensetzung nach Anspruch 4, wobei die Zusammensetzung eine Detergenszusammensetzung ist, wobei die Detergenszusammensetzung wahlweise aus der Gruppe ausgewählt ist, die aus einem Wäschedetergens, einem Weichspüldetergens, einem Geschirrspüldetergens und einem Reinigungsdetergens für harte Flächen besteht.

6. Zusammensetzung nach einem der Ansprüche 3 bis 5, die Zusammensetzung weiterhin umfassend ein Tensid, wahlweise ausgewählt aus der Gruppe, bestehend aus einem anionischen Tensid, einem kationischen Tensid, einem zwitterionischen Tensid, einem ampholytischen Tensid, einem semipolaren nichtionischen Tensid und einer Kombination davon; zumindest einen Stabilisator; ungefähr 0,001 bis ungefähr 10 Gew.-% des Subtilisinenzyms; zumindest ein Bleichmittel; zumindest einen Zusatzinhaltsstoff; und/oder ein oder mehrere zusätzliche Enzyme oder Enzymderivate, ausgewählt aus der Gruppe, bestehend aus Acyltransferasen, Alpha-Amylasen, Beta-Amylasen, Alpha-Galaktosidasen, Arabinosidasen, Arylesterasen, Beta-Galaktosidasen, Carrageenasen, Katalasen, Cellobiohydrolasen, Cellulasen, Chondroitinasen, Cutinasen, Endo-beta-1,4-Glukanasen, Endo-beta-Mannanasen, Esterasen, Exo-Mannanasen, Galaktanasen, Glukoamylasen, Hemicellulasen, Hyaluronidasen, Keratinasen, Laccasen, Laktasen, Ligninasen, Lipasen, Lipoxygenasen, Mannanasen, Oxidasen, Pektatlyasen, Pektinacetylesterasen, Pektinasen, Pentosanasen, Peroxidasen, Phenoloxidasen, Phosphatasen, Phospholipasen, Phytasen, Polygalakturonasen, Proteasen, Pullulanasen, Reduktasen, Rhamnogalakturonasen, Beta-Glukanasen, Tannasen, Transglutaminasen, Xylanacetylesterasen, Xylanasen, Xyloglukanasen, und Xylosidasen, zusätzlichen Metalloproteaseenzymen und Kombinationen davon.

7. Zusammensetzung nach einem der Ansprüche 3-6, wobei die Reinigungszusammensetzung phosphatfrei ist.

8. Reinigungsverfahren, umfassend In-Kontakt-Bringen einer Fläche oder eines Gegenstands mit einer Reinigungszusammensetzung, umfassend zumindest ein Subtilisinenzym nach einem der Ansprüche 1-2, oder einer Reinigungszusammensetzung nach einem der Ansprüche 3-7; und gegebenenfalls Spülen der Fläche oder des Gegenstands nach In-Kontakt-Bringen von jeweils der Fläche oder dem Gegenstand mit der Reinigungszusammensetzung.

9. Verfahren nach Anspruch 8, wobei der Gegenstand Geschirr oder eine Textilie ist.

10. Textilverarbeitungs-, Tierfutter-, Lederverarbeitungs-, Federverarbeitungs-, Kornverarbeitungs-, Cellulose-Ethanol-Verarbeitungs-, Linsenreinigungs-, Gewebe-Débridement- oder Gewebezellkulturadditiv-Zusammensetzung, umfassend das Subtilisinenzym nach einem der Ansprüche 1-2.

## Revendications

1. Enzyme subtilisine variante du clade LG12 ou un fragment actif de celle-ci comprenant une modification d'acide aminé sur une enzyme subtilisine du clade LG12 parente, dans laquelle la modification est à la position productive 166, où ledit variant a au moins 75% d'identité avec la séquence d'acides aminés d'une enzyme subtilisine du clade LG12 parente choisie parmi une subtilisine LG12sprC de l'espèce Bacillus présentée dans la SEQ ID NO:3; une enzyme subtilisine M3-13 de l'espèce Bacillus E(1) présentée dans la SEQ ID NO:7; DSM 8719, Bhon03321 d'une souche de Bacillus horikoshii présentée dans la SEQ ID NO:10; DSM_9711 d'une souche de Bacillus horikoshii présentée dans la SEQ ID NO:13; DSM_9712 d'une souche de Bacillus horikoshii présentée dans la SEQ ID NO:16; DSM_6951 d'une souche de Bacillus horikoshii présentée dans la SEQ ID NO:19; B_sp_sprD_AAC43581 comme il est montré dans les Fig. 9A-C; Bacillus_sp_WP_010192405 comme il est montré dans les Fig. 9A-C; Bacillus_sp_BAD11988 comme il est montré dans les Fig. 9A-C; et/ou KSM-LD1, SB (BAD21128.1) de l'espèce Bacillus présentée dans la SEQ ID NO:20, dans laquelle la modification est choisie dans le groupe constitué des résidus 166 C, F, I, L, A, D, E, H, M, N, Q, S, T et Y, dans laquelle les positions d'acides aminés du variant de subtilisine du clade LG12 sont numérotées en correspondance avec la séquence d'acides aminés de la subtilisine LG12sprC de l'espèce Bacillus présentée dans la SEQ ID NO:3.

2. Enzyme subtilisine variante du clade LG12 ou un fragment actif de celle-ci selon la revendication 1, dans laquelle ladite enzyme subtilisine du clade LG12 parente contient:
une triade catalytique comprenant Ser221, His64 et Asp32;
un motif P, N/D, A, L provenant des positions 55-58;
un motif Thr103-Leu104;
un motif Tyr86-Asn/Asp87;
un motif Ala16-His17;
un motif Val21-Thr22;
un motif V,I,N/D,V/L,E,X,A,L,Q provenant des positions 267-275;
un motif Tyr86-Asn/Asp87 et un motif Val21-Thr22; et/ou un motif Tyr86-Asn/Asp87, un motif Alal6-Hisl7 et un motif Val21-Thr22;
dans laquelle les positions d'acides aminés sont numérotées en correspondance avec la séquence d'acides aminés de la protéase LG12 sprC présentée dans la SEQ ID NO:3.

3. Composition comprenant au moins une enzyme subtilisine selon l'une quelconque des revendications 1-2, optionnellement où ladite composition est choisie parmi une composition granulaire, en poudre, solide, en barre, liquide, en comprimé, en gel, en dose unitaire ou en pâte.

4. Composition selon la revendication 3, où ladite composition est une composition nettoyante.

5. Composition selon la revendication 4, où ladite composition est une composition détergente, optionnellement où ladite composition détergente est choisie dans le groupe constitué d'un détergent pour laver le linge, d'un détergent assouplissant pour tissus, d'un détergent pour laver la vaisselle et d'un détergent de nettoyage de surface dure.

6. Composition selon l'une quelconque des revendications 3 à 5, où ladite composition comprend en outre un tensioactif, optionnellement choisi dans le groupe constitué d'un tensioactif anionique, d'un tensioactif cationique, d'un tensioactif zwitterionique, d'un tensioactif ampholytique, d'un tensioactif non ionique semi-polaire et d'une combinaison de ceux-ci; au moins un stabilisant; d'environ 0,001 à environ 10% en poids de ladite enzyme subtilisine; au moins un agent de blanchiment; au moins un ingrédient adjuvant; et/ou un ou plusieurs enzymes ou dérivés d'enzymes supplémentaires choisis dans le groupe constitué de: acyltransférases, alpha-amylases, bêta-amylases, alpha-galactosidases, arabinosidases, arylestérases, bêta-galactosidases, carraghénases, catalases, cellobiohydrolases, cellulases, chrondroïtinases, cutinases, endo-bêta-1,4-glucanases, endo-bêta-mannanases, estérases, exo-mannanases, galactanases, glucoamylases, hémicellulases, hyaluronidases, kératinases, laccases, lactases, ligninases, lipases, lipoxygénases, mannanases, oxydases, pectate lyases, pectine acétylestérases, pectinases, pentosanases, peroxydases, phénoloxydases, phosphatases, phospholipases, phytases, polygalacturonases, protéases, pullulanases, réductases, rhamnogalacturonases, bêta-glucanases, tannases, transglutaminases, xylane acétyl-estérases, xylanases, xyloglucanases et xylosidases, enzymes métalloprotéases supplémentaires et des combinaisons de celles-ci.

7. Composition selon l'une quelconque des revendications 3-6, où ladite composition nettoyante est sans phosphate.

8. Méthode pour un nettoyage, comprenant la mise en contact d'une surface ou d'un article avec une composition nettoyante comprenant au moins une enzyme subtilisine selon l'une quelconque des revendications 1-2 ou une composition nettoyante selon l'une quelconque des revendications 3-7; et optionnellement le rinçage de ladite surface ou dudit article après la mise en contact de ladite surface ou dudit article, respectivement, avec ladite composition nettoyante.

9. Méthode selon la revendication 8, dans laquelle ledit article est de la vaisselle ou un tissu.

10. Composition d'additif de traitement de textile, d'alimentation animale, de traitement de cuir, de traitement de plume, de traitement de grains, de traitement d'éthanol cellulosique, de nettoyage de lentille de contact, de débridement de tissus ou d'additif de culture de cellules de tissus comprenant l'enzyme subtilisine selon l'une quelconque des revendications 1-2.
